# EUROPEAN PATENT SPECIFICATION

(11) **EP 0 934 939 B1**
(45) Date of publication and mention of the grant of the patent: **02.05.2003**
(21) Application number: 97934769.7
(22) Date of filing: 12.08.1997
(51) Int. Cl.: C07D 309/28, A61K 31/35

(54) **NEURAMINIC ACID COMPOUNDS**
VERBINDUNGEN DER NEURAMINSAEURE
COMPOSES D'ACIDE NEURAMINIQUE

(30) Priority: 13.08.1996 JP 21345696
(43) Date of publication of application: 11.08.1999
(73) Proprietor: Sankyo Company, Limited, Tokyo 103-8426 (JP)
(72) Inventor: HONDA, Takeshi, c/o Sankyo Company, Ltd., Tokyo 140 (JP); KOBAYASHI, Yoshiyuki, c/o Sankyo Company, Ltd., Tokyo 140 (JP); YAMASHITA, Makoto, c/o Sankyo Company, Ltd., Tokyo 140 (JP)
(74) Representative: Gibson, Christian John Robert
(86) International application number: JP9702810
(87) International publication number: WO98006712

(56) References cited:
- WO-A-91/16320
- WO-A-95/20583
- JP-A- 6 025 209

## Description

### [Technical Field]

The present invention relates to a neuraminic acid compound or its pharmacologically acceptable salts having excellent in vivo sialidase inhibitory activity, compositions for treatment or prevention of influenza viral infections containing them as their active ingredients, their use for preparing a pharmaceutical for treatment or prevention of influenza viral infections, a treatment method or prevention method of influenza viral infections by administration of their pharmacologically effective amounts to warm-blooded animals, or their preparation process.

### [Background Art]

Influenza is a disease that is caused by viral infection. As one of the processes by which this virus proliferates, subviruses that have budded on the cell surface dissociate from the cell. Such subviruses are coupled to sialic acid on the cell surface mediated by hemagglutinin of the subvirus surface. Subviruses dissociate from the cell as a result of sialidase on the subvirus surface breaking down the sialic acid, thereby resulting in secondary infection of surrounding cells. Thus, inhibition of sialidase would make it possible to inhibit dissociation of subviruses from the cell surface, thereby preventing secondary infection. Accordingly, a substance that has the effect of inhibiting sialidase is considered to be effective in treating or preventing (but preferably treating) influenza.

Known compounds having sialidase inhibitory activity and a sialic acid (neuraminic acid) backbone are described in WO 91/16320 (Japanese PCT Application (Kokai) No. Hei 5-507068). Among those, Compound A (GG-167), represented by the following formula, is being developed as a drug for the treatment of influenza.

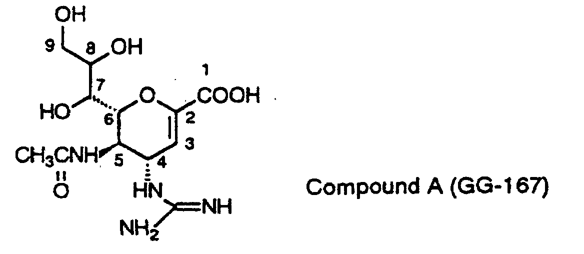

### [Disclosure of the Invention]

The present inventors conducted earnest research on the synthesis of a derivative having influenza infection therapeutic effects superior to those of Compound A (GG-167) described in WO 91/16320 (Japanese PCT Application (Kokai) No. Hei 5-507068), and on its pharmacological activity. As a result, they found that acyl derivatives of the hydroxyl group at the 7- and 8-positions and/or the 9-position and ester derivatives of the carboxyl group at the 1-position of Compound A exhibit excellent in vivo viral replication inhibitory activity and sialidase inhibitory activity similar to Compound A, while also exhibiting infection therapeutic effects superior to Compound A when administered to mice infected with influenza virus, therefore being useful as an anti-influenza drug, and they accomplished the present invention.

The present invention provides a neuraminic acid compound or its pharmacologically acceptable salts having excellent in vivo sialidase inhibitory activity, compositions for treatment or prevention of influenza viral infections containing them as their active ingredients, their use for preparing a pharmaceutical for treatment or prevention of influenza viral infections, a treatment method or prevention method of influenza viral infections by administration of their pharmacologically effective amounts to warm-blooded animals, or their preparation process.

The neuraminic acid of the present invention has the formula: 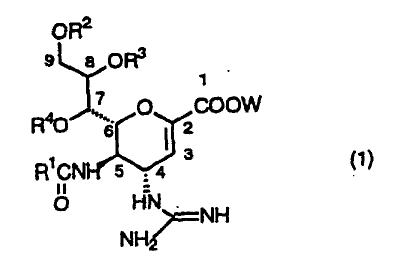 [wherein R¹ represents an alkyl group having from 1 to 4 carbon atoms which may be substituted with one or more halogen atoms; R², R³ and R⁴ are the same or different and each represents a hydrogen atom or an aliphatic acyl group having from 3 to 25 carbon atoms, and W represents a hydrogen atom or an ester residue, provided that compounds wherein each of R², R³, R⁴ and W is a hydrogen atom are excluded.

In the above general formula (1):
"The alkyl group having from I to 4 carbon atoms" of "the alkyl group having from 1 to 4 carbon atoms which may be substituted with one or more halogen atoms" of R¹ includes, for example, methyl, ethyl, n-propyl, isopropyl, n-butyl, isobutyl, s-butyl and tert-butyl groups, preferably a methyl group.
"The halogen atom" of "the alkyl group having from 1 to 4 carbon atoms which may be substituted with one or more halogen atoms" of R¹ includes, for example, fluorine, chlorine and bromine atoms, preferably a fluorine atom.
"The alkyl group having from 1 to 4 carbon atoms substituted with one or more halogen atoms" of "the alkyl group having from 1 to 4 carbon atoms which may be substituted with one or more halogen atoms" of R¹ includes, for example, monofluoromethyl, difluoromethyl, trifluoromethyl, 1-fluoroethyl, 2-fluoroethyl, 1-fluoropropyl, 2-fluoropropyl, 3-fluoropropyl, 4-fluorobutyl, monochloromethyl, dichloromethyl, trichloromethyl, 1-chloroethyl, 2-chloroethyl, 1-chloropropyl, 2-chloropropyl, 3-chloropropyl, 4-chlorobutyl, monobromomethyl, 1-bromoethyl, 2-bromoethyl, 1-bromopropyl, 2-bromopropyl, 3-bromopropyl, 4-bromobutyl and fluorochloromethyl groups, preferably a methyl group substituted with a fluorine atom, more preferably monofluoromethyl and difluoromethyl groups.

Therefore, "the alkyl group having from 1 to 4 carbon atoms which may be substituted with one or more halogen atoms" of R¹ as a whole includes preferably a methyl group which may be substituted with a fluorine atom, more preferably methyl, monofluoromethyl and difluoromethyl groups, most preferably a methyl group,
"The aliphatic acyl group having from 3 to 25 carbon atoms" of 2, R³ and R⁴ includes, for example, an alkylcarbonyl group such as propionyl, butyryl, isobutyryl, pentanoyl, pivaloyl, valeryl, isovaleryl, octanoyl, nonylcarbonyl, decylcarbonyl, 3-methylnonylcarbonyl, 8-methylnonylcarbonyl, 3-ethyloctylcarbonyl, 3,7-dimethyloctylcarbonyl, undecylcarbonyl, dodecylcarbonyl, tridecylcarbonyl, tetradecylcarbonyl, pentadecylcarbonyl, hexadecylcarbonyl, 1-methylpentadecylcarbonyl, 14-methylpentadecylcarbonyl, 13,13-dimethyltetradecylcarbonyl, heptadecylcarbonyl, 15-methylhexadecylcarbonyl, octadecylcarbonyl, 1-methylheptadecylcarbonyl, nonadecylcarbonyl, eicosylcarbonyl, tricosylcarbonyl and tetracosylcarbonyl, preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group.

R², R³ and R⁴ as a whole each are preferably a hydrogen atom or an aliphatic acyl group having from 6 to 25 carbon atoms, more preferably a hydrogen atom or an aliphatic acyl group having from 6 to 20 carbon atoms, particularly preferably a hydrogen atom or a hexanoyl, octanoyl, decanoyl, dodecanoyl, myristoyl, palmitoyl or stearoyl group.

As the combination of R², R³ and R⁴,
(a) the combination wherein R² is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and each of R³ and R⁴ is a hydrogen atom,
(b) the combination wherein R³ is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and each of R² and R⁴ is a hydrogen atom,
(c) the combination wherein R⁴ is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and each of R² and R³ is a hydrogen atom,
(d) the combination wherein each of R² and R³ is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and R⁴ is a hydrogen atom,
(e) the combination wherein each of R² and R⁴ is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and R³ is a hydrogen atom,
(f) the combination wherein each of R³ and R⁴ is an aliphatic acyl group having from 3 to 25 carbon atoms (preferably an aliphatic acyl group having from 6 to 25 carbon atoms, more preferably an aliphatic acyl group having from 6 to 20 carbon atoms, particularly preferably a hexanoyl, octanoyl, decanoyl, dodecanoyl, myristoyl, palmitoyl or stearoyl group), and R² is a hydrogen atom,
(g) the combination wherein each of R², R³ and R⁴ is an aliphatic acyl group having from 3 to 25 carbon atoms (preferably the aliphatic acyl group having from 6 to 25 carbon atoms, more preferably the aliphatic acyl group having from 6 to 20 carbon atoms, particularly preferably hexanoyl, octanoyl, decanoyl, dodecanoyl, myristoyl, palmitoyl and stearoyl groups), and
(h) the combination wherein each of R², R³ and R⁴ is a hydrogen atom.

Of these combinations, the combination of (a) or (h) is preferred.

"The ester residue" of W includes, for example, "an alkyl group" such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methylhexyl, 2-methylhexyl, 3-methylhexyl, 4-methylhexyl, 5-methylhexyl, 1-propylbutyl, 4,4-dimethylpentyl, octyl, 1-methylheptyl, 2-methylheptyl, 3-methylheptyl, 4-methylheptyl, 5-methylheptyl, 6-methylheptyl, 1-propylpentyl, 2-ethylhexyl, 5,5-dimethylhexyl, nonyl, 3-methyloctyl, 4-methyloctyl, 5-methyloctyl, 6-methyloctyl, 1-propylhexyl, 2-ethylheptyl, 6,6-dimethylheptyl, decyl, 1-methylnonyl, 3-methylnonyl, 8-methylnonyl, 3-ethyloctyl, 3,7-dimethyloctyl, 7,7-dimethyloctyl, undecyl, 4,8-dimethylnonyl, dodecyl, tridecyl, tetradecyl, pentadecyl, 3,7,11-trimethyldodecyl, hexadecyl, 4,8,12-trimethyltridecyl, 1-methylpentadecyl, 14-methylpentadecyl, 13,13-dimethyltetradecyl, heptadecyl, 15-methylhexadecyl, octadecyl, 1-methylheptadecyl, nonadecyl, eicosyl, 3,7,11,15-tetramethylhexadecyl, heneicosyl, docosyl, tricosyl and tetracosyl groups; "an alkenyl group" such as ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl and 5-hexenyl; "an alkynyl group" such as ethynyl, 2-propynyl, 1-methyl-2-propynyl, 2-methyl-2-propynyl, 2-ethyl-2-propynyl, 2-butynyl, 1-methyl-2-butynyl, 2-methyl-2-butynyl, 1-ethyl-2-butynyl, 3-butynyl, 1-methyl-2-butynyl, 2-methyl-3-butynyl, 1-ethyl-3-butynyl, 2-pentynyl, 1-methyl-2-pentynyl, 2-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl and 5-hexynyl; "a halogeno lower alkyl group" such as trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, 3-chloropropyl, 4-fluorobutyl, 6-iodohexyl and 2,2-dibromoethyl; "a hydroxy lower alkyl group" such as 2-hydroxyethyl, 2,3-dihydroxypropyl, 3-hydroxypropyl, 3,4-dihydroxybutyl and 4-hydroxybutyl; "an aliphatic acyl lower alkyl group" such as acetylmethyl; "a lower alkyl group substituted with from 1 to 3 aryls" such as benzyl, phenethyl, 3-phenylpropyl, α-naphthylmethyl, β-naphthylmethyl, diphenylmethyl, triphenylmethyl, 6-phenylhexyl, α-naphthyldiphenylmethyl and 9-anthrylmethyl; "an aralkyl group of which the aryl ring is substituted with lower alkyl, lower alkoxy, nitro, halogen, cyano or alkoxycarbonyl" such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, 4-cyanobenzyldiphenylmethyl, bis(2-nitrophenyl)methyl, piperonyl and 4-methoxycarbonylbenzyl; "a tri(alkyl and/or phenyl)silyl group" such as trimethylsilyl, triethylsilyl, isopropyldimethylsilyl, tert-butyldimethylsilyl, methyldiisopropylsilyl, methyldi-tert-butylsilyl, triisopropylsilyl, methyldiphenylsilyl, isopropyldiphenylsilyl, butyldiphenylsilyl and phenyldiisopropylsilyl; "a protecting group which is cleavable according to a biological method such as hydrolysis in a living body", that is, an ester which produces a free acid or a salt thereof by being hydrolized in a human body, for example, "a lower alkoxy lower alkyl group" such as methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, n-propoxymethyl, isopropoxymethyl, n-butoxymethyl and tert-butoxymethyl; "a lower alkoxylated lower alkoxy lower alkyl group" such as 2-methoxyethoxymethyl; "an aryloxy lower alkyl group" such as phenoxymethyl; "a halogenated lower alkoxy lower alkyl group" such as 2,2,2-trichloroethoxymethyl and bis(2-chloroethoxy)methyl; "a lower alkoxycarbonyl lower alkyl group" such as methoxycarbonylmethyl; "a cyano lower alkyl group" such as cyanomethyl and 2-cyanoethyl; "a lower alkylthiomethyl group" such as methylthiomethyl and ethylthiomethyl; "an arylthiomethyl group" such as phenylthiomethyl and naphthylthiomethyl; "a lower alkylsulfonyl lower alkyl group which may be substituted with halogen" such as 2-methanesulfonylethyl and 2-trifluoromethanesulfonylethyl; "an arylsulfonyl lower alkyl group" such as 2-benzenesulfonylethyl and 2-toluenesulfonylethyl; "an aliphatic acyloxy lower alkyl group" such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl; "a cycloalkylcarbonyloxy lower alkyl group" such as cyclopentanoyloxymethyl, cyclohexanoyloxymethyl, 1-cyclopentanoyloxyethyl, 1-cyclopentanoyloxypropyl, 1-cyclopentanoyloxypropyl, 1-cyclohexanoyloxypropyl, 1-cyclopentanoyloxybutyl and 1-cyclohexanoyloxybutyl; "an aromatic acyloxy lower alkyl group" such as benzoyloxymethyl; "an (alkoxycarbonyloxy)alkyl group" such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-propoxycarbonyloxyethyl, 1-(isopropoxycarbonyloxy)ethyl, 1-butoxycarbonyloxyethyl, 1-isobutoxycarbonyloxyethyl, 1-(tert-butoxycarbonyloxy)ethyl, 1-pentyloxycarbonyloxyethyl, 1-hexyloxycarbonyloxyethyl, 1-cyclopentyloxycarbonyloxyethyl, 1-cyclopentyloxycarbonyloxypropyl, 1-cyclohexyloxycarbonyloxypropyl, 1-cyclopentyloxycarbonyloxybutyl, 1-cyclohexyloxycarbonyloxybutyl, 1-(cyclohexyloxycarbonyloxy)ethyl, 1-(ethoxycarbonyloxy)propyl, 2-methoxycarbonyloxyethyl, 2-ethoxycarbonyloxyethyl, 2-propoxycarbonyloxyethyl, 2-isopropoxycarbonyloxyethyl, 2-butoxycarbonyloxyethyl, 2-isobutoxycarbonyloxyethyl, 2-pentyloxycarbonyloxyethyl, 2-hexyloxycarbonyloxyethyl, 1-methoxycarbonyloxypropyl, 1-ethoxycarbonyloxypropyl, 1-propoxycarbonyloxypropyl, 1-isopropoxycarbonyloxypropyl, 1-butoxycarbonyloxypropyl, 1-isobutoxycarbonyloxypropyl, 1-pentyloxycarbonyloxypropyl, 1-hexyloxycarbonyloxypropyl, 1-methoxycarbonyloxybutyl, 1-ethoxycarbonyloxybutyl, 1-propoxycarbonyloxybutyl, 1-isopropoxycarbonyloxybutyl, 1-butoxycarbonyloxybutyl, 1-isobutoxycarbonyloxybutyl, 1-methoxycarbonyloxypentyl, 1-ethoxycarbonyloxypentyl, 1-methoxycarbonyloxyhexyl and 1-ethoxycarbonyloxyhexyl; "an oxodioxolenylmethyl group" such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl; "a phthalidyl group" such as phthalidyl, dimethylphthalidyl and dimethoxyphthalidyl; "an aryl group" such as phenyl and indanyl; and "a carboxyalkyl group" such as carboxymethyl, preferably "an alkyl group", more preferably an alkyl group having from 1 to 18 carbon atoms.

In the case where at least one of R², R³ and R⁴ is an aliphatic acyl group having from 3 to 25 carbon atoms, "the ester residue" of W is preferably an'alkyl group having from 1 to 18 carbon atoms. In this case, W is, as a whole, preferably a hydrogen atom or an alkyl group having from I to 18 carbon atoms, more preferably a hydrogen atom.

In the case where each of R², R³ and R⁴ is a hydrogen atom, "the ester residue" of W is preferably an alkyl group having from 1 to 18 carbon atoms, more preferably an alkyl group having from 6 to 18 carbon atoms. In this case, W is, as a whole, preferably an ester residue, more preferably an alkyl group having from 6 to 18 carbon atoms.

"The pharmacologically acceptable salt thereof' includes alkali metal salts such as salts of sodium, potassium and lithium, alkali earth metal salts such as salts of calcium and magnesium, metal salts such as salts of aluminium, iron, zinc, copper, nickel and cobalt; inorganic amine salts such as ammonium salts, organic amine salts such as salts of t-octylamine, dibenzylamine, morpholine, glucosamine, phenylglycinealkyl ester, ethylenediamine, N-methylglucamine, guanidine, diethylamine, triethylamine, dicyclohexylamine, N,N'-dibenzylethylenediamine, chloroprocaine, procaine, diethanolamine, N-benzylphenethylamine, piperazine, tetramethylammonium and tris(hydroxymethyl)aminomethane; halogenated hydroacid salts such as hydrofluoride, hydrochloride, hydrobromide and hydroiodide, inorganic acid salts such as nitrate, perchlorate, sulfate and phosphate; organic acid salts including lower alkanesulfonates such as methanesulfonate, trifluoromethanesulfonate and ethanesulfonate, arylsulfonates such as benzenesulfonate and p-toluenesulfonate, acetate, trifluoroacetate, malate, fumarate, succinate, citrate, tartrate, oxalate and maleate; and amino acid salts such as glycine salt, lysine salt, arginine salt, ornithine salt, glutamate and aspartate, preferably alkali metal salts such as sodium, potassium and lithium salts, and organic acid salts such as acetate and trifluoroacetate, and inorganic acid salts such as hydrochloride and sulfate.

Of the compounds of the present invention, the preferable one includes the following:
(1) compounds in which R¹ is a methyl group which may be substituted with one or more fluorine atoms,
(2) compounds in which R¹ is a methyl, monofluoromethyl or difluoromethyl group,
(3) compounds in which R¹ is a methyl group,
(4) compounds in which R² is a hydrogen atom or an aliphatic acyl group having from 6 to 25 carbon atoms,
(5) compounds in which R² is a hydrogen atom or an aliphatic acyl group having from 6 to 20 carbon atoms,
(6) compounds in which R² is a hydrogen atom, or a hexanoyl, octanoyl, decanoyl, dodecanoyl, myristoyl, palmitoyl or stearoyl group,
(7) compounds in which R³ is a hydrogen atom or an aliphatic acyl group having from 6 to 25 carbon atoms,
(8) compounds in which R³ is a hydrogen atom or an aliphatic acyl group having from 6 to 20 carbon atoms,
(9) compounds in which R³ is a hydrogen atom, or a hexanoyl, octanoyl, decanoyl, dodecanoyl, myristoyl, palmitoyl or stearoyl group,
(10) compounds in which R⁴ is a hydrogen atom or an aliphatic acyl group having from 6 to 25 carbon atoms,
(11) compounds in which R⁴ is a hydrogen atom or an aliphatic acyl group having from 6 to 20 carbon atoms,
(12) compounds in which R⁴ is a hydrogen atom, or a hexanoyl, octanoyl, decanoyl, dodecanoyl, myristoyl, palmitoyl or stearoyl group,
(13) compounds in which R² is an aliphatic acyl group having from 3 to 25 carbon atoms, and each of R³ and R⁴ is a hydrogen atom,
(14) compounds in which R² is an aliphatic acyl group having from 6 to 25 carbon atoms, and each of R³ and R⁴ is a hydrogen atom,
(15) compounds in which R² is an aliphatic acyl group having from 6 to 20 carbon atoms, and each of R³ and R⁴ is a hydrogen atom,
(16) compounds in which R² is a hexanoyl, octanoyl, decanoyl, dodecanoyl, myristoyl, palmitoyl or stearoyl group, and each of R³ and R⁴ is a hydrogen atom,
(17) compounds in which W is a hydrogen atom or an alkyl group having from 1 to 18 carbon atoms,
(18) compounds in which W is a hydrogen atom,
(19) compounds in which W is an ester residue,
(20) compounds in which W is an alkyl group having from 6 to 18 carbon atoms.
   Further, the compounds obtained by combining the substituents R¹, R², R³, R⁴ and W selected in the compounds of the above (1) to (20) are more preferable and includes, for example, the following compounds.
(21) compounds in which each of R², R³ and R⁴ is a hydrogen atom, and W is an ester residue,
(22) compounds in which each of R², R³ and R⁴ is a hydrogen atom, and W is an alkyl group having from 6 to 18 carbon atoms,
(23) compounds in which R¹ is a methyl group which may be substituted with one or more fluorine atoms, R² is an aliphatic acyl group having from 3 to 25 carbon atoms, each of R³ and R⁴ is a hydrogen atom, and W is a hydrogen atom or an ester residue,
(24) compounds in which R¹ is a methyl group, R² is an aliphatic acyl group having from 6 to 25 carbon atoms, each of R³ and R⁴ is a hydrogen atom, and W is a hydrogen atom or an alkyl group having from 1 to 18 carbon atoms,
25) compounds in which R¹ is a methyl group, R² is an aliphatic acyl group having from 6 to 20 carbon atoms, and each of R³, R⁴ and W is a hydrogen atom,
(26) compounds in which R¹ is a methyl group which may be substituted with one or more fluorine atoms, each of R², R³ and R⁴ is a hydrogen atom, and W is an ester residue, and
(27) compounds in which R¹ is a methyl group, each of R², R³ and R⁴ is a hydrogen atom, and W is an alkyl group having from 6 to 18 carbon atoms.

In the following, the compounds of the present invention are exemplified but the present invention is not limited to these. Compounds 1, 179 & 358 are comparative examples.

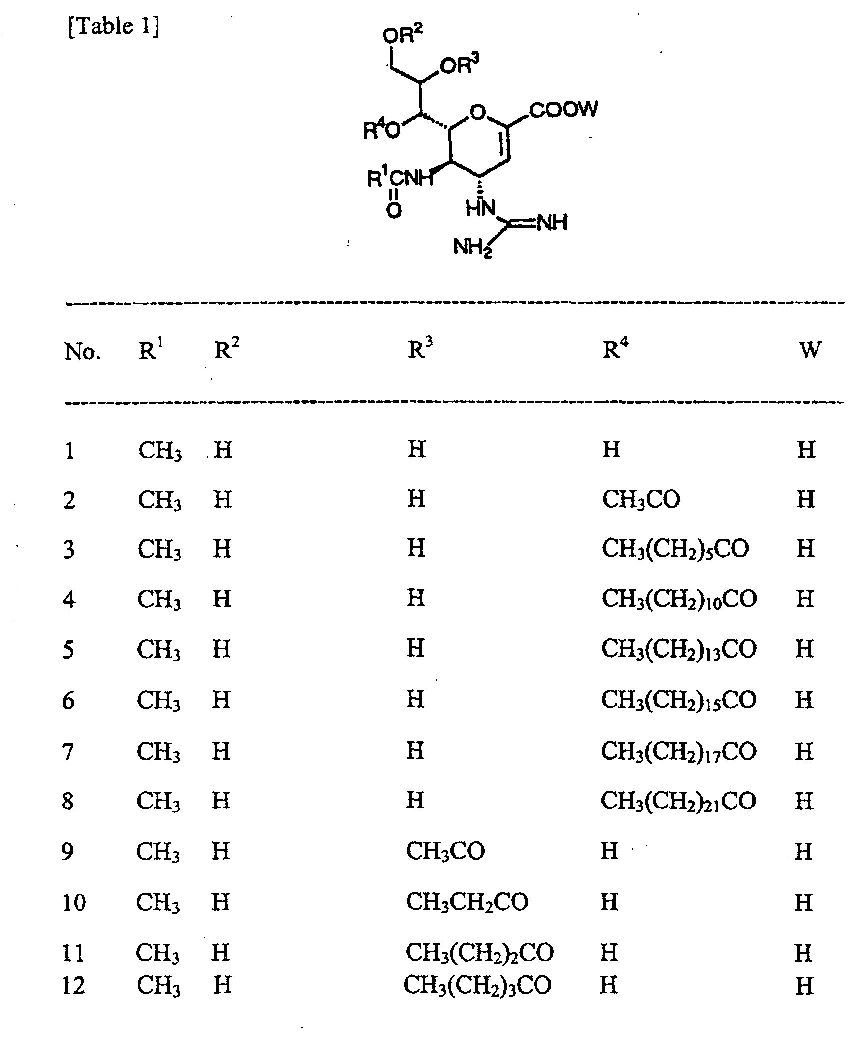

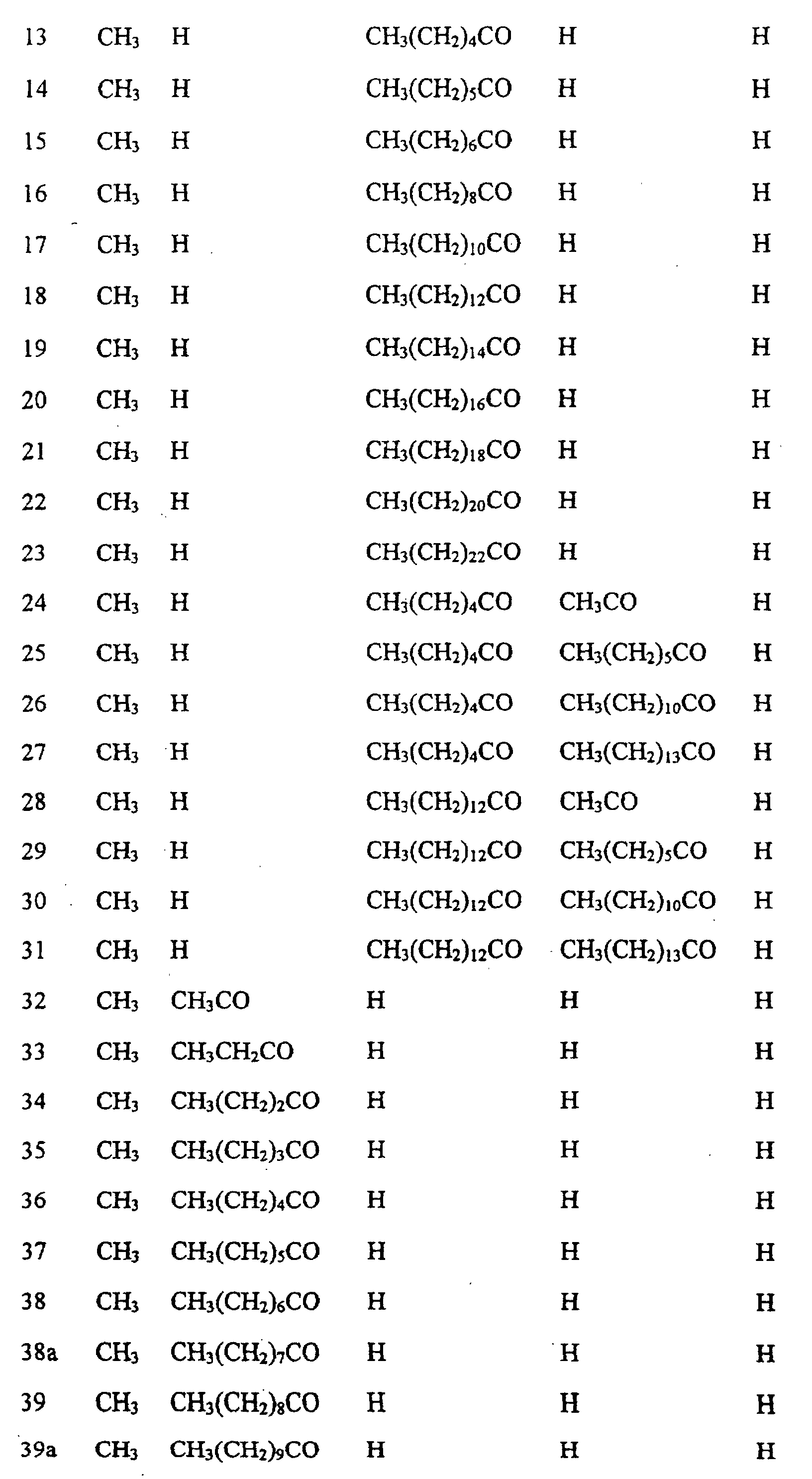

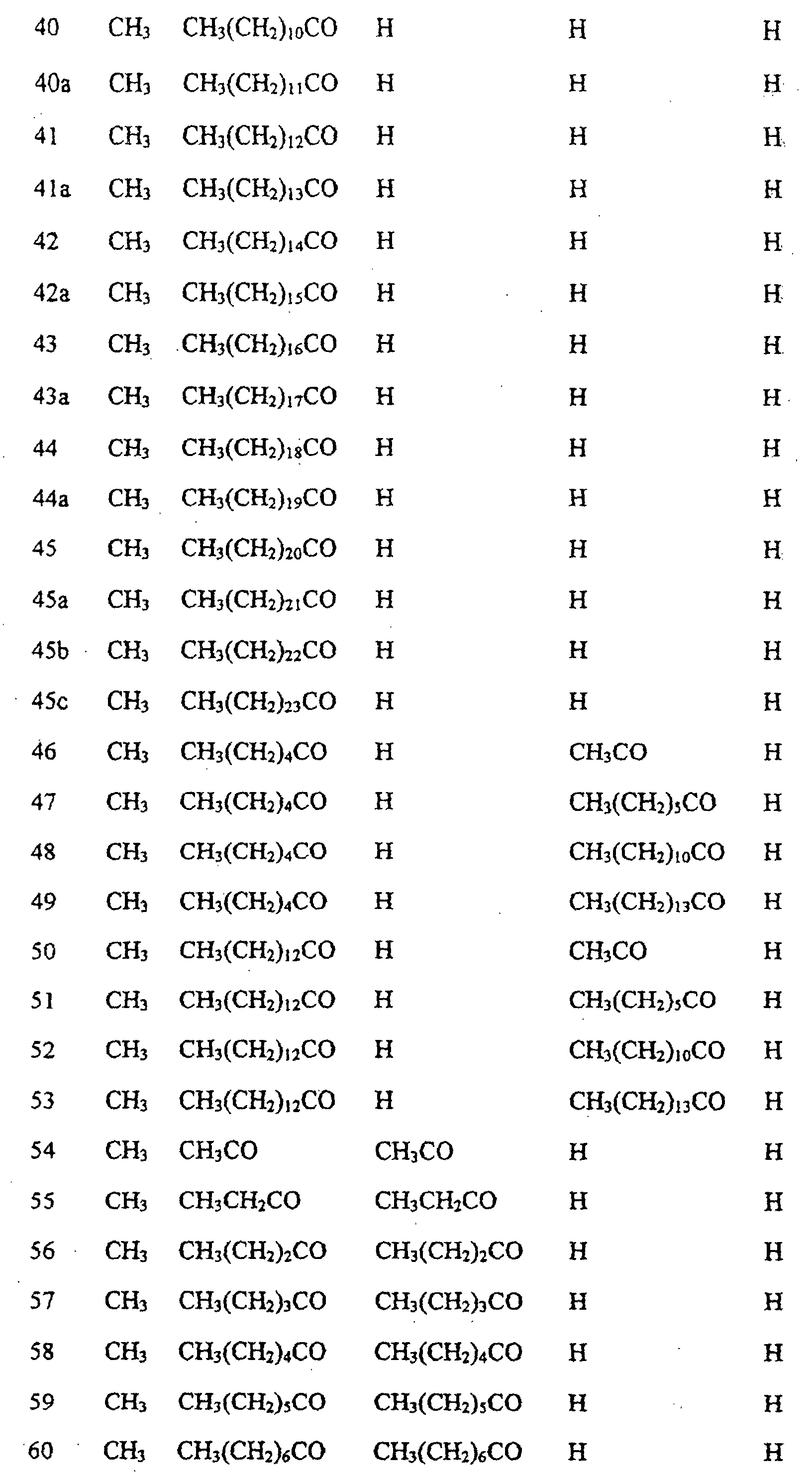

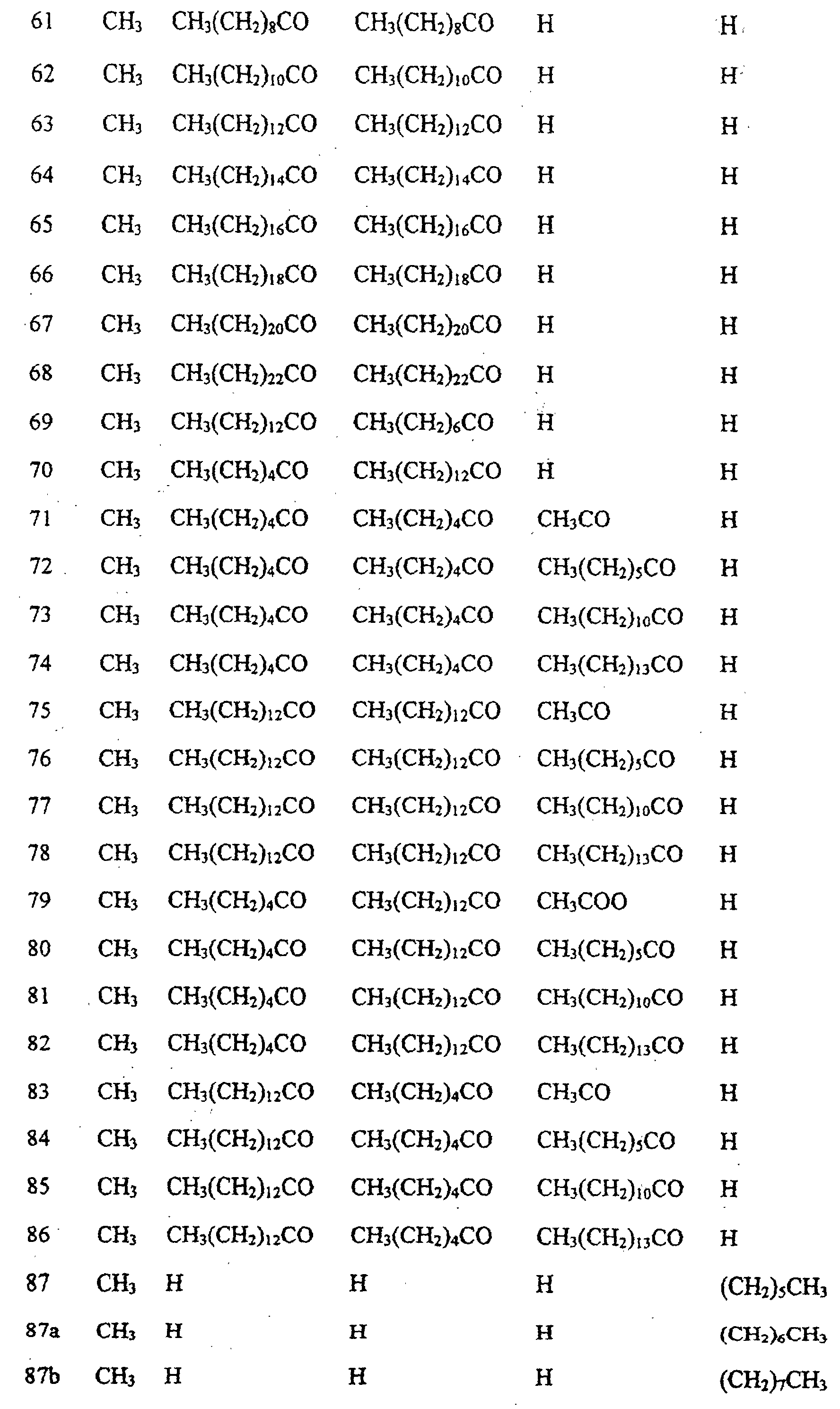

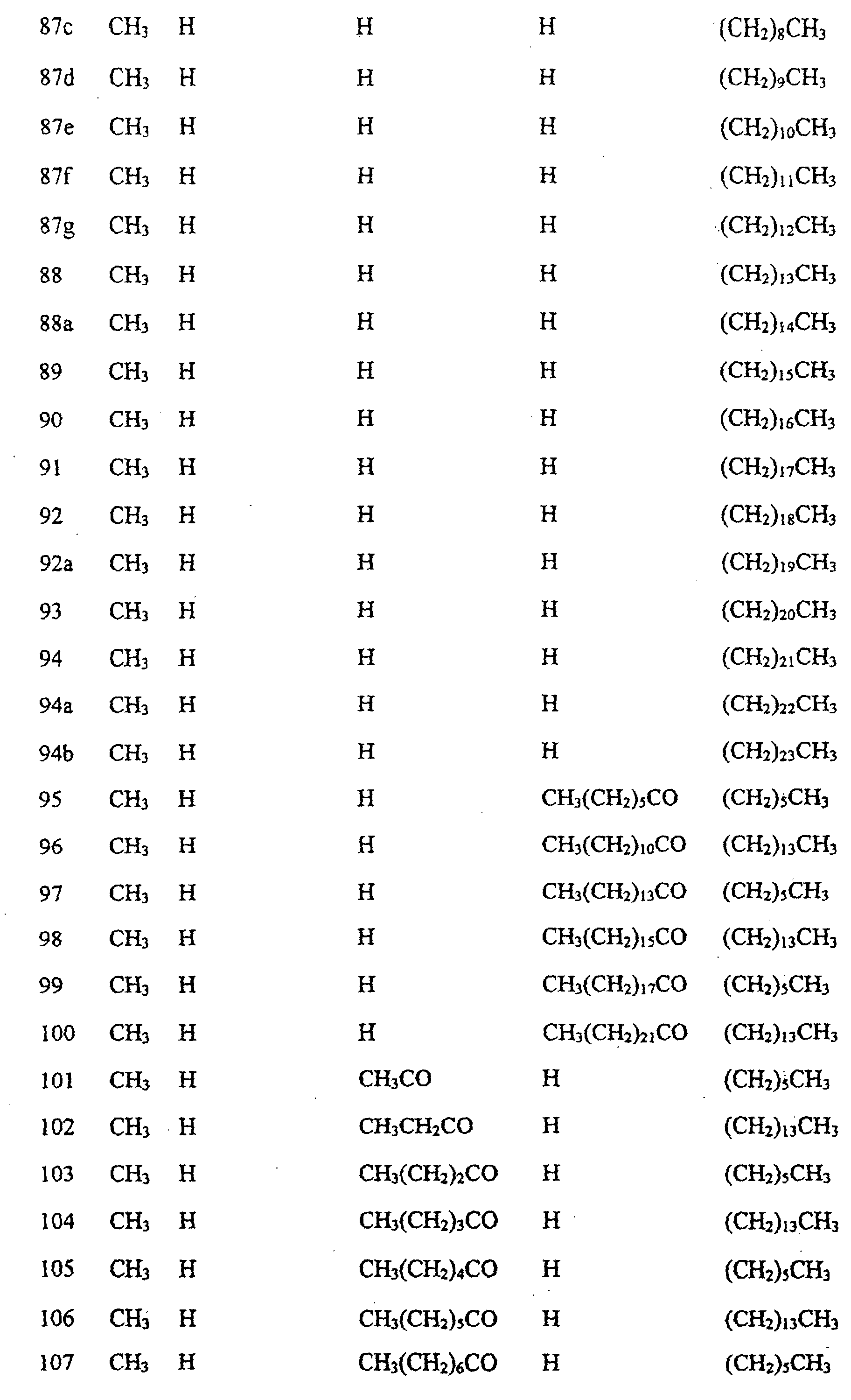

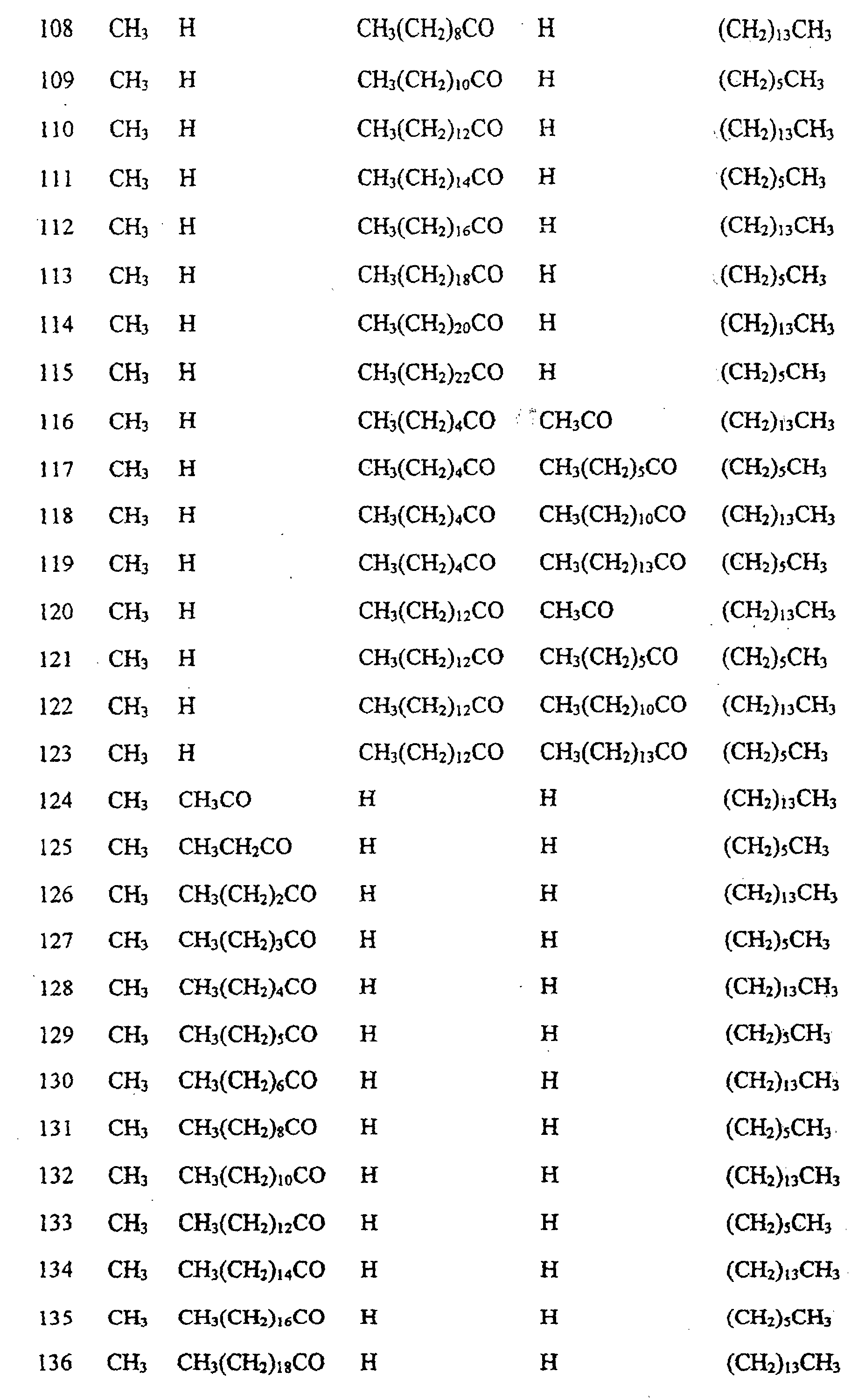

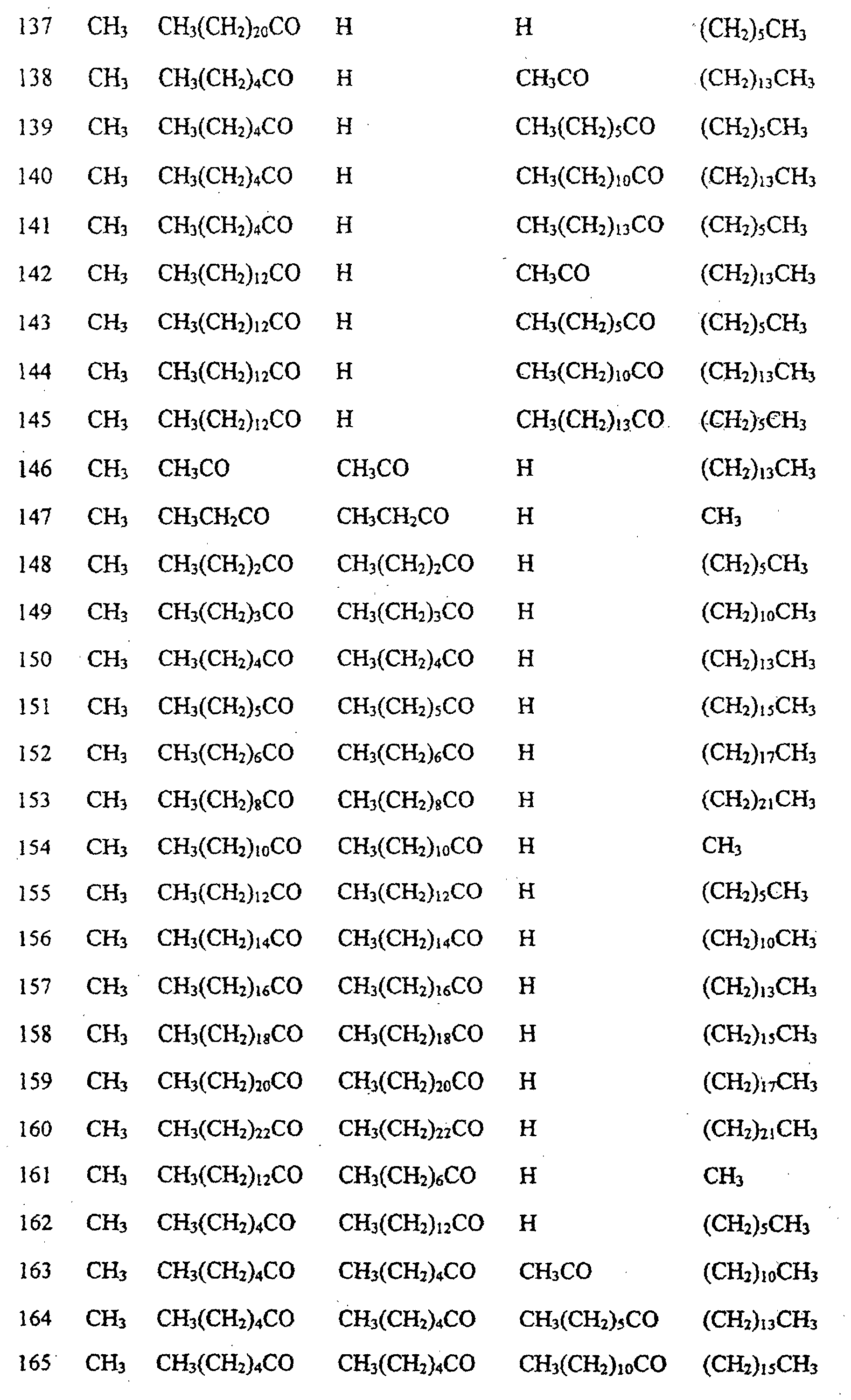

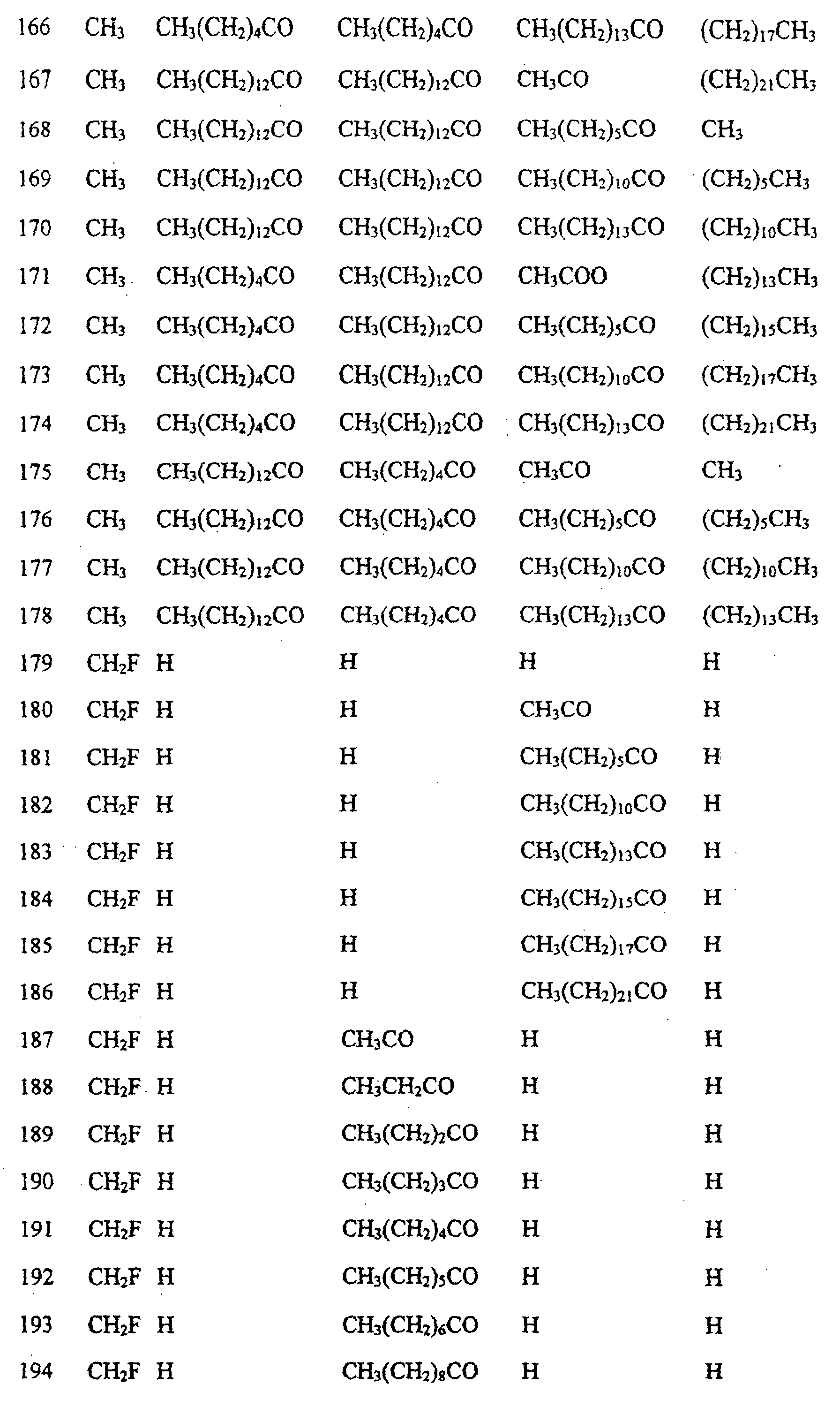

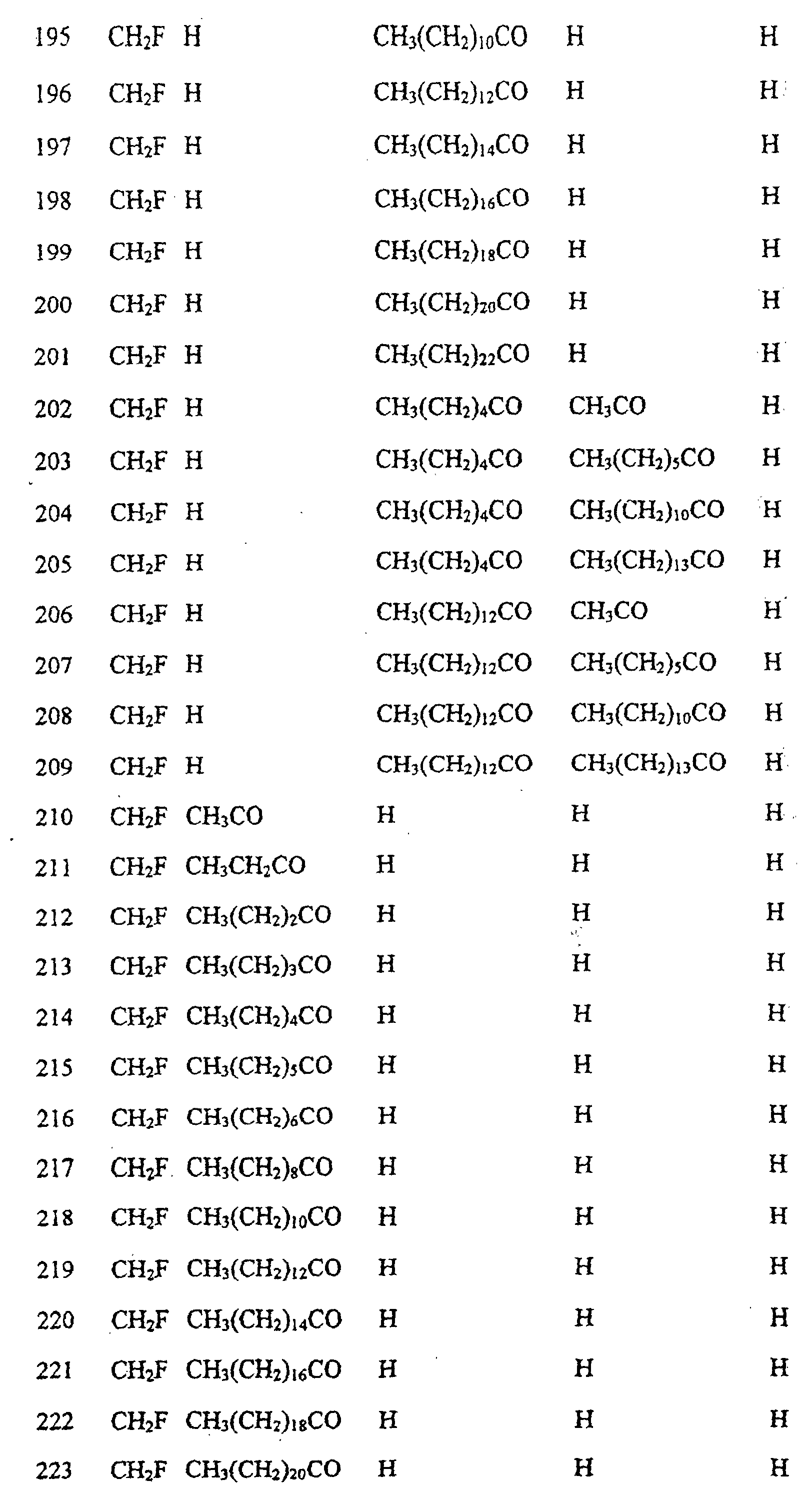

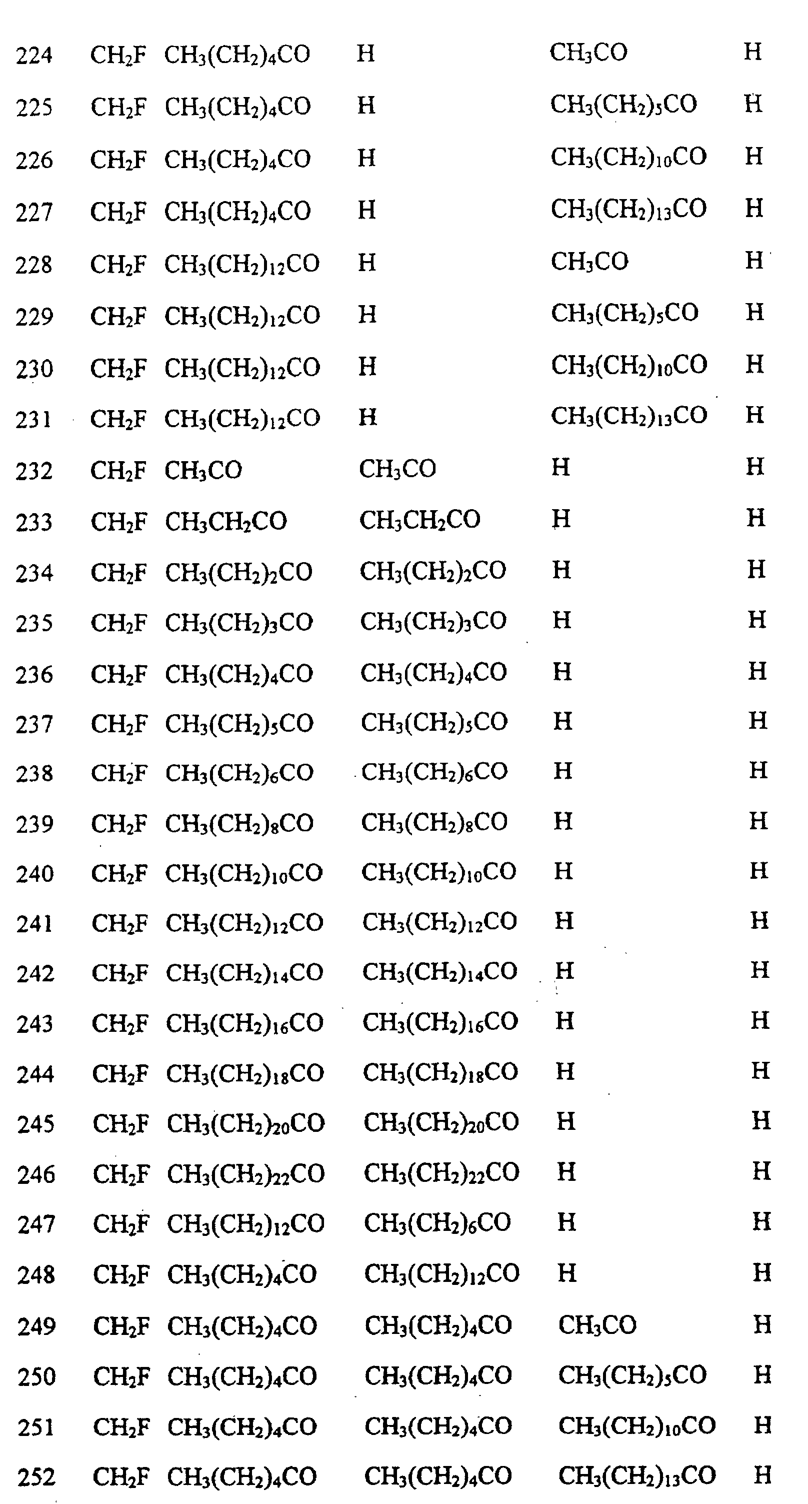

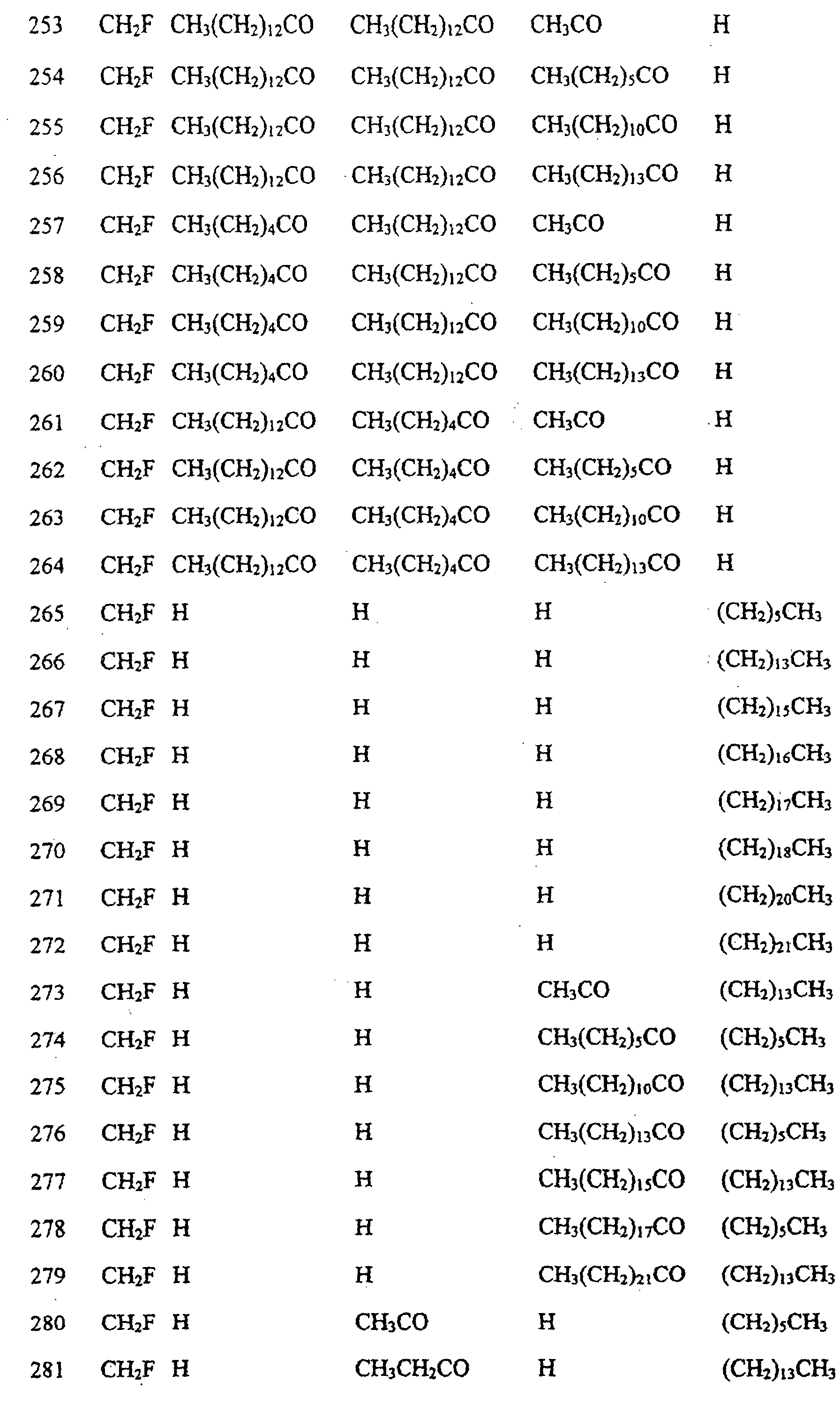

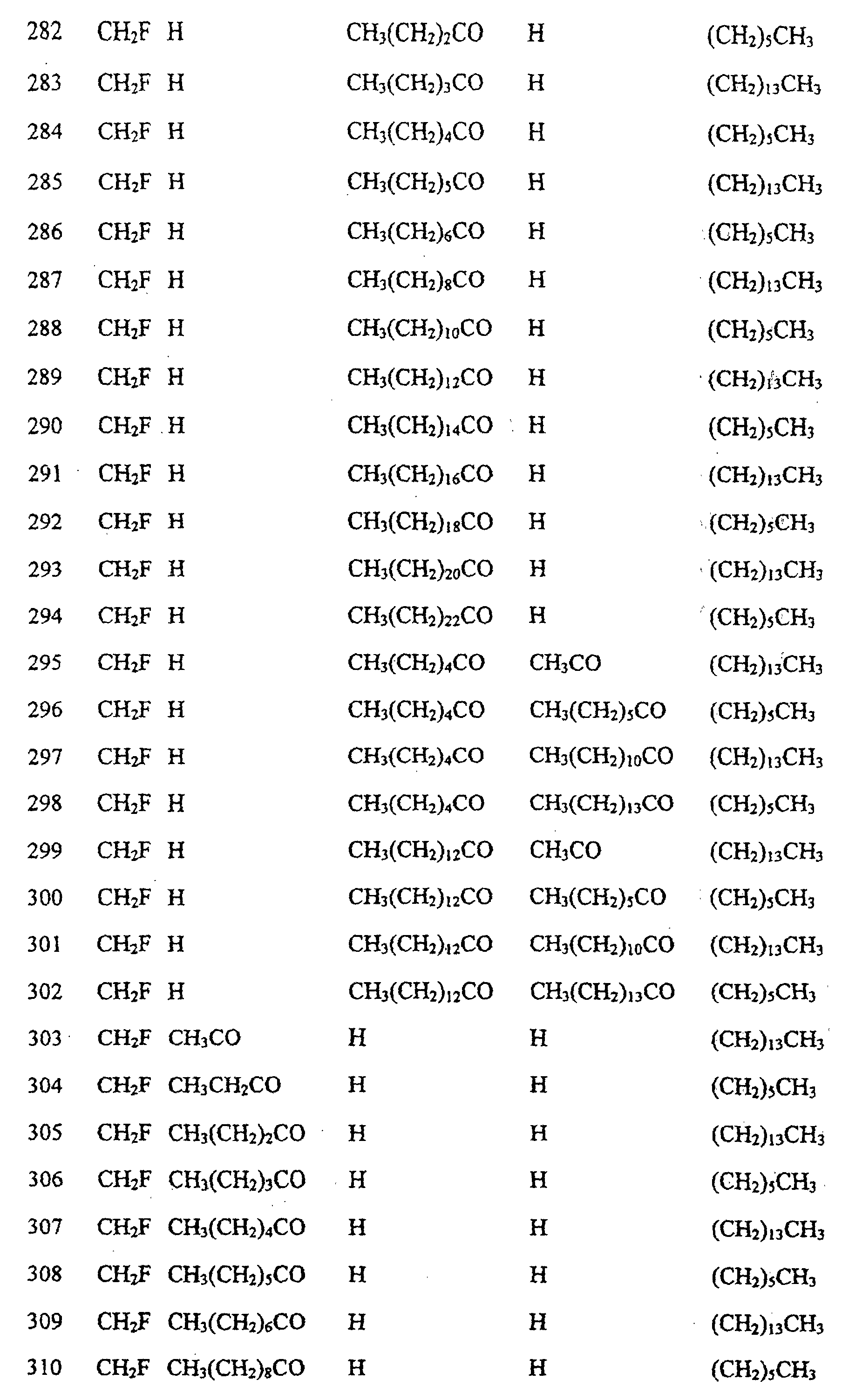

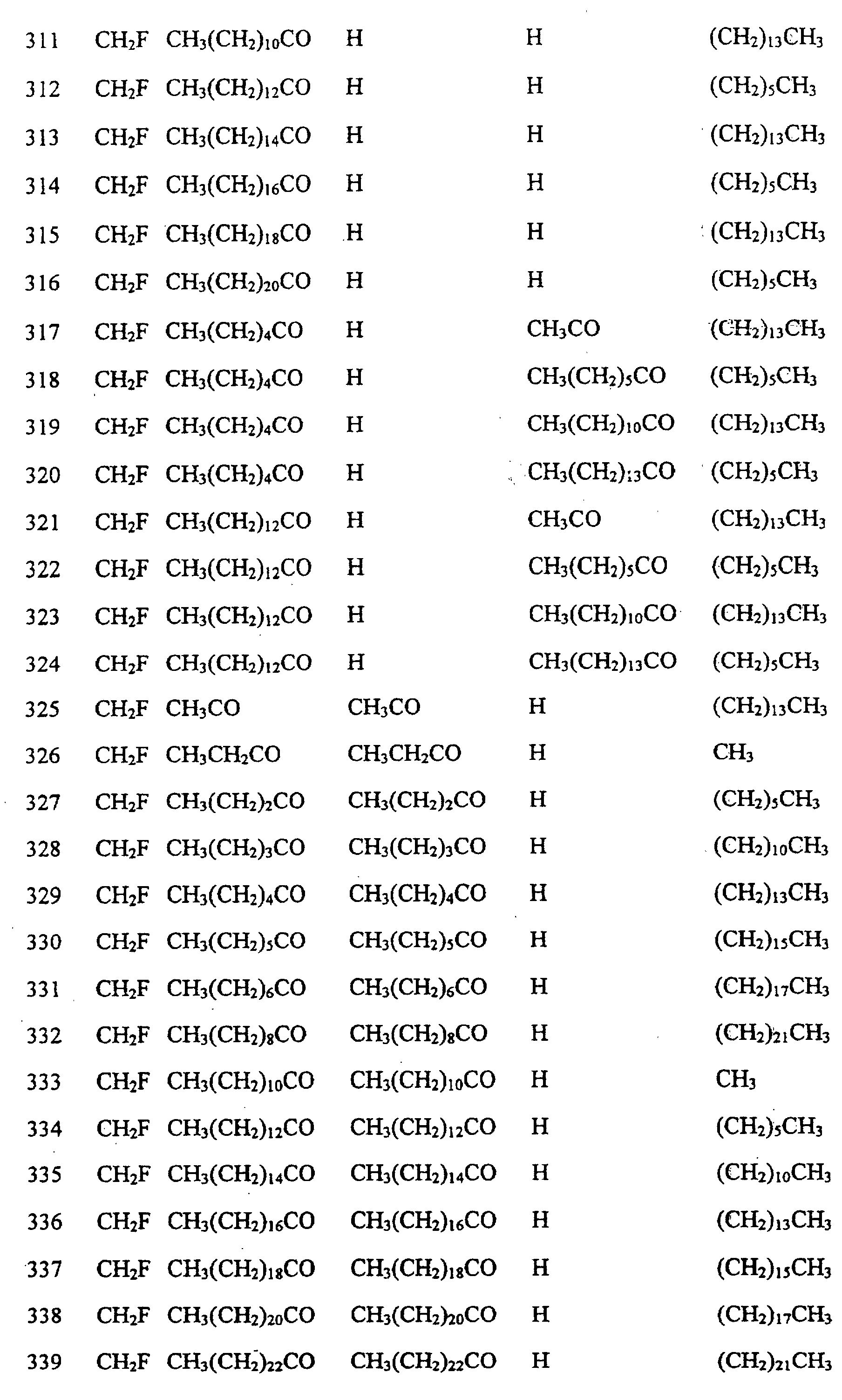

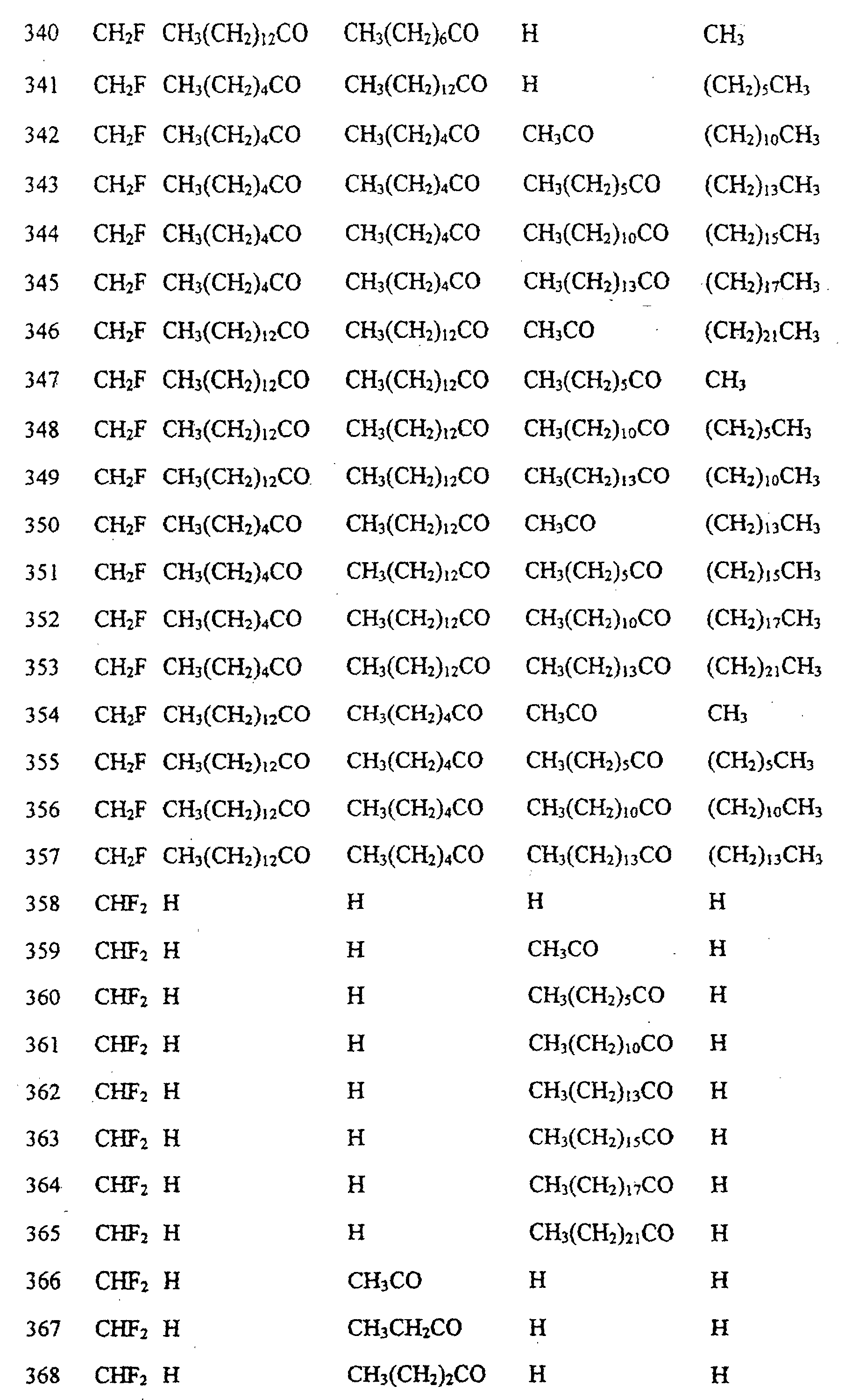

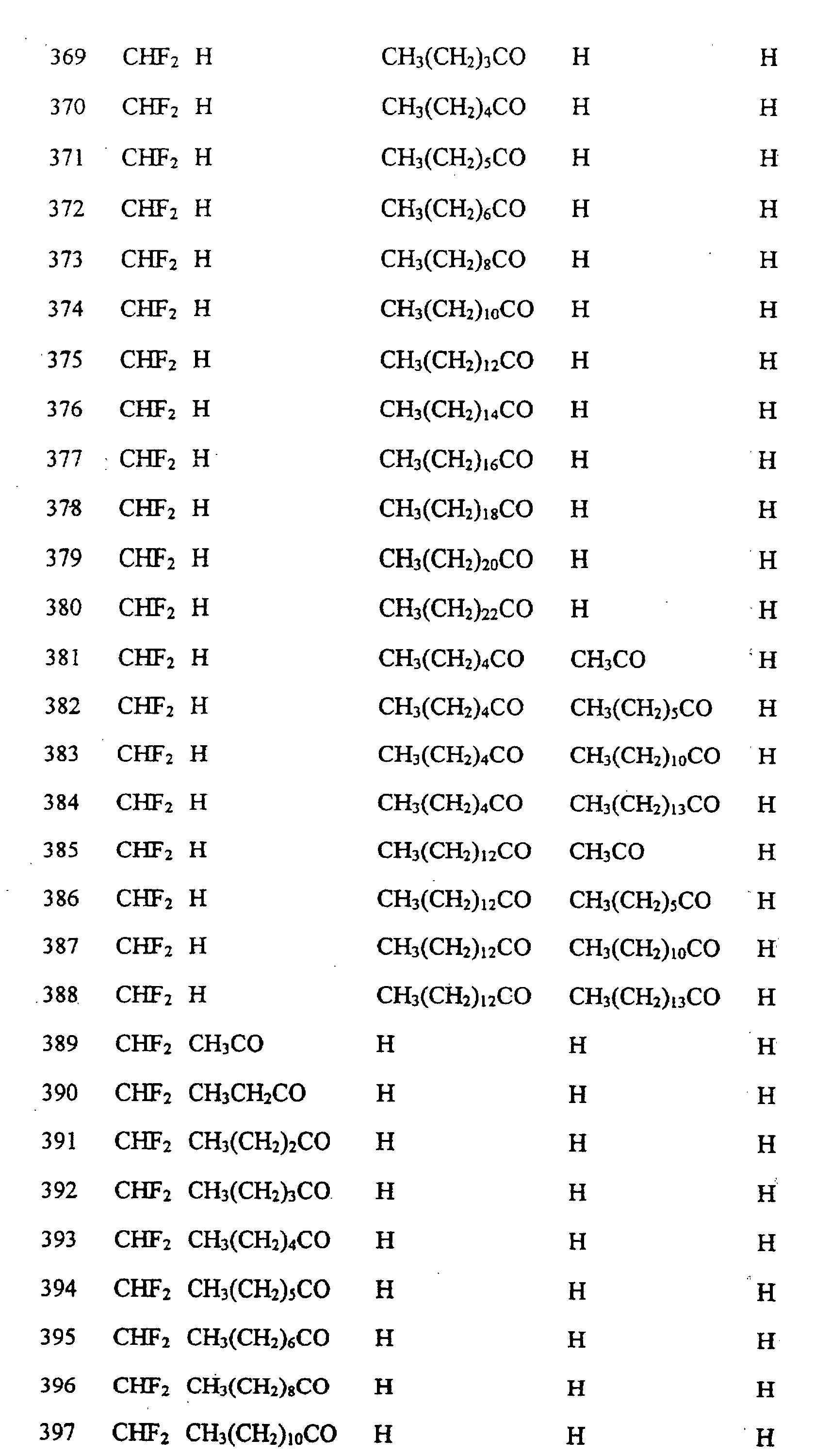

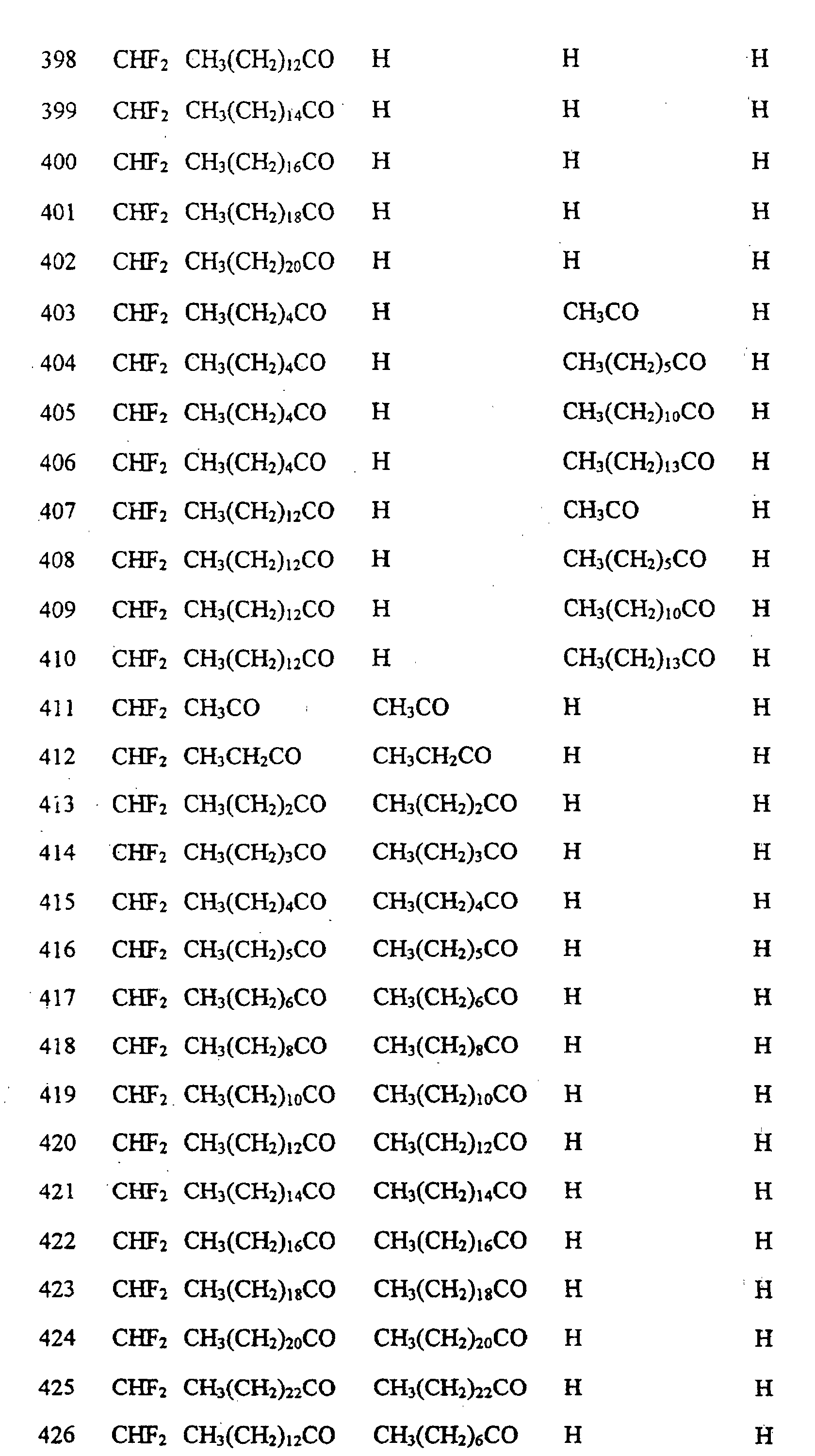

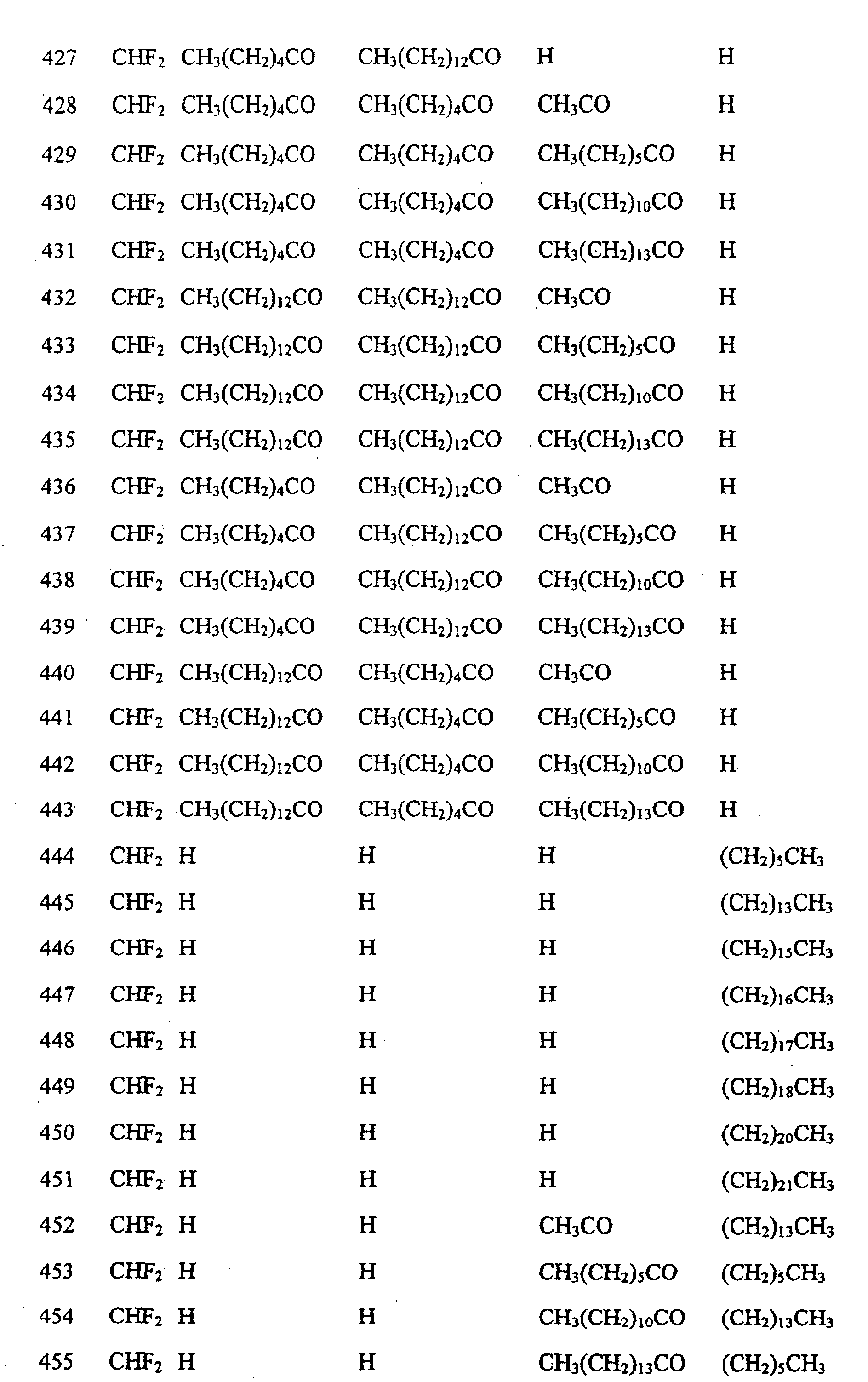

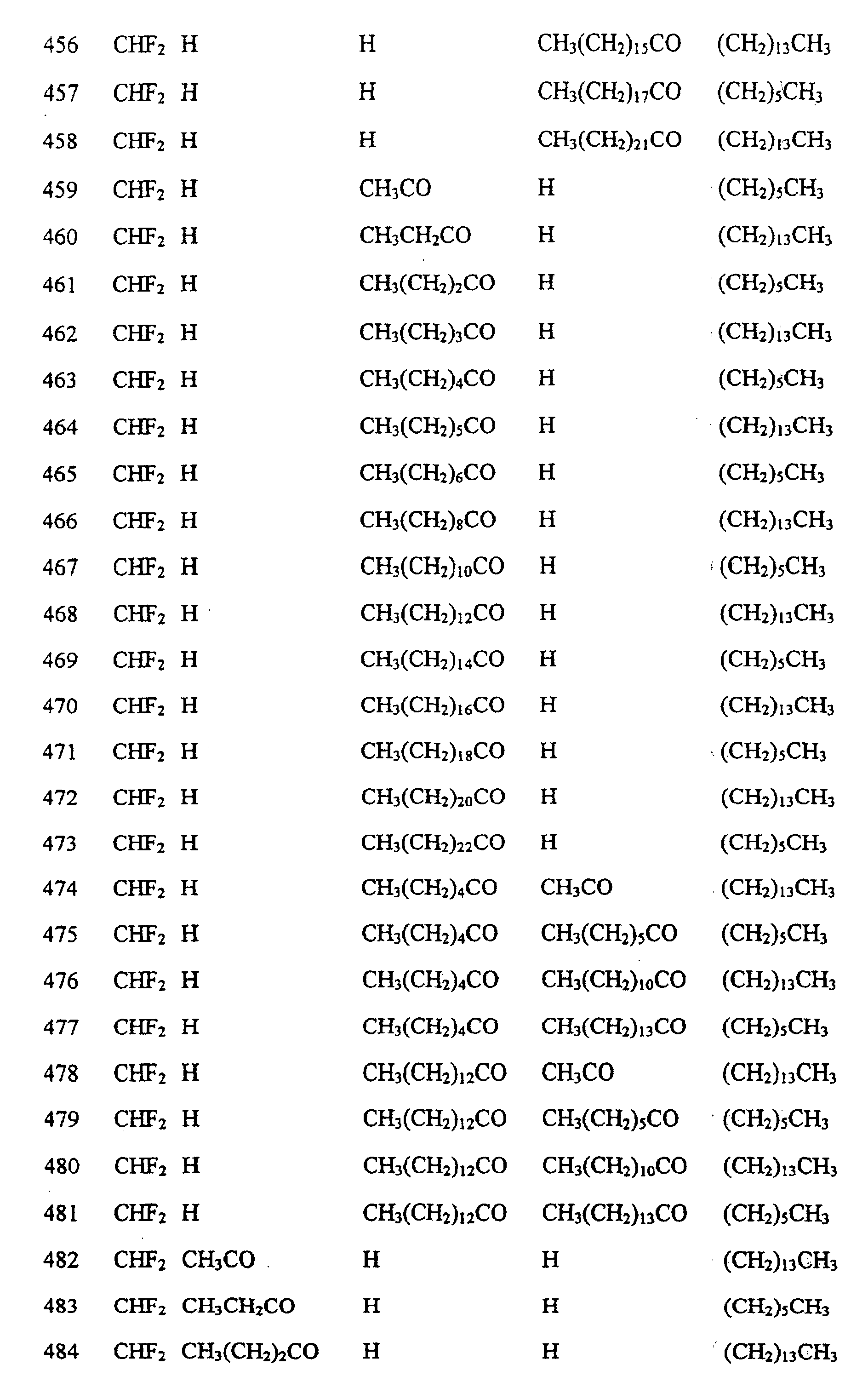

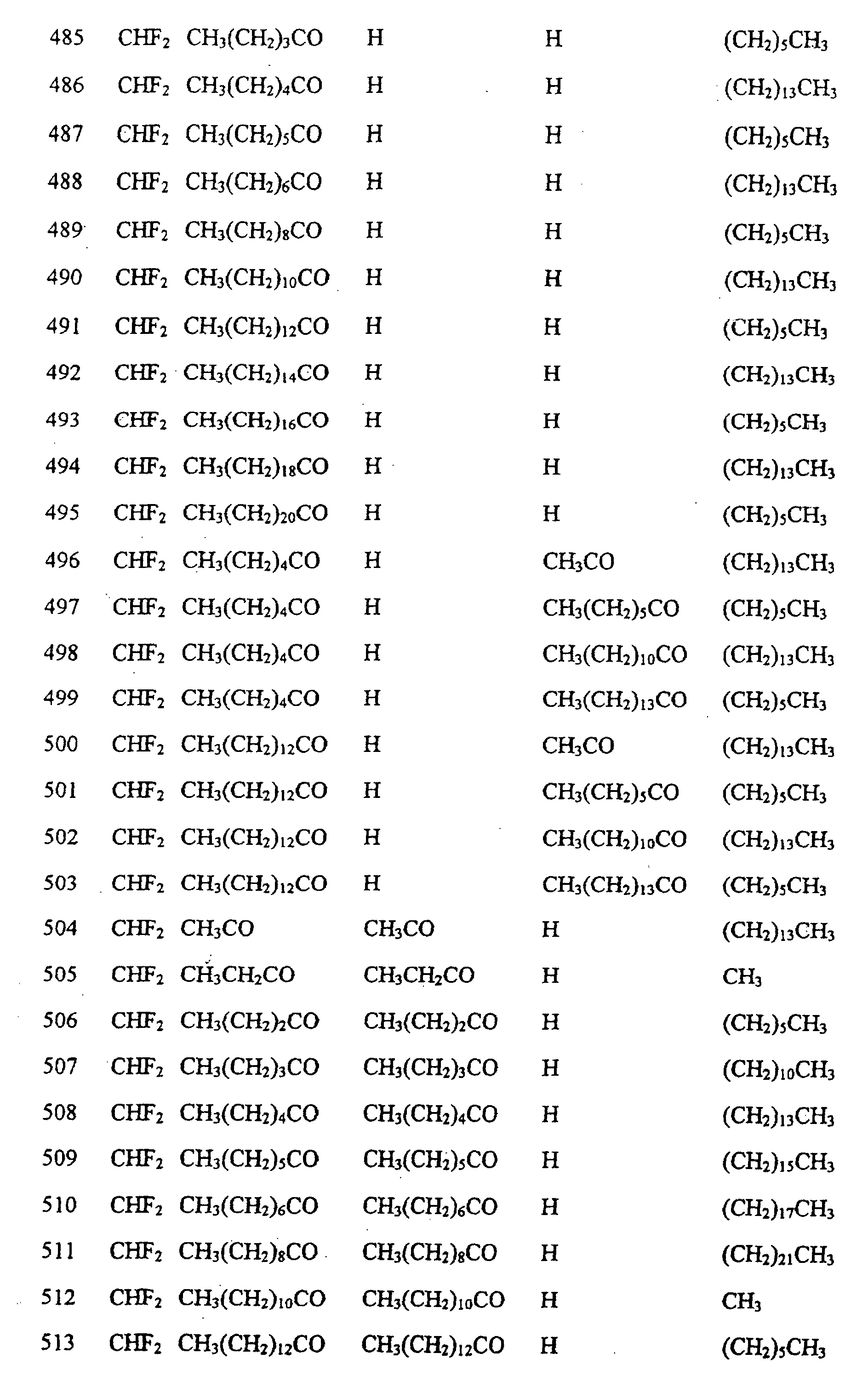

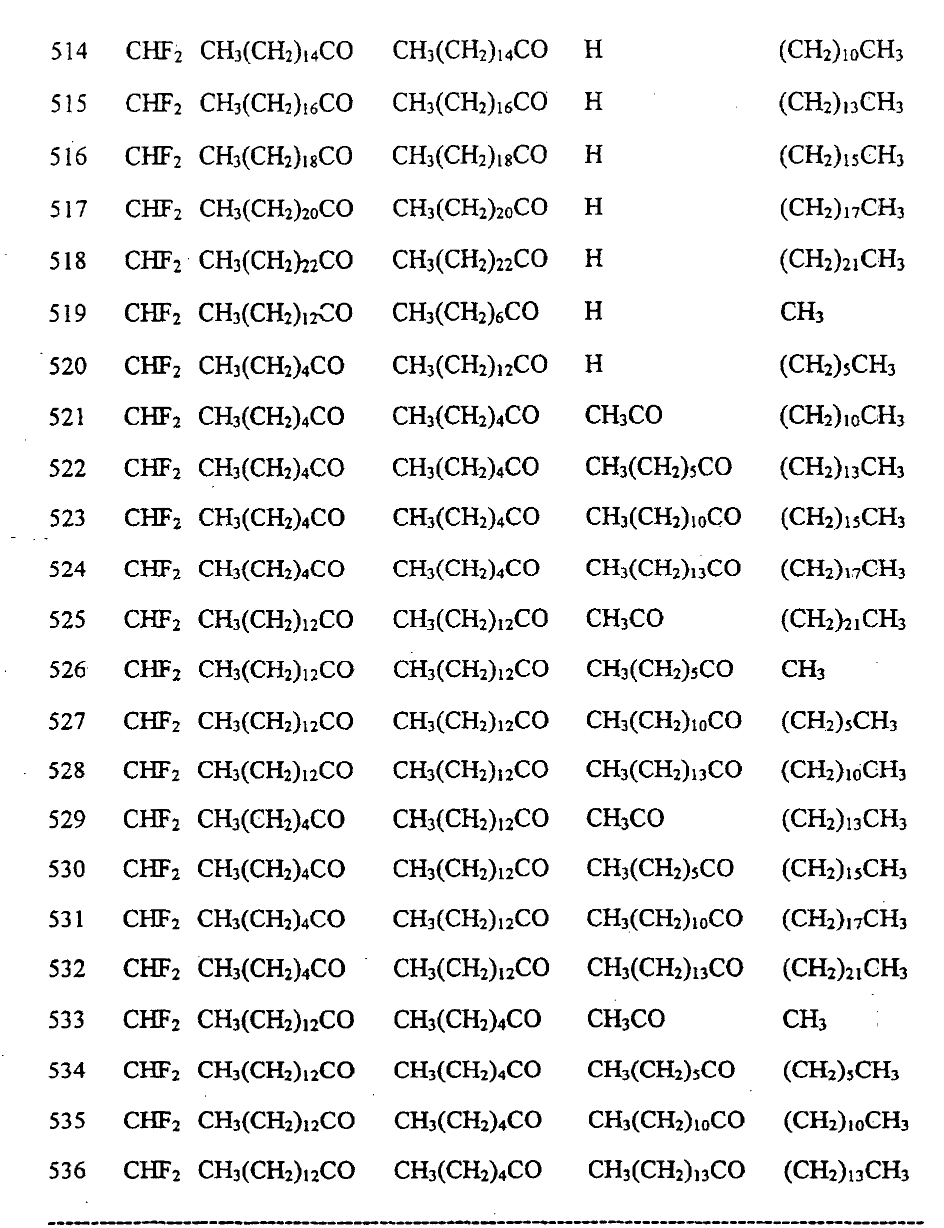

Of the above exemplary compounds, preferred are Compounds 36, 37, 38, 38a, 39, 39a, 40, 40a, 41, 41a, 42, 42a, 43, 43a, 44, 44a, 45, 45a, 45b, 45c, 87, 87a, 87b, 87c, 87d, 87e, 87f, 87g, 88, 88a, 89, 90, 91, 92, 92a, 93, 94, 94a, 94b, 214, 215, 216, 217, 218, 219, 220, 221, 222, 223, 265, 266, 267, 268, 269, 270, 271, 272, 393, 394, 395, 396, 397, 398, 399, 400, 401, 402, 444, 445, 446, 447, 448, 449, 450 and 451.

More preferred are Compounds 36, 37, 38, 38a, 39, 39a, 40, 40a, 41, 41a, 42, 42a, 43, 43a, 44, 44a, 45, 45a, 45b, 45c, 87, 87a, 87b, 87c, 87d, 87e, 87f, 87g, 88, 88a, 89, 90, 91, 92, 92a, 93, 94, 94a, 94b, 219, 220, 221, 222, 269, 270, 271, 272, 398, 399, 400, 401, 448, 449, 450 and 451.

The following compounds are most preferred.
5-acetamido-2,3,4,5-tetradeoxy-4-guanidino-9-O-hexanoyl-D-glycero-D-galacto-non-2-enopyranosoic acid (Exemplary compound No. 36)
5-acetamido-2,3,4,5-tetradeoxy-4-guanidino-9-O-octanoyl-D-glycero-D-galacto-non-2-enopyranosoic acid (Exemplary compound No. 38)
5-acetamido-2,3,4,5-tetradeoxy-4-guanidino-9-O-decanoyl-D-glycero-D-galacto-non-2-enopyranosoic acid (Exemplary compound No. 39)
5-acetamido-2,3,4,5-tetradeoxy-4-guanidino-9-O-dodecanoyl-D-glycero-D-galacto-non-2-enopyranosoic acid (Exemplary compound No. 40)
5-acetamido-2,3,4,5-tetradeoxy-4-guanidino-9-O-myristoyl-D-glycero-D-galacto-non-2-enopyranosoic acid (Exemplary compound No. 41)
5-acetamido-2,3,4,5-tetradeoxy-4-guanidine-9-O-palmitoyl-D-glycero-D-galacto-non-2-enopyranosoic acid (Exemplary compound No. 42)
5-acetamido-2,3,4,5-tetradeoxy-4-guanidino-9-O-stearoyl-D-glycero-D-galacto-non-2-enopyranosoic acid (Exemplary compound No. 43)
hexyl 5-acetamido-2,3,4,5-tetradeoxy-4-guanidino-D-glycero-D-galacto-non-2-enopyranosoate (Exemplary compound No. 87)
myristyl 5-acetamido-2,3,4,5-tetradeoxy-4-guanidino-D-glycero-D-galacto-non-2-enopyranosoate (Exemplary compound No. 88)
cetyl 5-acetamido-2,3,4,5-tetradeoxy-4-guanidino-D-glycero-D-galacto-non-2-enopyranosoate (Exemplary compound No. 89)
stearyl 5-acetamido-2,3,4,5-tetradeoxy-4-guanidino-D-glycero-D-galacto-non-2-enopyranosoate (Exemplary compound No. 91)

In the following, the process for preparing the compound (1) of the present invention will be described.

The compound (1) of the present invention can be prepared according to the process described in Process A, B or C shown below. Further, the compound (1) can be also prepared according to Process J described later.

The raw material compound (2) used in Processes A and B can be prepared according to the processes described in Process D, E, F or G shown below. Further, the raw material compound (5) used in Process C can be prepared according to the process described in Process H shown below.

The meanings of R¹, R², R^{2a}, R^{2b}, R³, R^{3a}, R⁴, R^{4a}, R⁶, R⁷, R⁸, W, W^{a}, Me, Ac and Boc used in the steps of Processes A to J are shown below.

That is, R¹, R², R³, R⁴ and W have the same meanings as defined above,
R^{2a} has the same meaning as defined for the above R² or represents a protecting group of the hydroxyl group (preferably a t-butyldimethylsilyl group or an isopropylidene group taken together with the protecting group of the hydroxyl group of R^{3a}),
R^{2b} represents a protecting group of the hydroxyl group (preferably a t-butyldimethylsilyl group),
R^{3a} has the same meaning as defined for the above R³ or represents a protecting group of the hydroxyl group (preferably a t-butyldimethylsilyl group or an isopropylidene group taken together with the protecting group of the hydroxyl group of R^{2a}),
R^{4a} has the same meaning as defined for the above R⁴ or represents a protected hydroxyl group (preferably a t-butyldimethylsilyl group),
R⁶, R⁷ and R⁸ may be the same or different and each represent an aliphatic acyl group having from 3 to 25 carbon atoms,
W^{a} has the same meaning as defined for the above W or represents a protecting group of the carboxyl group (preferably a methyl, ethyl, benzyl, allyl, methoxymethyl, methylthiomethyl, 2-(trimethylsilyl)ethoxymethoxy or diphenylmethyl group, more preferably a methyl, benzyl or diphenylmethyl group),
Ac represents an acetyl group,
Boc represents a t-butoxycarbonyl group, and
Me represents a methyl group.

In the following, each Process will be described in detail.

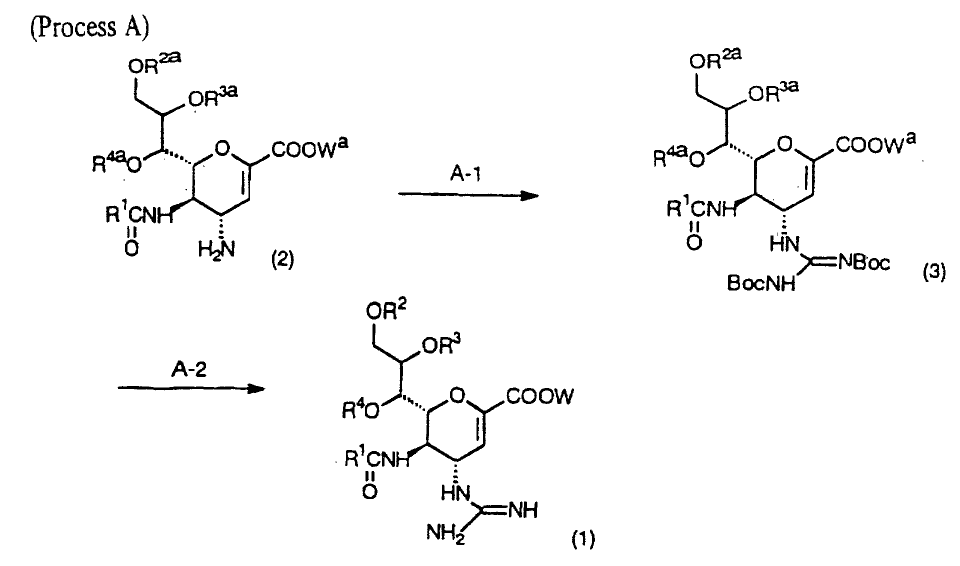

Process A is a process for preparing the compound (1) of the present invention by eliminating the protecting group of the compound (3) obtained by reacting the raw material compound (2) which is easily available according to the method described later with N,N'-di-t-butoxycarbonylthiourea.

### (Step A-1)

The present step is to prepare the compound (3) by reacting the compound (2) with N,N'-di-t-butoxycarbonylthiourea in an inert solvent in the presence of a base and mercuric chloride.

The solvent employable is not particularly limited so long as it does not affect the reaction and includes aromatic hydrocarbons such as benzene, toluene and xylene; ethers such as diethyl ether, diisopropyl ether, tetrahydrofuran, dioxane, dimethoxyethane and diethylene glycol dimethyl ether; and amides such as N,N-dimethylacetamide and dimethylformamide, preferably amides (particularly N,N-dimethylacetamide and dimethylformamide).

The base employable here includes preferably organic bases such as triethylamine and dimethylaminopyridine.

The reaction temperature is usually from -10 to 50°C, preferably from 10 to 30°C.

The reaction time varies depending on the material used, the base, the reaction temperature, etc. and is usually from 1 to 24 hours, preferably from 5 to 10 hours.

After the reaction, the desired compound is obtained, for example, by filtering the reaction mixture under reduced pressure to remove insolubles, adding a water-immiscible organic solvent such as ethyl acetate thereto, washing the mixture with water, separating the organic layer containing the desired compound, drying the layer over anhydrous magnesium sulfate, and distilling off the solvent.

If necessary, the desired compound can be further purified by recrystalization or various kinds of chromatographies.

### (Step A-2)

The present step is to prepare the compound (1) of the present invention by reacting the compound (3) with a reagent for eliminating the tert-butoxycarbonyl group in an inert solvent.

The solvent employable is not particularly limited so long as it does not affect the reaction and includes preferably alcohols such as methanol and ethanol, water and a mixture thereof.

The reagent for elimination is preferably an acid, and the acid is not particularly limited so long as it is used as an acid catalyst in the usual reaction and includes Bronsted acids such as inorganic acids, e.g. hydrochloric acid, hydrobromic acid, sulfuric acid, perchloric acid and phosphoric acid, and organic acids, e.g. acetic acid, formic acid, oxalic acid, methanesulfonic acid, para-toluenesulfonic acid, trifluoroacetic acid and trifluoromethanesulfonic acid; Lewis acids such as zinc chloride, tin tetrachloride, boron trichloride, boron trifluoride and boron tribromide; and acidic ion exchange resins, preferably organic acids (particularly acetic acid and trifluoroacetic acid).

The reaction temperature is usually from -10 to 50°C, preferably from 10 to 30°C.

The reaction time varies depending on the material, the base, the reaction temperature, etc. and is usually from 15 minutes to 10 hours, preferably from 1 to 5 hours.

After the reaction, the desired compound can be obtained, for example, by neutralizing the reaction mixture, distilling off the solvent under reduced pressure, and then purifying the residue over silica gel column chromatography.

Further, in the case where R^{2a}, R^{3a} or R^{4a} is a protecting group of a hydroxyl group or W^{a} is a protecting group of a carboxyl group, they are further eliminated to obtain the compound (1) of the present invention.

The method for eliminating the protecting group varies depending on the kind of the protecting group and can be carried out according to the methods usually used, for example, the methods described in Protective Groups in Organic Synthesis, Second Edition (1991, Green et al.).

In the case where the protecting group of a hydroxyl group is a trialkylsilyl group such as a tert-butyldimethylsilyl group, acetic acid is preferably used in a mixture of water and tetrahydrofuran, or tetrabutylammonium fluoride is used in tetrahydrofuran.

In the case where the protecting group of a hydroxyl group is an isopropylidene group, the method of Step E-2 or E-4 described later is used.

In the case where the protecting group of a carboxyl group is a diphenylmethyl group, catalytic reduction is carried out, an acid such as acetic acid and trifluoroacetic acid is used or trifluoroboran-diethyl ether complex is used.

In the case where the protecting group of a carboxyl group is a benzyl group, catalytic reduction is carried out, and in the case where the protecting group is the methyl group, hydrolysis is carried out. 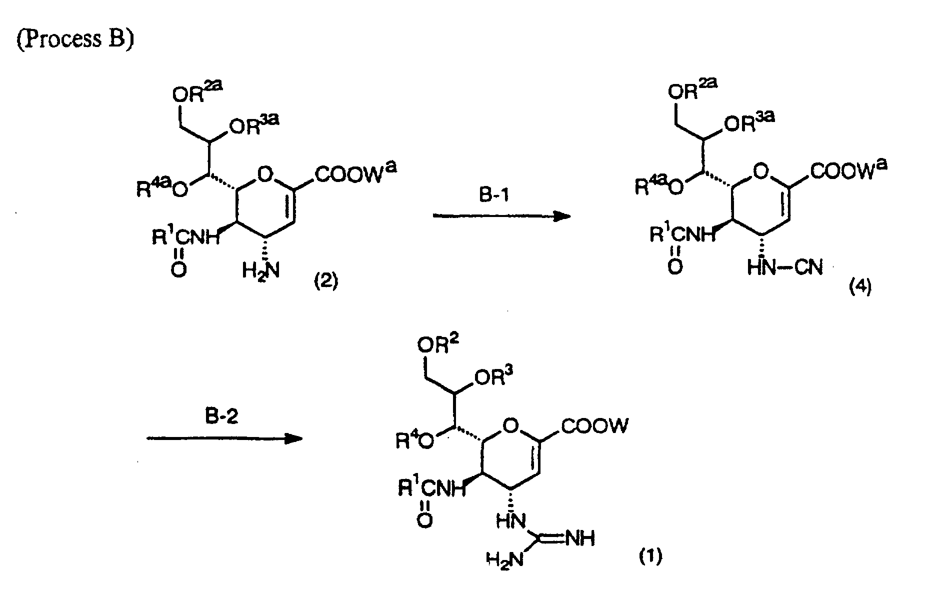 Process B is a process for preparing the compound (1) of the present invention by reacting the raw material compound (2), which is easily available according to the method described later, with a cyanating agent, reacting the resulting compound with ammonia, and further, if necessary, eliminating the protecting group.

### (Step B-1)

The present step is to prepare a compound (4) by reacting the compound (2) with a cyanating agent in an inert solvent.

The solvent employable is not particularly limited so long as it does not affect the reaction and includes alcohols such as methanol, ethanol, n-propanol, isopropanol, n-butanol, isobutanol, t-butanol, isoamyl alcohol, diethylene glycol, glycerin, octanol, cyclohexanol and methyl cellosolve; amides such as formamide, N,N-dimethylformamide, N,N-dimethylacetamide, N-methyl-2-pyrrolidone, N-methylpyrrolidinone and hexamethylphosphoric triamide; and sulfoxides such as dimethyl sulfoxide and sulfolane, preferably alcohols (particularly methanol).

The cyanating agent employable includes preferably cyanogen bromide, and sodium acetate is simultaneously employed as a base.

The reaction temperature is usually from -10 to 50°C, preferably from 10 to 40°C.

The reaction time varies depending on the material used, the base, the reaction temperature, etc. and is usually from 15 minutes to 10 hours, preferably from 1 to 5 hours.

After the reaction, the desired compound can be obtained, for example, by distilling off the solvent and then purifying the residue by recrystalization or silica gel column chromatography.

### (Step B-2)

The present step is to prepare the compound (1) of the present invention by reacting the compound (4) with ammonia in an inert solvent.

The solvent employable is not particularly limited so long as it does not affect the reaction and includes preferably alcohols (particularly methanol).

The reaction temperature is usually from -10 to 50°C, preferably from 10 to 40°C.

The reaction time varies depending on the material used, the base, the reaction temperature, etc. and is usually from 15 minutes to 10 hours, preferably from I to 5 hours.

After the reaction, the desired compound can be obtained, for example, by distilling off the solvent and then purifying the residue by recrystalization or silica gel column chromatography.

Incidentally, in the case where R^{2a}, R^{3a} or R^{4a} is a protecting group of a hydroxyl group, or W^{a} is a protecting group of a carboxyl group, the compound of the present invention is obtained by eliminating the protecting group in a similar manner to Process A.

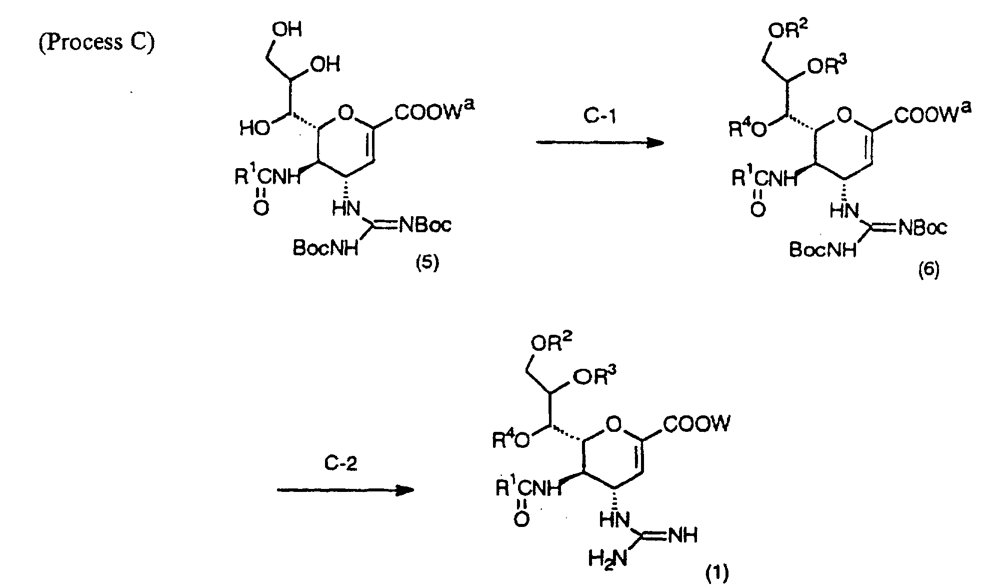

Process C is a process for preparing the compound (1) of the present invention by acylating partly or entirely the hydroxyl groups of the raw material compound (5) which is easily available according to the method described later, and then eliminating the protecting group of the resulting compound.

### (Step C-1)

The present step is to prepare the compound (6) by introducing a desired acyl group to the compound (5) in an inert solvent.

The acylation method includes the following methods 1 to 3.

### <Method 1>

Method 1 is to react a compound of the general formula: RCO-Hal or a compound of the general formula: RCO-O-COR
[wherein,
R represents an alkyl group, Hal represents a group to be eliminated, and the group to be eliminated is not particularly limited so long as it is a group to be eliminated as a nucleophilic residue and includes preferably a halogen atom such as chlorine, bromine and iodine; a lower alkoxycarbonyloxy group such as methoxycarbonyloxy and ethoxycarbonyloxy; a halogenated alkylcarbonyloxy group such as chloroacetyloxy, dichloroacetyloxy, trichloroacetyloxy and trifluoroacetyloxy; a lower alkanesulfonyloxy group such as methanesulfonyloxy and ethanesulfonyloxy; a halogeno lower alkanesulfonyloxy group such as trifluoromethanesulfonyloxy and pentafluoroethanesulfonyloxy; and an arylsulfonyloxy group such as benzenesulfonyloxy, p-toluenesulfonyloxy and p-nitrobenzenesulfonyloxy, more preferably a halogen atom, a halogeno lower alkanesulfonyloxy group and an arylsulfonyloxy group]
with the compound (5) in a solvent in the presence or absence of a base.

The solvent employable is not particularly limited so long as it does not inhibit the reaction and dissolves the starting material to some extent, and includes preferably aliphatic hydrocarbons such as hexane and heptane; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; nitriles such as acetonitrile and isobutyronitrile; and amides such as formamide, N,N-dimethylformamide, N,N-dimethylacetamide, N-methyl-2-pyrrolidone, N-methylpyrrolidinone and hexamethylphosphoric triamide.

The base employable is not particularly limited so long as it is used as a base in the usual reaction and includes preferably organic bases such as N-methylmorpholine, triethylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(tert-butyl)-4-methylpyridine, quinoline, N,N-dimethylaniline and N,N-diethylaniline.

Incidentally, 4-(N,N-dimethylamino)pyridine and 4-pyrrolidinopyridine can be used at a catalytic amount by combining it with other bases, and further quaternary ammonium salts such as benzyltriethylammonium chloride and tetrabutylammonium chloride and crown ethers such as dibenzo-18-crown-6 can be also added thereto in order to effectively carry out the reaction.

The reaction is usually carried out at from -20°C to the refluxing temperature of the solvent used, preferably from 0°C to the refluxing temperature of the solvent used.

The reaction time varies mainly depending on the reaction temperature, the raw material compound, the base used or the kind of the solvent used, and is usually from 10 minutes to 3 days, preferably from 1 to 6 hours.

### <Method 2>

Method 2 is to react a compound of the general formula: RCOOH [wherein R has the same meaning as defined above] with the compound (5) in a solvent in the presence or absence of "an esterifying agent" and a catalytic amount of base.

The "esterifying agent" employable includes a condensing agent; halogenated formates such as methyl chloroformate and ethyl chloroformate; and cyanophosphoric acid diesters such as diethyl cyanophosphate. Such "condensing agents" includes N-hydroxy derivatives such as N-hydroxysuccinimide, 1-hydroxybenzotriazole and N-hydroxy-5-norbornene-2,3-dicarboxyimide; disulfide compounds such as 2,2'-dipyridyl disulfide; succinic acid compounds such as N,N'-disuccinimidyl carbonate; phosphinic chloride compounds such as N,N'-bis(2-oxo-3-oxazolidinyl)phosphinic chloride; oxalate derivatives such as N,N'-disuccinimidyl oxalate (DSO), N,N'-diphthalimide oxalate (DPO), N,N'-bis(norbornenylsuccinimidyl)oxalate (BNO), 1,1'-bis(benzotriazolyl)oxalate (BBTO), 1,1'-bis(6-chlorobenzotriazolyl)oxalate (BCTO) and 1,1' -bis(6-trifluoromethylbenzotriazolyl)oxalate (BTBO); triarylphosphines such as triarylphosphines, e.g. triphenylphosphine and azodicarboxylic acid di-lower alkyl-triarylphosphines such as diethyl azodicarboxylate-triphenylphosphine; N-lower alkyl-5-arylisoxazolium-3'-sulfonates such as N-ethyl-5-phenylisoxazolium-3'-sulfonate; carbodiimide derivatives such as N',N'-dicycloalkylcarbodiimides, e.g. N',N'-dicyclohexylcarbodiimide (DCC) and 1-ethyl-3-(3-dimethylaminopropyl)carbodiimide (EDAPC); diheteroaryldiselenides such as di-2-pyridyldiselenide; arylsulfonyltriazolides such as p-nitrobenzenesulfonyltriazolide; 2-halo-1-lower alkylpyridinium halides such as 2-chloro-1-methylpyridinium iodide; diarylphosphorylazides such as diphenylphosphorylazide (DPPA); and imidazole derivatives such as 1,1'-oxazolyldiimidazole and N,N'-carbonyldiimidazole, preferably diarylphosphorylazides.

The solvent employable is not particularly limited so long as it does not inhibit the reaction and dissolves the starting material to some extent, and includes preferably aliphatic hydrocarbons such as hexane and heptane; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; nitriles such as acetonitrile and isobutyronitrile; amides such as formamide, N,N-dimethylformamide, N,N-dimethylacetamide, N-methyl-2-pyrrolidone, N-methylpyrrolidinone and hexamethylphosphoric triamide.

As the base to be used, similar bases to those described in the above <Method 1> can be employed.

The reaction is carried out at from -20°C to 80°C, preferably from 0°C to room temperature.

The reaction time varies depending mainly on the reaction temperature, the raw material compound, the reaction reagent or the kind of solvent used, and is usually from 10 minutes to 3 days, preferably from 30 minutes to one day.

### <Method 3>

Method 3 is to react the compound of the general formula: RCOOH [wherein R has the same meaning as defined above] with the compound (5) in a solvent in the presence of halogenated phosphoric acid dialkyl ester such as diethyl chlorophosphate and a base.

The solvent employable is not particularly limited so long as it does not inhibit the reaction and dissolves the starting material to some extent, and includes preferably aliphatic hydrocarbons such as hexane and heptane; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; nitriles such as acetonitrile and isobutyronitrile; and amides such as formamide, N,N-dimethylformamide, N,N-dimethylacetamide, N-methyl-2-pyrrolidone, N-methylpyrrolidinone and hexamethylphosphoric triamide.

As the base used, similar bases to those described in the above <Method 1> can be employed.

The reaction is carried out at from 0°C to the refluxing temperature of the solvent used, preferably from room temperature to 50°C.

The reaction time varies depending mainly on the reaction temperature, the raw material compound, the reaction reagent or the kind of solvent used, and is usually from 10 minutes to 3 days, preferably from 30 minutes to one day.

In the above <Method 1>, <Method 2> and <Method 3>, the compound (6) in which from 1 to 3 acyl groups are introduced to the compound (5) can be obtained by appropriately controlling an equivalent amount of the acylating agent used relative to the compound (5).

After the reaction, the desired compound (6) of the present reaction is collected from the reaction mixture according to the conventional method.

For example, the desired compound can be obtained by appropriately neutralizing the reaction mixture, eliminating insolubles by filtration if they exist, adding a water-immiscible organic solvent such as ethyl acetate thereto, washing it with water, separating the organic layer containing the desired compound, drying the layer over anhydrous magnesium sulfate and distilling off the solvent.

The desired compound thus obtained can be separated and purified, if necessary, by appropriately combining the conventional methods, for example, recrystalization, reprecipitation or a method usually employable for separation and purification of organic compounds, for example, adsorption column chromatography using carriers such as silica gel, alumina, Florisil of magnesium-silica gel system; a method using a synthesized adsorbing agent such as partition column chromatography using carriers such as Sephadex LH-20 (manufactured by Pharmacia Co., Ltd.), Amberlite XAD-11 (manufactured by Rohm & Haas Co., Ltd.) and Diaion HP-20 (Mitsubishi Chemical Corp.), or normal phase reversed phase column chromatography using silica gel or alkylated silica gel (preferably high performance liquid chromatography), and eluting it with appropriate eluting solutions.

### (Step C-2)

The present step is to prepare the compound (1) of the present invention by eliminating a t-butoxycarbonyl group in the compound (6) in an inert solvent.

The present step can be carried out similarly to the procedures of Step A-2.

Further, in the case where W^{a} is a protecting group of a carboxyl group, the compound (1) of the present invention can be obtained by further eliminating them similarly to the procedures of Step A-2.

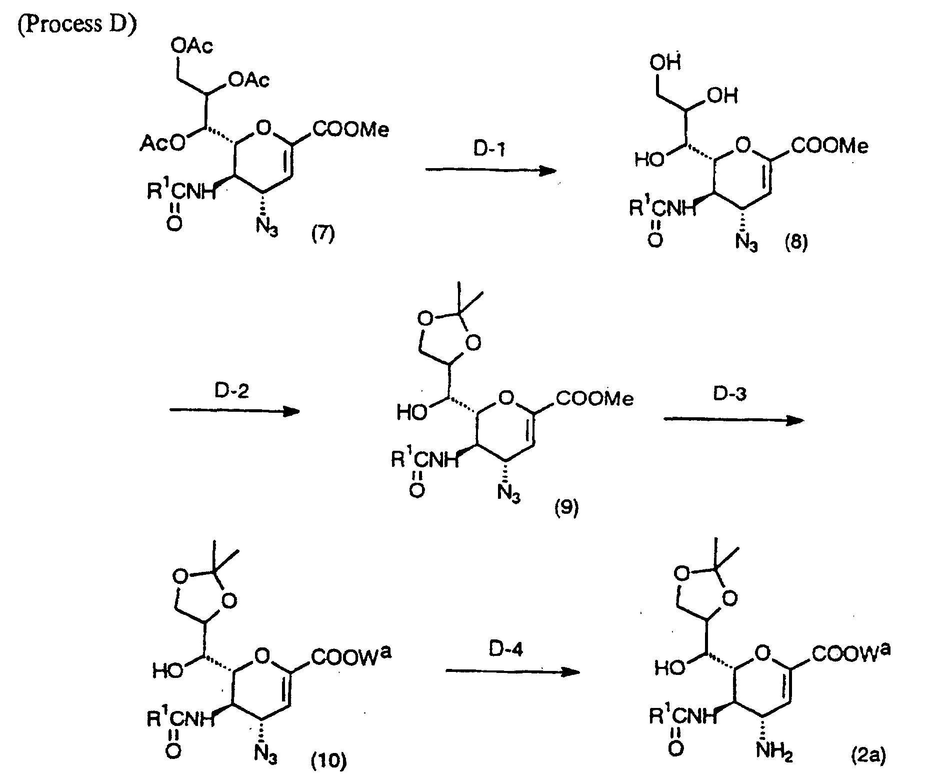

Process D is to prepare the compound (2a) which is one of the raw material compounds in Processes A and B using the raw material compound (7) easily available according to the method described later.

### (Step D-1)

The present step is to prepare a compound (8) by reacting the compound (7) with a base in an inert solvent.

The solvent employable is not particularly limited so long as it does not inhibit the reaction and dissolves the starting material to some extent, and includes preferably aliphatic hydrocarbons such as hexane, heptane, ligroin and petroleum ether, aromatic hydrocarbons such as benzene, toluene and xylene; halogenated hydrocarbons such as methylene chloride, chloroform, carbon tetrachloride, dichloroethane, chlorobenzene and dichlorobenzene; ethers such as diethyl ether, diisopropyl ether, tetrahydrofuran, dioxane, dimethoxyethane and diethylene glycol dimethyl ether; and methanol, preferably halogenated hydrocarbons and methanol.

The base employable is not particularly limited so long as it does not affect other functional groups (for example, methyl ester) and includes preferably alkali metal methoxides such as sodium methoxide and potassium methoxide.

The reaction temperature is usually from -10 to 50°C, preferably from 10 to 30°C.

The reaction time varies depending on the material used, the base, the reaction temperature, etc., and is usually from 15 minutes to 10 hours, preferably from 1 to 5 hours.

After the reaction, the desired compound can be obtained, for example, by neutralizing the reaction mixture with hydrochloric acid/dioxane solution, distilling off the solvent under reduced pressure, and then purifying the residue over silica gel column chromatography.

### (Step D-2)

The present step is to prepare the compound (9) by reacting the compound (8) with a reagent for introducing an isopropylidene group in an inert solvent.

The solvent employable is not particularly limited so long as it does not inhibit the reaction and dissolves the starting material to some extent, and includes aliphatic hydrocarbons such as hexane, heptane, ligroin and petroleum ether; aromatic hydrocarbons such as benzene, toluene and xylene; ethers such as diethyl ether, diisopropyl ether, tetrahydrofuran, dioxane, dimethoxyethane and diethylene glycol dimethyl ether; and ketones such as acetone, methyl ethyl ketone, methyl isobutyl ketone, isophorone and cyclohexanone, preferably ketones (particularly acetone).

The reagent employable includes preferably 2,2-dimethoxypropane and acids such as p-toluenesulfonic acid are used as a catalyst.

The reaction temperature is usually from -10 to 50°C, preferably from 10 to 30°C.

The reaction time varies depending on the material used, the base, the reaction temperature, etc., and is usually from 15 minutes to 10 hours, preferably from 1 to 5 hours.

After the reaction, the desired compound can be obtained, for example, by adding a water-immiscible solvent such as ethyl acetate and an aqueous sodium hydrogencarbonate solution to the reaction mixture, extracting the desired compound with ethyl acetate, and distilling off the solvent. The desired compound can be further purified, if necessary, by recrystalization or various kinds of chromatographies.

### (Step D-3)

The present step is, if necessary, 1) to substitute the methyl group of the methyl carboxylate portion with other ester residue, 2) to hydrolize the methyl carboxylate portion or 3) to introduce a protecting group of the carboxyl group or the ester residue after hydrolysis of 2).

### (Ester interchange)

The present step is to prepare the compound (10) by reacting the compound (9) with an alcohol which can give the desired ester group in an inert solvent in the presence of a base.

The solvent employable is not particularly limited so long as it does not inhibit the reaction and includes, for example, aliphatic hydrocarbons such as hexane, heptane, ligroin and petroleum ether; aromatic hydrocarbons such as benzene, toluene and xylene; halogenated hydrocarbons such as methylene chloride, chloroform, carbon tetrachloride, dichloroethane, chlorobenzene and dichlorobenzene; ethers such as diethyl ether, diisopropyl ether, tetrahydrofuran, dioxane, dimethoxyethane and diethylene glycol dimethyl ether; and alcohols such as methanol, ethanol, n-propanol, isopropanol, n-butanol, isobutanol, t-butanol, isoamyl alcohol, diethylene glycol, glycerin, octanol, cyclohexanol and methyl cellosolve. Preferably, an alcohol forming the desired ester group may be employed as the solvent.

The base employable includes preferably organic bases such as pyridine, triethylamine, diethylamine and 4-N,N-dimethylaminopyridine.

After the reaction, the desired compound can be obtained, for example, by neutralizing the reaction mixture with an acid, adding a water-immiscible solvent such as ethyl acetate to the mixture, extracting the desired compound with ethyl acetate, etc., washing it with water, and distilling off the solvent. The desired compound can be further purified, if necessary, by recrystalization or various kinds of chromatographies.

### (Diphenylmethylation)

The present step is to prepare the compound (10) by reacting the compound (9) with diphenyldiazomethane in an inert solvent in the presence of Lewis acid.

The solvent employable is not particularly limited-so long as it does not inhibit the reaction and dissolves the starting material to some extent, and includes preferably aliphatic hydrocarbons such as hexane, heptane, ligroin and petroleum ether; aromatic hydrocarbons such as benzene, toluene and xylene; halogenated hydrocarbons such as methylene chloride, chloroform, carbon tetrachloride, dichloroethane and chlorobenzene; ethers such as diethyl ether, diisopropyl ether, tetrahydrofuran, dioxane, dimethoxyethane and ethylene glycol dimethyl ether; and alcohols such as methanol, ethanol, n-propanol, isopropanol, n-butanol, isobutanol, t-butanol, isoamyl alcohol, diethylene glycol, glycerin, octanol, cyclohexanol and methyl cellosolve, more preferably alcohols (particularly methanol), halogenated hydrocarbons (dichloromethane); and mixtures thereof.

The Lewis acid employable includes preferably boron trifluoride-ether complex.

The reaction temperature is usually from 0 to 50°C, preferably room temperature.

The reaction time varies depending on the starting material, Lewis acid and the reaction temperature, and is usually from 10 minutes to 5 hours, preferably from 1 to 3 hours.

After the reaction, the solvent is distilled off and purification by recrystalization or chromatography is carried out to obtain the desired compound.

### (Hydrolysis)

The present step is to prepare the compound (10) in which W^{a} is a hydrogen atom by hydrolizing the compound (9) in an inert solvent in the presence of a base.

The solvent employable is not particularly limited so long as it does not inhibit the reaction and dissolves the starting material to some extent, and includes aromatic hydrocarbons such as benzene and toluene; ethers such as diethyl ether and tetrahydrofuran; halogenated hydrocarbons such as dichloromethane and chloroform; ketones such as acetone and methyl ethyl ketone; water; and mixtures of these organic solvents and water.

The base employable includes alkali metal hydroxides such as lithium hydroxide, sodium hydroxide and potassium hydroxide; and alkali metal hydrogencarbonates such as sodium hydrogencarbonate and potassium hydrogencarbonate.

The reaction temperature is usually from -10 to 50°C, preferably from 10 to 30°C.

The reaction time varies depending on the raw material used, the base, the reaction temperature, etc., and is usually from 15 minutes to 10 hours, preferably from 1 to 5 hours.

After the reaction, the desired compound can be obtained, for example, by cooling the reaction mixture, making it weakly acidic using dilute hydrochloric acid, adding a water-immiscible solvent such as ethyl acetate to the reaction mixture, extracting the desired compound with ethyl acetate, and distilling off the solvent. The desired compound can be further purified by recrystalization or various kinds of chromatographies.

### (Introduction of the protecting group of carboxyl group or the ester residue)

The present step is to prepare the compound (10) by introducing the protecting group or the ester residue to the carboxyl group at the 1-position of the compound (9) in which W^{a} is a hydrogen atom.

Introduction of the protecting group or the ester residue varies depending on the kind of ester residue or the protecting group, and can be carried out according to the methods generally used in the field of organic synthesis chemistry, for example, the methods described in Protective Groups in Organic Synthesis, Second Edition (1991, Green et al.).

### (Step D-4)

The present step is to prepare the compound (2a) from the compound (10) using a reducing agent in an inert solvent.

The solvent employable is not particularly limited so long as it does not inhibit the reaction and dissolves the starting material to some extent, and includes preferably aliphatic hydrocarbons such as hexane, heptane, ligroin and petroleum ether; aromatic hydrocarbons such as benzene, toluene and xylene; ethers such as diethyl ether, diisopropyl ether, tetrahydrofuran, dioxane, dimethoxyethane and diethylene glycol dimethyl ether; alcohols such as methanol, ethanol, n-propanol, isopropanol, n-butanol, isobutanol, t-butanol, isoamyl alcohol, diethylene glycol, glycerin, octanol, cyclohexanol and methyl cellosolve; ketones such as acetone, methyl ethyl ketone, methyl isobutyl ketone, isophorone and cyclohexanone; nitriles such as acetonitrile and isobutyronitrile; amides such as formamide, N,N-dimethylformamide, N,N-dimethylacetamide, N-methyl-2-pyrrolidone, N-methylpyrrolidinone and hexamethylphosphoric triamide; sulfoxides such as dimethyl sulfoxide and sulfolane; aliphatic carboxylic acids such as acetic acid; and mixtures of these organic solvents and water, preferably the alcohols (particularly methanol), the ethers such as tetrahydrofuran and dioxane, the aliphatic carboxylic acids such as acetic acid, and mixtures of these organic solvents and water.

As the reducing agent used, a catalyst such as palladium-carbon, platinum and Raney nickel may be employed in the presence of hydrogen gas, and a Lindlar catalyst (Pd-BaSO₄ or Pd-CaCO₃ and quinoline or lead acetate are employed in combination) is particularly preferably employed.

The reaction temperature is usually from -10°C to 50°C, preferably from 10 to 30°C.

The reaction time varies depending on the raw material used, the base, the reaction temperature, etc., and is usually from 15 minutes to 10 hours, preferably from 1 to 5 hours.

After the reaction, the desired compound can be obtained, for example, by filtering the reaction mixture under reduced pressure to remove the catalyst, and distilling off the solvent under reduced pressure. The desired compound can be further purified, if necessary, by recrystalization or various kinds of chromatographies.

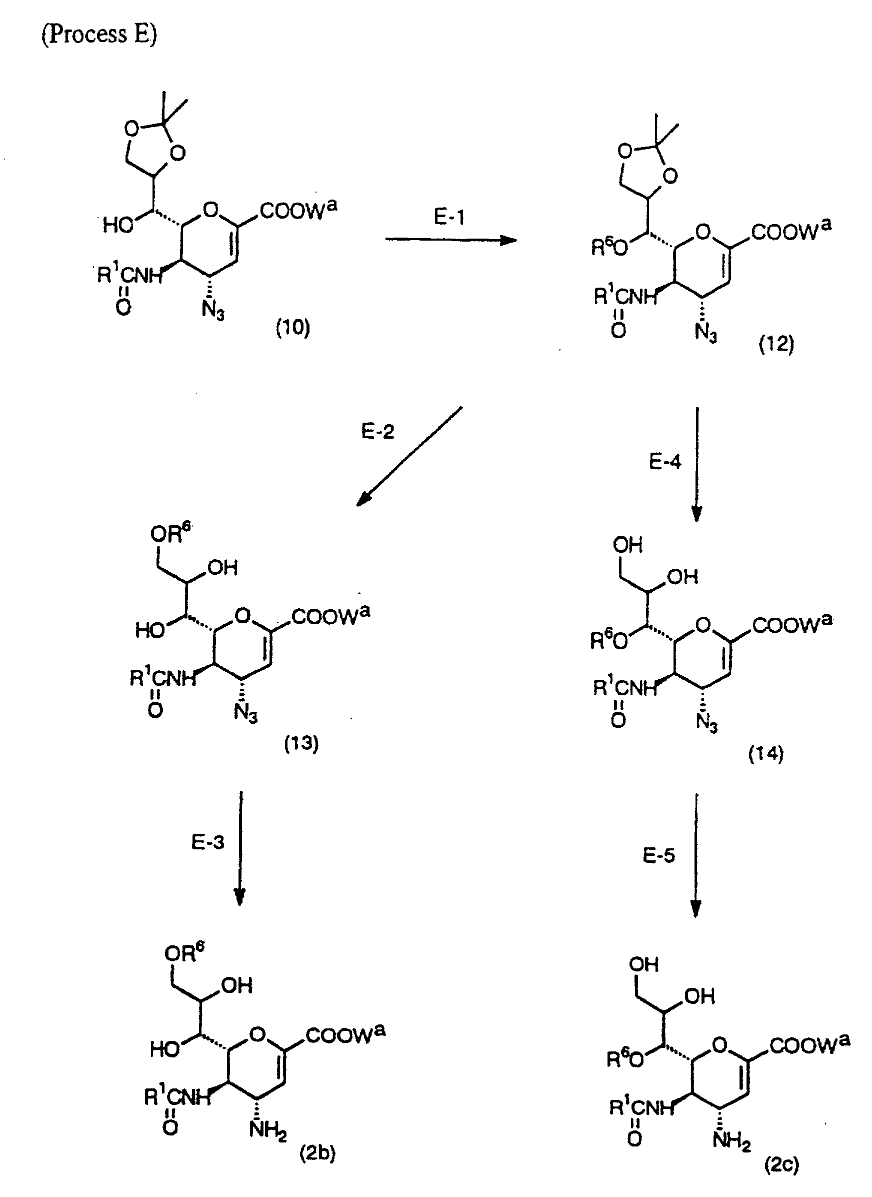

Process E is a process for preparing the compound (2b) or (2c) which is one of the raw material compounds in Processes A and B using the raw material compound (10) which is easily available according to the above method.

### (Step E-1)

The present step is to prepare the compound (12) by introducing the desired acyl group to the compound (10) in an inert solvent.

Further, the present step can be carried out similarly to the procedures of the above Step C-1.

### (Step E-2)

The present step is to prepare the compound (13) by treating the compound (12) with a reagent for eliminating the isopropylidene group in an inert solvent.

The solvent employable is not particularly limited so long as it does not inhibit the reaction and dissolves the starting material to some extent, and includes preferably halogenated hydrocarbons such as methylene chloride and chloroform.

As the reagent used for the elimination, an acid is preferable, and the acid is not particularly limited so long as it is used as an acid catalyst in the usual reaction and includes Bronsted acids such as inorganic acids, e.g. hydrochloric acid, hydrobromic acid, sulfuric acid, perchloric acid and phosphoric acid, and organic acids such as formic acid, oxalic acid, methanesulfonic acid, p-toluenesulfonic acid, trifluoroacetic acid and trifluoromethanesulfonic acid; and Lewis acids such as zinc chloride, tin tetrachloride, boron trichloride, boron trifluoride and boron tribromide and acidic ion exchange resins, preferably organic acids (particularly the trifluoroacetic acid).

The reaction temperature is usually from -10 to 50°C, preferably from 10 to 30°C.

The reaction time varies depending on the raw material used, the base, the reaction temperature, etc., and is usually from 15 minutes to 10 hours, preferably from I to 5 hours.

After the reaction, the desired compound can be obtained, for example, by neutralizing the reaction mixture, distilling off the solvent under reduced pressure, and then purifying the residue over silica gel chromatography.

Further, in the present step, the acyl group (R⁶) at the 7-position is transferred to the 9-position.

### (Step E-3)

The present step is to prepare the desired raw material compound (2b) from the compound (13) using a reducing agent in an inert solvent.

The present step can be carried out similarly to the procedures of the above Step D-4.

### (Step E-4)

The present step is to prepare the compound (14) of the present invention by treating the compound (12) with a reagent for eliminating the isopropylidene group in an inert solvent in the presence of an acid catalyst.

The solvent employable is not particularly limited so long as it does not inhibit the reaction and dissolves the starting material to some extent, and includes preferably a mixture of acetic acid (which is simultaneously utilized as the acid catalyst) and water.

The reaction temperature is usually from 10 to 70°C, preferably from 30 to 60°C.

The reaction time varies depending on the raw material used, the base, the reaction temperature, etc., and is usually from 15 minutes to 24 hours, preferably from 10 to 20 hours.

After the reaction, the desired compound can be obtained, for example, by distilling off the solvent under reduced pressure, adding a water-immiscible solvent such as ethyl acetate and an aqueous sodium hydrogencarbonate solution to the reaction mixture, extracting the desired compound with ethyl acetate, and distilling off the solvent. The desired compound can be further purified, if necessary, by recrystalization or various kinds of chromatographies.

### (Step E-5)

The present step is to prepare the desired raw material compound (2c) from the compound (14) using a reducing agent in an inert solvent.

The present step can be carried out similarly to the procedures in the above Step. D-4.

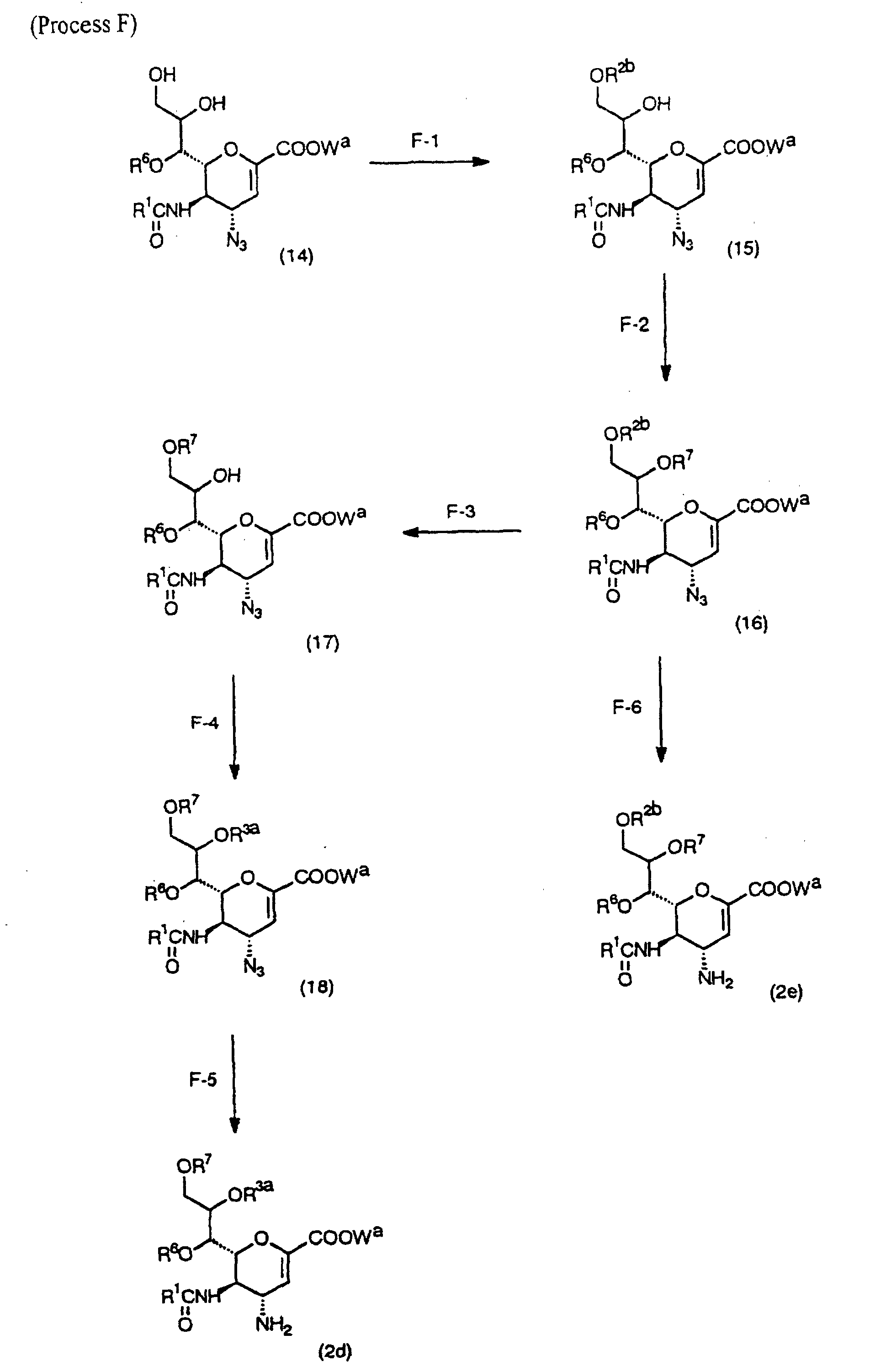

Process F is a process for preparing the compound (2d) or (2c) which is one of the raw material compounds in Processes A and B using the raw material compound (14) which is easily available according to the above method.

### (Step F-1)

The present step is to prepare the compound (15) by reacting the compound (14) with a reagent for protecting the hydroxyl group in an inert solvent.

The protecting group is not particularly limited and includes preferably a tert-butyldimethylsilyl group and a tert-butyldiphenylsilyl group.

Silylation can be carried out according to the usual methods. For example, silylation can be carried out by reacting tert-butyldimethylsilyl halide (particularly chloride) in dimethylformamide in the presence of a base such as triethylamine and 4-(N,N-dimethylamino)pyridine.

The reaction temperature is usually from -10 to 50°C, preferably from 10 to 40°C.

The reaction time varies depending on the raw material used, the base, the reaction temperature, etc., and is usually from 15 minutes to 24 hours, preferably from 10 to 20 hours.

After the reaction, the desired compound can be obtained, for example, by adding a water-immiscible solvent such as ethyl acetate and an aqueous sodium hydrogencarbonate solution to the reaction mixture, extracting the desired compound with ethyl acetate, and distilling off the solvent. The desired compound can be further purified, if necessary, by recrystalization or various kinds of chromatographies.

### (Step F-2)

The present step is to prepare the compound (16) by introducing the desired acyl group to the compound (15) in an inert solvent.

The present step can be carried out similarly to the procedures of Step C-1.

### (Step F-3)

The present step is to prepare the compound (17) by reacting the compound (16) with a reagent for eliminating the protecting group (preferably a tert-butyldimethylsilyl group or a tert-butyldiphenylsilyl group) of the hydroxyl group in an inert solvent.

The solvent employable includes preferably alcohols such as methanol and ethanol, water and mixtures thereof.

Acids are preferably used as the reagent for the elimination, and the acid is not particularly limited so long as it is used as the acid catalyst in the usual reactions and includes Bronsted acids such as inorganic acids, e.g. hydrochloric acid, hydrobromic acid, sulfuric acid, perchloric acid and phosphoric acid, and organic acids, e.g. acetic acid, formic acid, oxalic acid, methanesulfonic acid, p-toluenesulfonic acid, trifluoroacetic acid and trifluoromethanesulfonic acid, and Lewis acids such as zinc chloride, tin tetrachloride, boron trichloride, boron trifluoride and boron tribromide, and acidic ion exchange resins, preferably organic acids (particularly acetic acid and trifluoroacetic acid).

Reagents which produce a fluoride ion such as tetrabutylammonium fluoride can be also used, as desired.

The reaction temperature is usually from -10 to 50°C, preferably from 10 to 30°C.

The reaction time varies depending on the raw material used, the base, the reaction temperature, etc., and is usually from 15 minutes to 10 hours, preferably from 1 to 5 hours.

After the reaction, the desired compound can be obtained, for example, by neutralizing the reaction mixture, distilling off the solvent under reduced pressure, and then purifying the residue over silica gel chromatography.

In the present step, the acyl group (R⁷) at the 8-position is transferred to the 9-position.

### (Step F-4)

The present step is to prepare the compound (18) by 1) protecting the hydroxyl group at the 8-position of the compound (17) or 2) introducing the desired acyl group, as desired.

### (Introduction of the acyl group)

The present step can be carried out similarly to the procedures of Step C-1.

### (Introduction of the protecting group)

As the protecting group, tert-butyldimethylsilyl group is preferable, and introduction of the protecting group is carried out using tert-butyldimethylsilyl triflate and lutidine in methylene chloride.

### (Step F-5)

The present step is to prepare the desired raw material compound (2d) from the compound (18) using a reducing agent in an inert solvent.

The present step can be carried out similarly to the procedures of Step D-4.

### (Step F-6)

The present step is to prepare the desired raw material compound (2e) from the compound (16) using a reducing agent in an inert solvent.

The present step can be carried out similarly to the procedures of Step D-4.

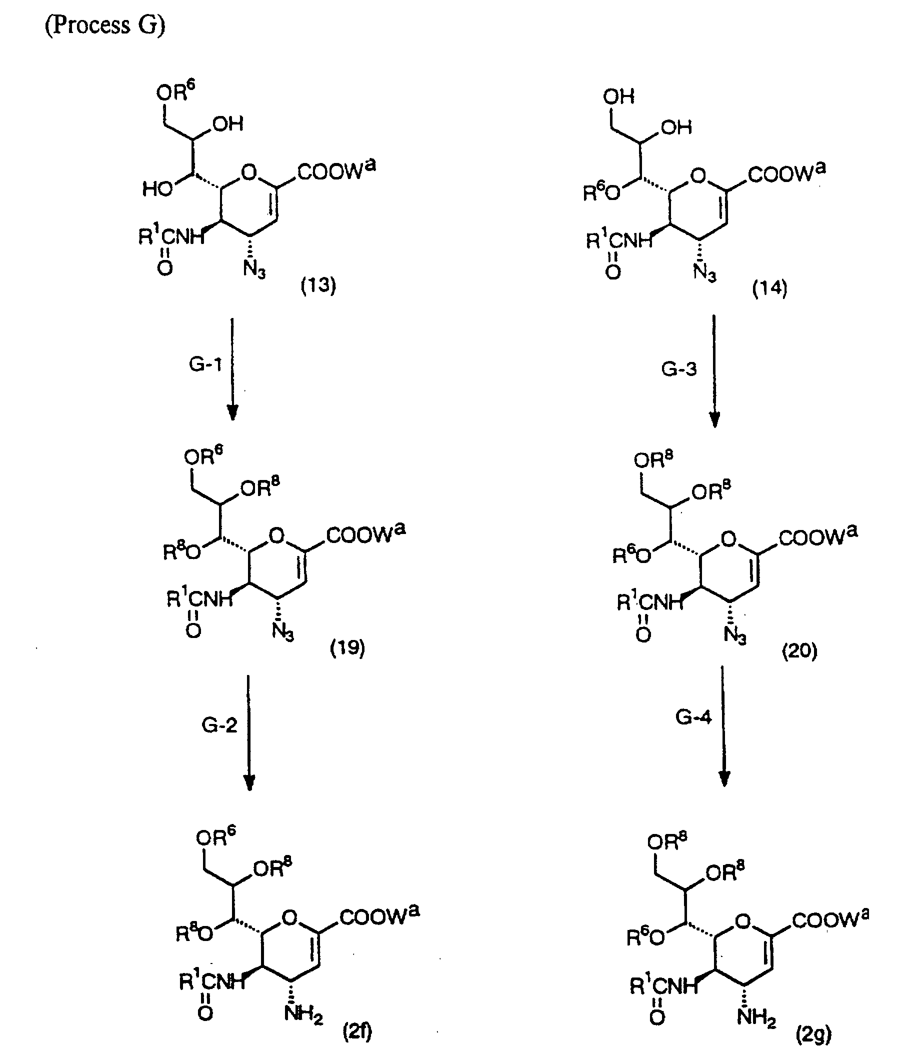

Process G is a process for preparing the compound (2f) or (2g) which is one of the raw material compounds in Processes A and B using the above compound (13) or (14) described above.

### (Step G-1)

The present step is to prepare the compound (19) by introducing the desired acyl group to the compound (13) in an inert solvent.

The present step can be carried out similarly to the procedures of Step C-1.

### (Step G-2)

The present step is to prepare the desired raw material compound (2f) from the compound (19) using a reducing agent in an inert solvent.

The present step can be carried out similarly to the procedures of Step D-4.

### (Step G-3)

The present step is to prepare the compound (20) by introducing the desired acyl group to the compound (14) in an inert solvent.

The present step can be carried out similarly to the procedures of Step C-1.

### (Step G-4)

The present step is to prepare the desired raw material compound (2g) from the compound (20) using a reducing agent in an inert solvent.

The present step can be carried out similarly to the procedures of Step D-4.

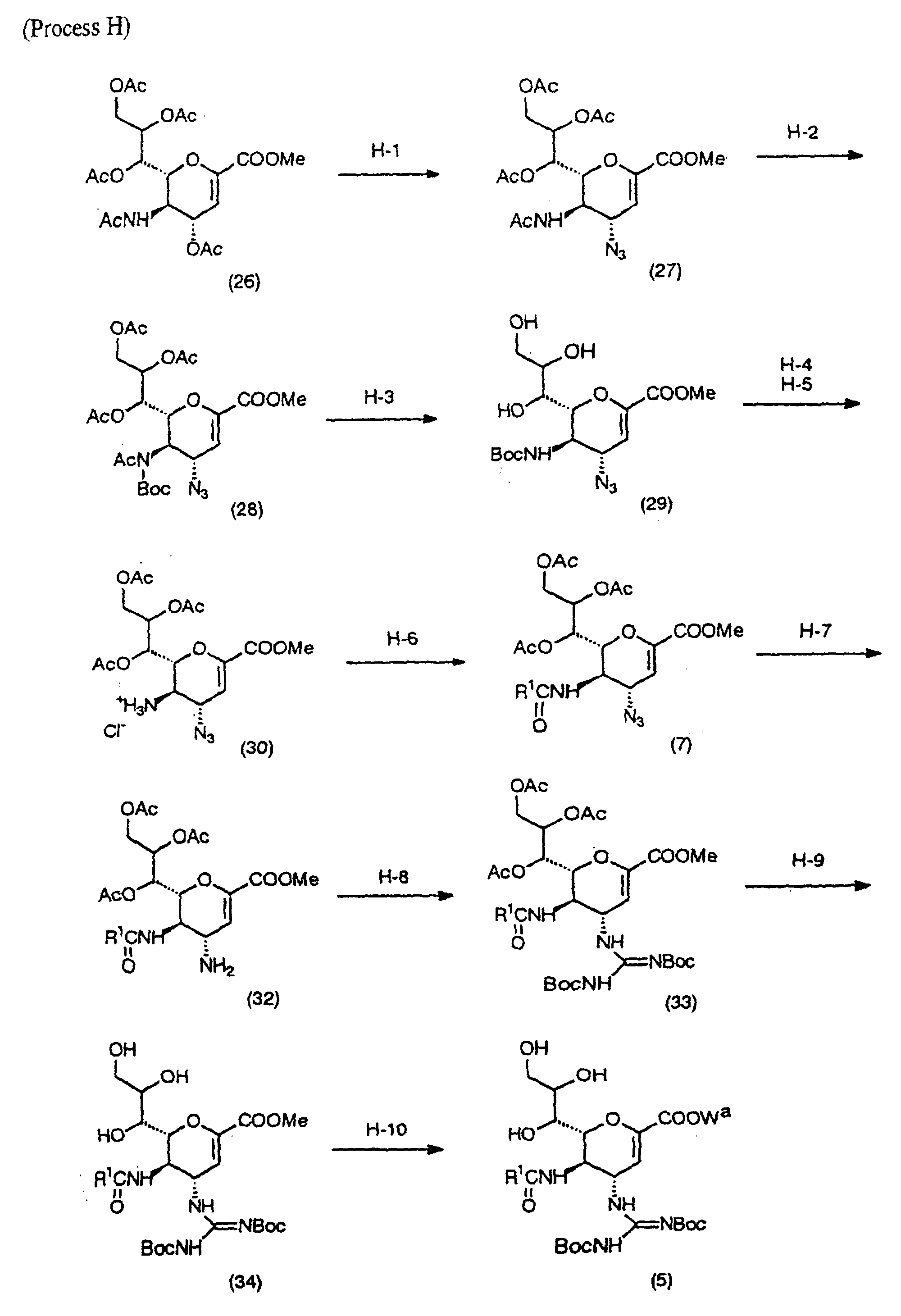

Process H is a process for preparing the compound (5) which is the raw material compound in Process C using the well known compound (26) described in Carbohydrate Research, 83, 163-169 (1980) or WO 95/32955.

### (Step H-1)

The present step is to prepare the compound (27) by reacting the known compound (26) with an azidating agent in an inert solvent.

The solvent employable is not particularly limited so long as it does not inhibit the reaction and dissolves the starting material to some extent, and includes preferably aromatic hydrocarbons such as benzene, toluene and xylene; halogenated hydrocarbons such as methylene chloride and chloroform; ethers such as diethyl ether, tetrahydrofuran, dioxane and dimethoxyethane; and nitriles such as acetonitrile.

The reagent employable is not particularly limited so long as it is usually used for azidation and includes preferably diaryl phosphoric azide derivatives such as diphenyl phosphoric azide; trialkylsilylazides such as trimethylsilylazide and triethylsilylazide and azidated alkali metal salts such as sodium azide and potassium azide, preferably the sodium azide.

The reaction temperature is usually from -10 to 50°C, preferably from 10 to 30°C.

The reaction time varies depending on the raw material used, the base, the reaction temperature, etc., and is usually from 15 minutes to 10 hours, preferably from 1 to 5 hours.

After the reaction, the desired compound can be obtained, for example, by neutralizing the reaction mixture with a hydrochloric acid/dioxane solution, and purifying the residue obtained by distilling off the solvent under reduced pressure over silica gel chromatography.

### (Step H-2)

The present step is to prepare the compound (28) by reacting the compound (27) with a t-butoxycarbonylating agent in an inert solvent.

The t-butoxycarbonylation can be carried out by reacting di-tert-butyl dicarbonate or 2-(tert-butoxycarbonyloxyimino)-2-phenylacetonitrile in an inert solvent (for example, aromatic hydrocarbons such as benzene and toluene; halogenated hydrocarbons such as methylene chloride and chloroform; ethers such as diethyl ether, tetrahydrofuran and dioxane; and amides such as dimethylformamide) in the presence of a base (for example, 4-(N,N-dimethylamino)pyridine).

After the reaction, the desired compound can be obtained, for example, by neutralizing the reaction mixture, distilling off the solvent under reduced pressure, adding a water-immiscible solvent such as ethyl acetate thereto, extracting the desired compound with ethyl acetate, and distilling off the solvent. The desired compound can be further purified, if necessary, by recrystalization or various kinds of chromatographies.

### (Step H-3)

The present step is to prepare the compound (29) by reacting the compound (28) with a base in an inert solvent.

The present step can be carried out similarly to the procedures of Step D-1.

### (Step H-4)

The present step is to acetylate the compound (29) in an inert solvent. The acetylation is carried out according to the usual method for protecting a hydroxyl group. For example, the acetylation can be carried out by 1) reacting with acetic anhydride in pyridine or 2) reacting with acetyl halide (particularly chloride) in methylene chloride in the presence of a base catalyst (for example, triethylamine and 4-N,N-dimethylaminopyridine).

After the reaction, the desired compound can be obtained by distilling off the solvent under reduced pressure, adding a water-immiscible solvent such as ethyl acetate and an aqueous sodium hydrogencarbonate solution to the residue, extracting the desired compound with ethyl acetate, and distilling off the solvent. The desired compound can be further purified, if necessary, by recrystalization or various kinds of chromatographies.

### (Step H-5)

The present step is to prepare the compound (30) by treating the compound obtained in Step H-4 with a reagent which eliminates a t-butoxycarbonyl group in an inert solvent.

The elimination of the t-butoxycarbonyl group is carried out according to the usual methods.

The solvent employable is not particularly limited so long as it does not inhibit the reaction and dissolves the starting material to some extent, and includes preferably aliphatic hydrocarbons such as hexane, heptane, ligroin and petroleum ether; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; amides such as formamide, N,N-dimethylformamide, N,N-dimethylacetamide, N-methyl-2-pyrrolidone, N-methylpyrrolidinone and hexamethylphosphoric triamide; and sulfoxides such as dimethyl sulfoxide and sulfolane, preferably the halogenated hydrocarbons (particularly methylene chloride).

The reagent employable is not particularly limited so long as it is usually used and includes preferably hydrochloric acid.

The reaction temperature is usually from -10 to 50°C, preferably from 10 to 30°C.

The reaction time varies depending on the raw material used, the base, the reaction temperature, etc., and is usually from 15 minutes to 24 hours, preferably from 1 to 10 hours.

After the reaction, the desired compound can be obtained, for example, by distilling off the solvent under reduced pressure, adding a water-immiscible solvent such as ethyl acetate and an aqueous sodium hydrogencarbonate solution to the reaction mixture, extracting the desired compound with ethyl acetate, and distilling off the solvent. The desired compound can be further purified, if necessary, by recrystalization or various kinds of chromatographies.

### (Step H-6)

The present step is to prepare the compound (7) by introducing the desired acyl group to the compound (30) in an inert solvent.

The present step can be carried out similarly to the procedures of Step C-1.

### (Step H-7)

The present step is to prepare the compound (32) from the compound (7) using a reducing agent in an inert solvent.

The present step can be carried out similarly to the procedures of Step D-4.

### (Step H-8)

The present step is to prepare the compound (33) by reacting the compound (32) with N,N'-di-t-butoxycarbonylthiourea in an inert solvent in the presence of a base and mercuric chloride.

The present step can be carried out similarly to the procedures of Step A-1.

### (Step H-9)

The present step is to prepare the compound (34) by reacting the compound (33) with a base in an inert solvent.

The present step can be carried out similarly to the procedures of Step D-1.

### (Step H-10) Ester interchange, hydrolysis, protection or esterification

The present step is 1) to substitute the methyl group of the methyl carboxylate portion with another ester residue, 2) to hydrolize the methyl carboxylate portion, or 3) to introduce a protecting group of the carboxyl group or an ester residue after hydrolysis in 2), as desired.

The present step can be carried out similarly to the procedures of Step D-3.

The compound (1) of the present invention can be also prepared according to processes other than those described above. Particularly, it is possible to prepare the compound (1) of the present invention by changing the order of the steps of Processes A to H described above depending on the situations. For example, the compound (1) of the present invention can be prepared according to Process J shown below using the compound (10) obtained as an intermediate in Process D.

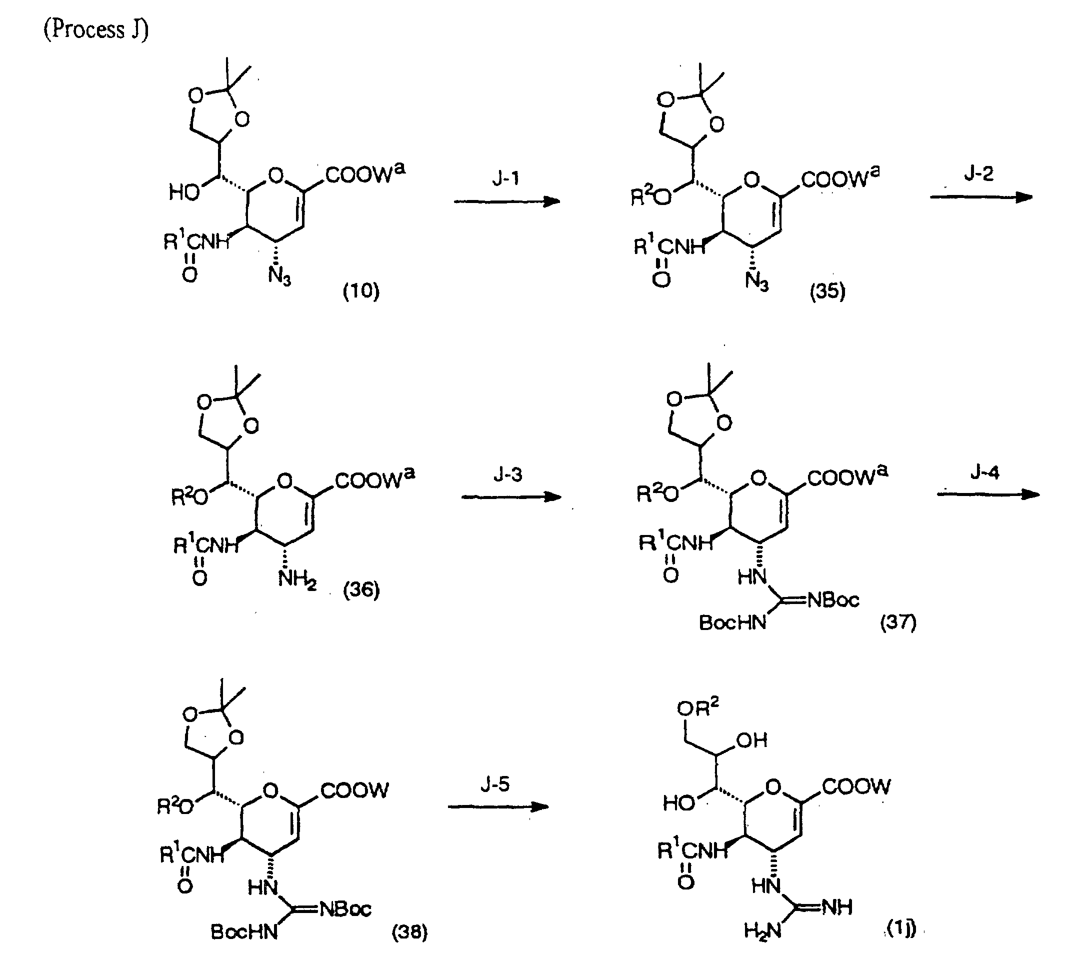

### (Step J-1)

The present step is carried out if necessary, and is to prepare the compound (35) by introducing the desired acyl group to the compound (10) in an inert solvent.

The present step can be carried out similarly to the procedures of Step C-1 described above.

### (Step J-2)

The present step is to prepare the compound (36) by reacting the compound (35) with a reducing agent in an inert solvent.

The present step can be carried out similarly to the procedures of Step D-4 described above.

### (Step J-3)

The present step is to prepare the compound (37) by reacting the compound (36) with N,N'-di-t-butoxycarbonylthiourea in an inert solvent in the presence of a base and mercuric chloride.

The present step can be carried out similarly to the procedures of Step A-1 described above.

### (Step J-4)

The present step is carried out if necessary, and is to prepare the compound (38) by eliminating the protecting group of the carboxyl group of the compound (37).

The elimination method of the protecting group varies depending on the kind of the protecting group, and can be carried out according to the methods usually used, for example, the methods described in Protective Groups in Organic Synthesis, Second Edition (1991, Green et al.).

In the case where the protecting group is a diphenylmethyl group, catalytic reduction is carried out, acids such as acetic acid and trifluoroacetic acid are used or a boron trifluoride-diethyl ether complex is used.

In the case where the protecting group of the carboxyl group is a benzyl group, catalytic reduction is carried out, and in the case where the protecting group is an alkyl group such as methyl and ethyl, hydrolysis is carried out.

### (Step J-5)

The present step is to prepare the compound (1j) of the present invention by reacting the compound (38) with a reagent for eliminating the tert-butoxycarbonyl group and isopropylidene group in an inert solvent.

The present step can be carried out similarly to the procedures of Step E-2.

The neuraminic acid compound (1) thus obtained or the salt thereof can be converted, if necessary, to other pharmacologically acceptable salts.

The neuraminic acid compound (1) of the present invention undergoes hydrolysis by hydrolase present in a living body and exhibits excellent viral replication inhibitory activity and sialidase inhibitory activity. In addition, if the neuraminic acid compound (1) is administered to mice infected with influenza virus, the compound exhibits infection therapeutic effects superior to Compound A (GG-167) described in WO91/16320 (Japanese PCT application (Kokai) No. Hei 5-507068). Thus, the neuraminic acid compound (1) of the present invention is useful as a therapeutic agent or a preventive agent (preferably a therapeutic agent) for viral infections (preferably influenza viral infections).

The administration form of the neuraminic acid compound of the present invention includes, for example, oral administration or intranasal administration by solutions such as liquid agents, aqueous co-solvents, etc., aerosols, powders, etc. Of these preparations, solutions such as liquid agents and aqueous co-solvents are prepared by the well known methods using purified water, pharmacologically acceptable organic solvents (for example, ethanol, propylene glycol, PEG 400, etc.), and stabilizers (paraoxybenzoates such as methylparaben and propylparaben; alcohols such as chlorobutanol, benzyl alcohol and phenylethyl alcohol; benzalkonium chloride; phenols such as phenol and cresol; thimerosal; dehydroacetic acid, etc.). The aerosols are prepared by the well known methods using a propellant such as various kinds of Freon gases and nitrogen gas, and a surface active agent such as lecitin. The powders are prepared by the well known methods using excipients (for example, organic excipients such as sugars, e.g. lactose, sucrose, glucose, mannitol and sorbitol; starches, e.g. corn starch, potato starch, α-starch, dextrin and carboxymethyl starch; celluloses, e.g. crystalline cellulose, low-substituted hydroxypropyl cellulose, hydroxypropyl cellulose, hydroxypropylmethyl cellulose, carboxymethyl cellulose, carboxymethyl cellulose calcium and internally bridged carboxymethyl cellulose sodium; gum arabic; dextran; and Pullulan, and inorganic excipients such as silicates, e.g. light silicic anhydride, synthesized aluminium silicate and magnesium aluminate metasilicate; phosphates, e.g. calcium phosphate; carbonates, e.g. calcium carbonate; and sulfates, e.g. calcium sulfate), lubricants (for example, stearic acid, stearic acid metal salts such as calcium stearate and magnesium stearate; talc; colloidal silica; waxes such as bee gum and spermaceti; boric acid; adipic acid; sulfates, e.g. sodium sulfate; glycol; fumaric acid; sodium benzoate; DL-leucine; fatty acid sodium salt; laurylsulfates such as sodium laurylsulfate and magnesium laurylsulfate; silicic acids such as silicic anhydride and silicic acid hydrate; and the above starches), stabilizers (paraoxybenzoates such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), corrigents (for example, sweeteners, vinegars, perfumes, etc. usually used), diluents, etc.

While the dose of the active ingredients will vary depending on the condition of disease, age of the patient, administration methods, etc., for example, in the case of solutions, it is desirable to administer the active ingredient in an amount of 0.1 mg (preferably 1 mg) as a lower limit and 1000 mg (preferably 500 mg) as an upper limit, in the case of dry powders, it is desirable to administer the active ingredient in an amount of 0.1 mg (preferably 1 mg) as a lower limit and 1000 mg (preferably 500 mg) as an upper limit, and in the case of aerosols, it is desirable to administer the active ingredient in an amount of 0.1 mg (preferably 1 mg) as a lower limit and 1000 mg (preferably 500 mg) as an upper limit, once or several times a day in the above administration methods, depending on the condition of disease.

### [Best mode for carrying out the invention]

The present invention will be described in more detail by way of Examples, Preparation examples and Test examples below.

### (Example 1)

### Myristyl 5-acetamido-2,3,4,5-tetradeoxy-4-guanidino-D-glycero-D-galacto-non-2-enopyranosoate trifluoroacetic acid salt (E8) (Exemplary compound No. 88)

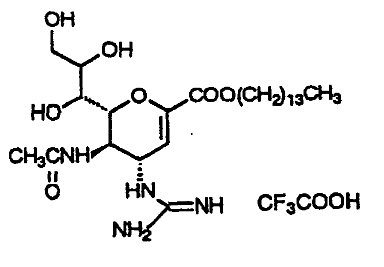

### i) Methyl 5-acetamido-4-azido-2,3,4,5-tetradeoxy-8,9-O-isopropylidene-D-glycero-D-galacto-non-2-enopyranosoate (E3)

1.3 g (2.8 mmol) of methyl 5-acetamido-7,8,9-tri-O-acetyl-4-azido-2,3,4,5-tetradeoxy-D-glycero-D-galacto-non-2-enopiranosonate (E1) (which was synthesized according to the processes described in Mark von Itzstein et al., Carbohydr. Res., 244, 181-185 (1993) and Carbohydr. Res., 259, 293-299 (1993)) were dissolved in 26 ml (20-times volume) at room temperature, and a catalytic amount of sodium methoxide was added to the system under the same conditions, followed by further stirring of the mixture for 1 hour. After confirmation of completion of the reaction, Dowex-50x8 (H⁺) was added to the system to neutralize the mixture. The reaction mixture was filtered and the filtered product was washed with methanol. The filtrate and the washing water were distilled off under reduced pressure, and the residue was purified over silica gel column chromatography (Kiesel gel 60, 100 g, methylene chloride:methanol = 5:1) to obtain 620 mg (yield: 66%) of the compound (E2) as a pale yellow solid.
Rf value: 0.3 (methylene chloride:methanol = 5:1)

580 mg (1.8 mmol) of the resulting compound (E2) were dissolved in 29 ml (50-times volume) of acetone at room temperature, and subsequently 0.7 ml (5.2 mmol) of 2,2-dimethoxypropane and 42 mg (0.18 mmol) of DL-10-camphorsulfonic acid were added to the system under ice-cooling, followed by stirring of the mixture at room temperature for 1 hour. After confirmation of completion of the reaction, triethylamine was poured into the system to neutralize the mixture. The reaction mixture was distilled off under reduced pressure, and the residue was purified over silica gel column chromatography (Kiesel gel 60, 60 g, benzene:acetonitrile = 1:1) to obtain 540 mg (yield: 84%) of the title compound (E3) as a white solid.
Rf value: 0.76 (benzene:acetonitrile = 1:1).
¹H-NMR (270MHz, CDCl₃, TMS): δ (ppm) 5.97 (1H, d, J=2.4Hz), 5.63 (1H, d, J=7.1Hz), 4.36 (1H, ddd, J=7.9, 6.4, 4.8Hz), 4.30-4.00 (6H, m), 3.81 (3H, s), 3.57 (1H, dd, J=7.9, 5.3Hz), 2.12 (3H, s), 1.40 (3H, s), 1.36 (3H, s); [α]_{D}²⁴ = +122.4° (c=1.0, CHCl₃).

### ii) 5-Acetamido-4-azido-2,3,4,5-tetradeoxy-8,9-O-isopropylidene-D-glycero-D-galacto-non-2-enopyranosoic acid (E4)

460 mg (1.2 mmol) of the compound (E3) were dissolved in 24 ml (30-times volume) of methanol at room temperature, and subsequently 2.9 ml (1.4 mmol) of 1M aqueous sodium hydroxide solution were added to the system at room temperature, followed by stirring of the mixture at room temperature for 1 hour. After confirmation of completion of the reaction, Dowex-50W was added to the system to neutralize the mixture. The reaction mixture was filtered, and the filtered product was washed with water. The filtrate and the washing solution were combined and distilled off under reduced pressure. The residue was purified over silica gel column chromatography (Kiesel gel 60, 60 g, ethyl acetate:2-propanol:water = 2:2:1) to obtain 0.44 g (yield: 100%) of the title compound (E4) as a white solid.
Rf value: 0.25 (ethyl acetate:2-propanol:water = 5:2:1);
¹H-NMR (270MHz, CD₃OD, TMS): δ (ppm) 5.72 (1H, d, J=2.1Hz), 4.37 (1H, dt, J=8.8, 5.5Hz), 4.27 (1H, dd, J=6.9, 1.4Hz), 4.20-4.04 (3H, m), 4.00 (1H, dd, J=8.8, 5.0Hz), 3.54 (1H, d, J=8.8Hz), 2.03 (3H, s), 1.37 (3H, s), 1.32 (3H, s),
[α]_{D}²⁴ = +43.4° (c=0.53, MeOH).

### iii) Myristyl 5-acetamido-4-azido-2,3,4,5-tetradeoxy-8,9-O-isopropylidene-D-glycero-D-galacto-non-2-enopyranosoate (E5)

460 mg (1.2 mmol) of the compound (E4), 530 mg (2.5 mmol) of myristyl alcohol and 510 mg (1.9 mmol) of 2-bromo-1-ethylpyridinium tetrafluoroborate were dissolved in 22 ml (50-times volume) of formamide. Subsequently, 0.9 ml (3.8 mmol) of tri-n-butylamine were poured into the system, and the mixture was stirred in an oil bath at 50°C for 6 hours. The reaction mixture was separated with ethyl acetate and a saturated aqueous NaCI solution, and the aqueous layer was extracted with ethyl acetate. The organic layer was dried over magnesium sulfate and filtered, followed by distilling off under reduced pressure. The residue was purified over silica gel column chromatography (Kiesel gel 60, 100 g, benzene:ethyl acetate = 1:1) to obtain 0.19 g (yield: 28%) of the title compound (E5) as a white solid.
Rf value: 0.4 (benzene:ethyl acetate = 1:1);
¹H-NMR (270MHz, CDCl₃, TMS): δ (ppm) 5.95 (1H, d, J=2.4Hz), 5.60 (1H, d, J=7.0Hz), 4.36 (1H, dt, J=7.7, 5.7Hz), 4.30-4.00 (8H, m), 3.58 (1H, d, J=7.7Hz), 2.12 (3H, s), 1.69 (2H, quintet, J=6.3Hz), 1.40 (3H, s), 1.35 (3H, s), 1.26 (22H, bs), 0.88 (3H, t, J=6.3Hz);
[α]_{D}²⁴ = +83.3° (c=0.54, CHCl₃).

### iv) Myristyl 5-acetamido-4-amino-2,3,4,5-tetradeoxy-8,9-O-isopropylidene-D-glycero-D-galacto-non-2-enopyranosoate (E6)

134 mg (0.24 mmol) of the compound (E5) were dissolved in 6.7 ml (50-times volume) of ethanol at room temperature, and subsequently 47 mg (0.35-times volume) of Lindlar catalyst were added to the system at room temperature, followed by stirring of the mixture at room temperature under a hydrogen atmosphere of 1 atm. for 2 hours. After confirmation of completion of the reaction, the reaction mixture was filtered, and the filtered product was washed with ethanol. The filtrate and the washing solution were combined and distilled off under reduced pressure. The residue thus obtained was purified over silica gel column chromatography (Kiesel gel 60, 60 g, ethyl acetate:2-propanol:water = 5:2:1) to obtain 0.11 g (yield: 88%) of the title compound (E6) as a white solid.
Rf value: 0.28 (ethyl acetate:2-propanol:water =8:2:1);
¹H-NMR (270MHz, CD₃OD, TMS): δ (ppm) 5.93 (1H, d, J=2.6Hz), 4.31 (1H, quintet, J=6.9Hz), 4.23-3.93 (5H, m), 3.88 (1H, t, J=9.0Hz), 3.67-3.50 (2H, m), 2.05 (3H, s), 1.68 (2H, quintet, J=8.1Hz), 1.37 (3H, s), 1.33 (3H, s), 1.27 (22H, bs), 0:88 (3H, t, J=8.1Hz).

### v) Myristyl 5-acetamido-4-(N,N'-bis-tert-butoxycarbonyl)guanidino-2,3,4,5-tetradeoxy-8,9-O-isopropylidene-D-glycero-D-galacto-non-2-enopyranosoate (E7)

110 mg (0.21 mmol) of the compound (E6), 72 mg (0.26 mmol) of N,N'-bis-tert-butoxycarbonylthiourea and 80 ml (0.57 mmol) of triethylamine were dissolved in 22 ml (50-times volume) of dimethylformamide at room temperature. Subsequently, 70.5 mg (0.26 mmol) of mercuric chloride were added to the system under ice-cooling, followed by stirring of the mixture at room temperature for 2 hours. The reaction mixture was diluted with ethyl acetate, and the mixture was filtered using Celite, and the filtered product was washed with ethyl acetate. The filtrate thus obtained and the washing solution were combined, and ethyl acetate and a saturated aqueous NaCl solution were added to the mixture, followed by separation. The organic layer was dried over magnesium sulfate and filtered, followed by distilling off of the solvent under reduced pressure. The residue was purified over silica gel column chromatography (Kiesel gel 60, 100 g, hexane:ethyl acetate = 3:2) to obtain 120 mg (yield: 71%) of the title compound (E7) as a white solid.
Rf value: 0.65 (hexane:ethyl acetate = 3:2);
¹H-NMR (270MHz, CDCl₃, TMS): δ (ppm) 11.4 (1H, s), 8.63 (1H, d, J=7.8Hz), 7.99 (1H, d, J=4.8Hz), 5.78 (1H, d, J=2.4Hz), 5.32 (1H, d, J=4.4Hz), 5.14 (1H, tt, J=7.8, 1.6Hz), 4.40 (1H, dt, J=10.6, 4.8Hz), 4.33-3.90 (6H, m), 3.52 (1H, dd, J=8.7, 3.9Hz), 2.02 (3H, s), 1.64 (2H, m), 1.52 (9H, s), 1.49 (9H, s), 1.43 (3H, s), 1.36 (3H, s), 1.26 (22H, bs), 0.88 (3H, t, J=6.7Hz);
[α]_{D}²⁴ = -20.9° (c:0.58, CHCl₃).

### vi) Myristyl 5-acetamido-2,3,4,5-tetradeoxy-4-guanidino-D-glycero-D-galacto-non-2-enopyranosoate trifluoroacetic acid salt (E8)

84 mg (0.11 mmol) of the compound (E7) were dissolved in 4.7 ml (50-times volume) of methylene chloride at room temperature, and subsequently 0.85 ml (10-times volume) of trifluoroacetic acid were added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 15 g, ethyl acetate:2-propanol:water = 8:2:1) to obtain 82 mg (yield: 88%) of the title compound (E8) as a white solid.
Rf value: 0.6 (ethyl acetate:2-propanol:water =8:2:1);
¹H-NMR (270MHz, CD₃OD): δ (ppm) 5.83 (1H, d, J=2.7Hz), 4.44 (1H, dd, J=9.0, 2.7Hz), 4.38 (1H, dd, J=9.0, <1Hz), 4.18 (2H, t, J=6.2Hz), 4.17 (1H, t, J=9.0Hz), 3.90-3.74 (2H, m), 3.68 (1H, dd, J=12.0, 4.5Hz), 3.65 (1H, d, J=9.0Hz), 1.99 (3H, s), 1.67 (2H, quintet, J=6.2Hz), 1.26 (24H, bs), 0.87 (3H, t, J=6.2Hz);
FAB-MS (positive): 529 (M+H)⁺;
HR-MS: Calcd. for C₂₆H₄₉N₄O₇, 529.3597, Found 529.3605 (M+H)⁺.

### (Example 2)

### Hexyl 5-acetamido-2,3,4,5-tetradeoxy-4-guanidino-D-glycero-D-galacto-non-2-enopyranosoate trifluoroacetic acid salt (E12) (Exemplary compound No. 87)

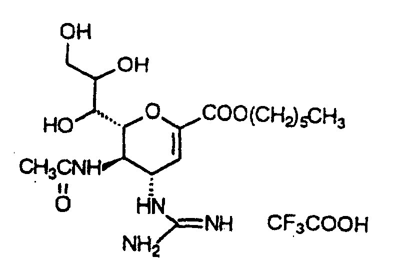

### i) Hexyl 5-acetamido-4-azido-2,3,4,5-tetradeoxy-8,9-O-isopropylidene-D-glycero-D-galacto-non-2-enopyranosoate (E9)

940 mg (2.6 mmol) of the compound (E4), 0.66 ml (5.3 mmol) of 1-hexanol and 1.1 g (4.0 mmol) of 2-bromo-1-ethylpyridinium tetrafluoroborate were dissolved in 47 ml (50-times volume) of dimethylformamide at room temperature. Subsequently, 1.9 ml (8.0 mmol) of tri-n-butylamine were poured into the system, and the mixture was stirred in an oil bath at 50°C for 6 hours. Ethyl acetate and a saturated aqueous NaCl solution were added to the reaction mixture, and the mixture was separated. The organic layer thus obtained was dried over magnesium sulfate and filtered, followed by concentration under reduced pressure. The residue was purified over silica gel column chromatography (Kiesel gel 60, 150 g, benzene:acetonitrile = 2:1) to obtain 560 mg (yield: 44%) of the title compound (E9) as a pale brown solid.
Rf value: 0.43 (benzene:acetonitrile = 2:1);
¹H-NMR (270MHz, CDCl₃, TMS): δ (ppm) 6.53 (1H, d, J=6.9Hz), 5.92 (1H, d, J=2.5Hz), 4.48 (1H, bs), 4.41-4.29 (2H, m), 4.24-4.00 (7H, m), 3.63 (1H, bd, J=7.3Hz), 1.70 (2H, quintet, J=6.5Hz), 1.40 (3H, s), 1.35 (9H, bs), 1.30 (3H, s), 0.89 (3H, t, J=6.5Hz);
[α]_{D}²⁴ = +8° (c=0.4, CHCl₃).

### ii) Hexyl 5-acetamido-4-(N,N'-bis-tert-butoxycarbonyl)guanidino-2,3,4,5-tetradeoxy-8,9-O-isopropylidene-D-glycero-D-galacto-non-2-enopyranosoate (E11)

533 mg (1.1 mmol) of the compound (E9) were dissolved in 25 ml (50-times volume) of ethanol at room temperature, and subsequently 190 mg (0.35-times volume) of Lindlar catalyst were added to the system at room temperature, followed by stirring of the mixture at room temperature under a hydrogen atmosphere of 1 atm. for 2.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 50 g, ethyl acetate:2-propanol:water = 5:2:1) to obtain 303 mg (yield: 60%) of the title compound (E10) as a pale brown foamy solid.
Rf value: 0.44 (ethyl acetate:2-propanol:water = 5:2:1).

290 mg (0.63 mmol) of the resulting compound (E10), 210 mg (0.75 mmol) of N,N'-bis-tert-butoxycarbonylthiourea and 0.22 ml (1.6 mmol) of triethylamine were dissolved in 15 ml (50-times volume) of dimethylformamide at room temperature. Subsequently, 220 mg (0.8 mmol) of mercury chloride were added to the system under ice-cooling and the mixture was stirred at room temperature for 2 hours. The reaction mixture was diluted with ethyl acetate and filtered using Celite, and the filtered product was washed with ethyl acetate. The filtrate thus obtained and the washing solution were combined, and ethyl acetate and a saturated aqueous NaCl solution were added thereto to separate the mixture, followed by extraction of the aqueous layer with ethyl acetate. The organic layer was dried over magnesium sulfate and filtered, followed by concentration under reduced pressure. The residue was purified over silica gel column chromatography (Kiesel gel 60, 75 g, hexane:ethyl acetate = 1:1) to obtain 273 mg (yield: 66%) of the desired compound (E11) as a white solid.
Rf value: 0.38 (benzene:ethyl acetate = 5:1);
¹H-NMR (270MHz, CDCl₃, TMS): δ (ppm) 11.4 (1H, s), 8.64 (1H, d, J=7.7Hz), 7.99 (1H, d, J=4.7Hz), 5.78 (1H, d, J=2.4Hz), 5.33 (1H, d, J=4.4Hz), 5.14 (1H, tt, J=7.9, <1Hz), 4.40 (1H, dt, J=7.8, 5.7Hz), 4.23-3.90 (7H, m), 3.51 (1H, dd, J=8.2, 4.5Hz), 2.01 (3H, s), 1.68 (2H, quintet, J=6.4Hz), 1.51 (9H, s), 1.49 (9H, s), 1.43 (3H, s), 1.36 (3H, s), 1.35-1.20 (6H, m), 0.89 (3H, t, J=6.4Hz);
[α]_{D}²⁴ = -22.0° (c:0.5, CHCl₃).

### iii) Hexyl 5-acetamido-2,3,4,5-tetradeoxy-4-guanidino-D-glycero-D-galacto-non-2-enopyranosoate trifluoroacetic acid salt (E12)

158 mg (0.24 mmol) of the compound (E11) were dissolved in 8.0 ml (50-times volume) of methylene chloride at room temperature, and subsequently 1.6 ml (10-times volume) of trifluoroacetic acid were added to the system at room temperature, followed by stirring of the mixture at room temperature for 18 hours. After confirmation of completion of the reaction, the reaction mixture was concentrated under reduced pressure. The residue was purified over silica gel column chromatography (Kiesel gel 60, 15 g, ethyl acetate:2-propanol:water = 5:2:1) to obtain 155 mg (yield: 100%) of the title compound (E12) as a white solid.
Rf value: 0.8 (ethyl acetate:2-propanol:water = 5:2:1);
¹H-NMR (270MHz, CD₃OD): δ (ppm) 5.94 (1H, d, J=2.4Hz), 4.46 (1H, dd, J=9.3, 2.4Hz), 4.38 (1H, dd, J=10.4, 1.3Hz), 4.20 (1H, t, J=9.3Hz), 4.20 (2H, t, J=6.2Hz), 3.91-3.76 (2H, m), 3.66 (1H, bd, J=9.3Hz), 3.61 (1H, dd, J=12.5, 6.3Hz), 1.97 (3H, s), 1.63 (2H, quintet, J=6.3Hz), 1.40-1.10 (6H, m), 0.80 (3H, t, J=6.3Hz);
[α]_{D}²⁴ = +18.1° (c=0.48, CHCl₃).

### (Example 3)

### 5-Acetamido-2,3,4,5-tetradeoxy-4-guanidino-9-O-myristoyl-D-glycero-D-galacto-non-2-enopyranosoic acid trifluoroacetic acid salt (E17) (Exemplary compound No. 41)

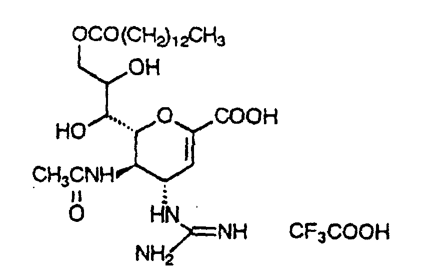

### i) Methyl 5-acetamido-4-azido-2,3,4,5-tetradeoxy-8,9-O-isopropylidene-7-O-myristoyl-D-glycero-D-galacto-non-2-enopyranosoate (E13)

61 mg (0.16 mmol) of the compound (E3) were dissolved in 3.0 ml (50-times volume) of methylene chloride at room temperature, and subsequently 54 ml (0.2 mmol) of myristoyl chloride and 24.1 mg (0.2 mmol) of 4-dimethylaminopyridine were added to the system under ice-cooling, followed by stirring of the mixture at room temperature for 30 minutes. Next, 27 ml (0.2 mmol) of triethylamine were poured into the reaction mixture at room temperature, followed by further stirring of the mixture for 6 hours. After confirmation of completion of the reaction, methanol was poured into the system, followed by stirring of the mixture for 30 minutes. Next, ethyl acetate and a saturated aqueous NaCl solution were added to the reaction mixture to separate the mixture. The organic layer thus obtained was dried over magnesium sulfate and filtered, followed by concentration under reduced pressure. The residue was purified over silica gel column chromatography (Kiesel gel 60, 15 g, benzene:ethyl acetate = 1:1) to obtain 70 mg (yield: 74%) of the desired compound (E13) as a colorless transparent syrup.
Rf value: 0.42 (benzene:ethyl acetate = 2:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9.1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (20H, s), 0.88 (3H, t, J=7.5Hz).

### ii) Methyl 5-acetamido-4-amino-2,3,4,5-tetradeoxy-8,9-O-isopropylidene-7-O-myristoyl-D-glycero-D-galacto-non-2-enopyranosoate (E14)

70 mg (0.12 mmol) of the compound (E13) were dissolved in 3.5 ml (50-times volume) of ethanol at room temperature, and subsequently 25 mg (0.35-times volume) of Lindlar catalyst were added to the system at room temperature, followed by stirring of the mixture at room temperature under a hydrogen atmosphere of 1 atm. for 1.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 15 g, ethyl acetate:2-propanol:water = 5:2:1) to obtain 57 mg (yield: 84%) of the title compound (E14) as a white solid.
Rf value: 0.49 (methylene chloride:methanol = 10:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (20H, s), 0.88 (3H, t, J=7.3Hz).

### iii) Methyl 5-acetamido-4-(N,N'-bis-tert-butoxycarbonyl)guanidino-2,3,4,5-tetradeoxy-8,9-O-isopropylidene-7-O-myristoyl-D-glycero-D-galacto-non-2-enopyranosoate (E15)

57 mg (0.1 mmol) of the compound (E14), 34 mg (0.12 mmol) of N,N'-bis-tert-butoxycarbonylthiourea and 35 ml (0.25 mmol) of triethylamine were dissolved in 28 ml (50-times volume) of dimethylformamide at room temperature. Subsequently, 35 mg (0.13 mmol) of mercury chloride were added to the system under ice-cooling, and the mixture was stirred at room temperature for 2 hours. Ethyl acetate was added to the reaction mixture to dilute it, followed by filtration using Celite. The filtered product was washed with ethyl acetate. The filtrate thus obtained and the washing solution were combined, and ethyl acetate and a saturated aqueous NaCl solution were added to the mixture to separate it. The organic layer was dried over magnesium sulfate and filtered, followed by concentration under reduced pressure. The residue was purified over silica gel column chromatography (Kiesel gel 60, 15 g, hexane:ethyl acetate = 2:1) to obtain 75 mg (yield: 100%) of the title compound as a colorless transparent syrup.
Rf value: 0.33 (hexane:ethyl acetate = 2:1);
¹H-NMR (270MHz, CDCl₃, TMS): δ (ppm) 11.4 (1H, s), 8.46 (1H, d, J=8.7Hz), 6.06 (1H, d, J=8.7Hz), 5.88 (1H, d, J=2.4Hz), 5.37 (1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87 (3H, s), 1.61 (2H, quintet, J=7.5Hz), 1.49 (9H, s), 1.48 (9H, s), 1.38 (3H, s), 1.35 (3H, s), 1.25 (20H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-myristoyl-D-glycero-D-galacto-non-2-enopyranosoic acid (E16)

51 mg (0.07 mmol) of the compound (E15) were dissolved in a mixture of 2 ml (40-times volume) of methanol and 0.5 ml (10-times volume) of water, and 3.3 mg (0.078 mmol) of lithium hydroxide monohydrate were added to the system at room temperature, followed by stirring of the mixture at room temperature for 8 hours. After confirmation of completion of the reaction, Dowex-50W was added to the system to neutralize the mixture. The reaction mixture was filtered, and the filtered product was washed with methanol. The filtrate and the washing solution were combined and concentrated under reduced pressure. The residue was purified over silica gel column chromatography (Kiesel gel 60, 60 g, ethyl acetate:2-propanol:water = 10:2:1) to obtain 33 mg (yield: 66%) of the desired compound (E16) as a white solid.
Rf value: 0.45 (methylene chloride:methanol = 10:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (18H, s), 1.39 (3H, s), 1.37 (3H, s), 1.25 (20H, bs), 0.88 (3H, t, J=6.5Hz).

### v) 5-Acetamido-2,3,4,5-tetradeoxy-4-guanidino-9-O-myristoyl-D-glycero-D-galacto-non-2-enopyranosoic acid trifluoroacetic acid salt (E17)

33 mg (0.046 mmol) of the compound (E16) were dissolved in 1.6 ml (50-times volume) of methylene chloride at room temperature, and subsequently 60 ml (10-times volume) of trifluoroacetic acid were added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, 2-propanol:water = 5:1) to obtain 30 mg (yield: 84%) of the title compound (E17) as a pale yellow solid.
Rf value: 0.4 (2-propanol:water = 5:1);
¹H-NMR (270MHz, CD₃OD): δ (ppm) 5.63 (1H, d, J=2.3Hz), 4.49 (1H, dd, J=9.3, 2.3Hz), 4.39 (1H, d, J=10.6Hz), 4.25-4.00 (4H, m), 3.77 (1H, d, J=9.3Hz), 2.36 (2H, t, J=7.4Hz), 1.92 (3H, s), 1.70-1.50 (2H, m), 1.30 (20H, bs), 0.90 (3H, t, J=7.4Hz);
FAB-MS (positive): 543 (M+H)⁺;
HR-MS Calcd. for C₂₆H₄₇O₈N₄, 543.3378, Found 543.3412 (M+H)⁺;
[α]_{D}²⁴ = +25° (c=0.12, MeOH).

### (Example 4)

### 5-Acetamido-2,3,4,5-tetradeoxy-4-guanidino-9-O-hexanoyl-D-glycero-D-galacto-non-2-enopyranosoic acid trifluoroacetic acid salt (E22) (Exemplary compound No. 36)

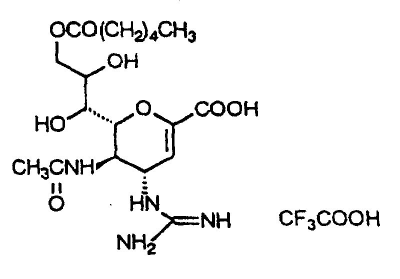

### i) Methyl 5-acetamido-4-azido-2,3,4,5-tetradeoxy-8,9-O-isopropylidene-7-O-hexanoyl-D-glycero-D-galacto-non-2-enopyranosoate (E18)

270 mg (0.73 mmol) of the compound (E3) were dissolved in 13.5 ml (50-times volume) of methylene chloride, and subsequently 0.14 ml (1.0 mmol) of hexanoyl chloride and 110 mg (0.9 mmol) of 4-dimethylaminopyridine were added to the system under ice-cooling, followed by stirring of the mixture at room temperature for 30 minutes. Next, 0.13 ml (0.9 mmol) of triethylamine were poured into the reaction mixture at room temperature, followed by further stirring of the mixture for 19 hours. After confirmation of completion of the reaction, methanol was poured into the system, followed by stirring of the mixture for 30 minutes. Next, ethyl acetate and a saturated aqueous NaCl solution were added to the reaction mixture to separate the mixture. The organic layer was dried over magnesium sulfate and filtered, followed by concentration under reduced pressure. The residue thus obtained was purified over silica gel column chromatography (Kiesel gel 60, 75 g, benzene:ethyl acetate = 1:1) to obtain 220 mg (yield: 74%) of the title compound (E18) as a colorless transparent syrup.
Rf value: 0.45 (benzene:ethyl acetate = 1:1);
¹H-NMR (270MHz, CDCl₃, TMS): δ (ppm) 6.11 (1H, d, J=8.0Hz), 5.95 (1H, d, J=2.6Hz), 5.38 (1H, d, J=5.3, 2.0Hz), 4.69 (1H, dd, J=9.2, 2.6Hz), 4.65 (1H, dd, J=10.6, 2.0Hz), 4.38 (1H, q, J=5.5Hz), 4.14 (1H, dd, J=9.5, 5.5Hz), 3.95 (1H, dd, J=9.5, 5.4Hz), 3.81 (3H, s), 3.59 (1H, ddd, J=10.6, 9.2, 8.0Hz), 2.44 (1H, dt, J=15.9, 7.4Hz), 2.36 (1H, dt, J=15.9, 7.4Hz), 2.01 (3H, s), 1.64 (2H, quintet, J=7.4Hz), 1.37 (3H, s), 1.35 (3H, s), 1.35-1.30 (4H, m). 0.90 (3H, t, J=7.4Hz);
[α]_{D}²⁴ = +94.3° (c:0.35, CHCl₃).

### ii) Methyl 5-acetamido-4-(N,N'-bis-tert-butoxycarbonyl)guanidino-2,3,4,5-tetradeoxy-8,9-O-isopropylidene-7-O-hexanoyl-D-glycero-D-galacto-non-2-enopyranosoate (E20)

190 mg (0.41 mmol) of the compound (E18) were dissolved in 9.3 ml (50-times volume) of ethanol at room temperature, and subsequently 70 mg (0.38-times volume) of Lindlar catalyst were added to the system at room temperature, followed by stirring of the mixture at room temperature under a hydrogen atmosphere of 1 atm. for 2.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 60 g, ethyl acetate:2-propanol:water = 5:2:1) to obtain 140 mg (yield: 80%) of the compound (E19) as a pate yellow foamy solid.
Rf value: 0.4 (ethyl acetate:2-propanol = 5:2:1).

140 mg (0.3 mmol) of the resulting compound (E19), 120 mg (0.43 mmol) of N,N'-bis-tert-butoxycarbonylthiourea and 0.12 ml (0.86 mmol) of triethylamine were dissolved in 7 ml (50-times volume) of dimethylformamide at room temperature. Subsequently, 110 mg (0.41 mmol) of mercury chloride were added to the system under ice-cooling, followed by stirring of the mixture at room temperature for 1.5 hours. Ethyl acetate was added to the reaction mixture to dilute it, and the mixture was filtered using Celite, followed by washing of the filtered product with ethyl acetate. The filtrate and the washing solution were combined, and ethyl acetate and a saturated aqueous NaCI solution were added thereto to separate it. The organic layer was dried over magnesium sulfate and filtered, followed by concentration under reduced pressure. The residue was purified over silica gel column chromatography (Kiesel gel 60, 50 g, benzene:ethyl acetate = 3:1) to obtain 220 mg (yield: 100%) of the title compound (E20) as a white foamy solid.
Rf value: 0.5 (benzene:ethyl acetate = 3:1);
¹H-NMR (270MHz, CDCl₃, TMS): δ (ppm) 11.4 (1H, s), 8.46 (1H, d, J=8.7Hz), 5.97 (1H, d, J=8.7Hz), 5.88 (1H, d, J=2.3Hz), 5.37 (1H, dd, J=6.3, 1.6Hz), 5.15 (1H, dt, J=8.7, 2.3Hz), 4.37 (1H, q, J=6.4Hz), 4.29 (1H, dd, J=8.7, 1.5Hz), 4.24 (1H, t, J=8.7Hz), 4.11 (1H, dd, J=8.8, 6.4Hz), 3.96 (1H, dd, J=8.8, 6.4Hz), 3.81 (3H, s), 2.46 (1H, dt, J=15.9, 7.4Hz), 2.33 (1H, dt, J=15.9, 7.4Hz), 1.88 (3H, s), 1.70-1.50 (2H, m), 1.49 (9H, s), 1.48 (9H, s), 1.38 (3H, s), 1.37 (3H, s), 1.40-1.25 (4H, m), 0.89 (3H, t, J=7.4Hz);
[α]_{D}²⁴ = -28.3° (c=0.4, CHCl₃).

### iii) 5-Acetamido-4-(N,N'-bis-tert-butoxycarbonyl)guanidino-2,3,4,5-tetradeoxy-8,9-O-isopropylidene-7-O-hexanoyl-D-glycero-D-galacto-non-2-enopyranosoic acid (E21)

180 mg (0.26 mmol) of the compound (E20) were dissolved in a mixture of 10.8 ml (60-times volume) of methanol and 1.8 ml (10-times volume) of water, and 12 mg (0.29 mmol) of lithium hydroxide monohydrate were added to the system at room temperature, followed by stirring of the mixture at room temperature for 17 hours. After confirmation of completion of the reaction, Dowex-50W was added to the system to neutralize it. The reaction mixture was filtered, and the filtered product was washed with methanol. The filtrate and the washing solution were combined and concentrated under reduced pressure. The residue was purified over silica gel column chromatography (Kiesel gel 60, 50 g, methylene chloride:methanol = 10:1) to obtain 0.13 g (yield: 75%) of the title compound (E21) as a white foamy solid.
Rf value: 0.24 (methylene chloride:methanol = 10:1);
¹H-NMR (270MHz, CDCl₃, TMS): δ (ppm) 11.4 (1H, bs), 8.52 (1H, bs), 5.95 (2H, bs), 5.32 (1H, bs), 5.15 (1H, bs), 4.60-4.10 (3H, m), 3.95 (1H, bs), 2.70-2.20 (2H, m), 1.89 (3H, s), 1.75-1.00 (28H, m), 0.85 (3H, m).

### iv) 5-Acetamido-2,3,4,5-tetradeoxy-4-guanidino-9-O-hexanoyl-D-glycero-D-galacto-non-2-enopyranosoic acid trifluoroacetic acid salt (E22)

95 mg (0.15 mmol) of the compound (E21) were dissolved in 4.8 ml (50-times volume) of methylene chloride, and subsequently 0.95 ml (10-times volume) of trifluoroacetic acid were added to the system at room temperature, followed by stirring of the mixture at room temperature for 4.5 hours. After confirmation of completion of the reaction, the reaction mixture was concentrated under reduced pressure. The residue was purified over silica gel column chromatography (Kiesel gel 60, 20 g, ethyl acetate:2-propanol:water = 4:2:1) to obtain 54 mg (yield: 57%) of the desired compound as a white solid.
Rf value: 0.3 (ethyl acetate:2-propanol:water = 4:2:1);
¹H-NMR (270MHz, CD₃OD): δ (ppm) 5.53 (1H, d, J=2.2Hz), 4.50-4.00 (6H, m), 3.59 (1H, d, J=9.2Hz), 2.35 (2H, t, J=7.5Hz), 2.01 (3H, s), 1.70-1.50 (2H, m), 1.40-1.20 (4H, m), 0.91 (3H, t, J=7.5Hz);
IR (KBr) (cm⁻¹) 3333, 1665, 1617, 1401, 1385, 1176;
FAB-MS (positive) 431 (M+H)⁺;
HR-MS Calcd. for C₁₈H₃₁N₄O₈ 431.2165, Found 431.2125 (M+H)⁺;
[α]_{D}²⁴ = +29° (c=0.2, MeOH).

### (Example 5)

### 5-Acetamido-2,3,4,5-tetradeoxy-4-guanidino-9-O-octanoyl-D-glycero-D-galacto-non-2-enopyranosoic acid trifluoroacetic acid salt (E27) (Exemplary compound No. 38)

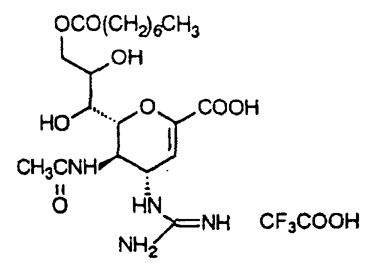

### i) Methyl 5-acetamido-4-azido-2,3,4,5-tetradeoxy-8,9-O-isopropylidene-7-O-myristoyl-D-glycero-D-galacto-non-2-enopyranosoate (E23)

185 mg (0.5 mmol) of the compound (E3) were dissolved in 5.0 ml of methylene chloride at room temperature, and 132 µl (0.75 mmol) of octanoyl chloride and 91 mg (0.75 mmol) of 4-dimethylaminopyridine were added to the system under ice-cooling, followed by stirring of the mixture at room temperature for 30 minutes. Next, 104 µl (0.75 mmol) of triethylamine were poured into the reaction mixture at room temperature, followed by further stirring of the mixture for 6 hours. After confirmation of completion of the reaction, methanol was poured into the system, followed by stirring of the mixture for 30 minutes. Next, ethyl acetate and a saturated aqueous NaCI solution were added to the reaction mixture to separate it. The organic layer thus obtained was dried over magnesium sulfate and filtered, followed by concentration under reduced pressure. The residue was purified over silica gel column chromatography (Kiesel gel 60, methylene chloride:methanol = 50:1) to obtain 150 mg (yield: 64%) of the desired compound (E23) as a colorless transparent syrup.
Rf value: 0.50 (methylene chloride:methanol = 20:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9.1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (8H, s), 0.88 (3H, t, J=7.5Hz).

### ii) Methyl 5-acetamido-4-amino-2,3,4,5-tetradeoxy-8,9-O-isopropylidene-7-O-octanoyl-D-glycero-D-galacto-non-2-enopyranosoate (E24)

130 mg (0.28 mmol) of the compound (E23) were dissolved in 6.0 ml of ethanol at room temperature, and subsequently 46 mg (0.35-times volume) of Lindlar catalyst were added to the system at room temperature, followed by stirring of the mixture at room temperature under a hydrogen atmosphere of 1 atm. for 1.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 15 g, ethyl acetate:2-propanol:water = 5:2:1) to obtain 95 mg (yield: 77%) of the title compound (E24) as a white solid.
Rf value: 0.35 (n-hexane:ethyl acetate = 2:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (8H, s), 0.88 (3H, t, J=7.3Hz).

### iii) Methyl 5-acetamido-4-(N,N'-bis-tert-butoxycarbonyl)guanidino-2,3,4,5-tetradeoxy-8,9-O-isopropylidene-7-O-octanoyl-D-glycero-D-galacto-non-2-enopyranosoate (E25)

90 mg (0.2 mmol) of the compound (E24), 83 mg (0.30 mmol) of N,N'-bis-tert-butoxycarbonylthiourea and 84 µl (0.25 mmol) of triethylamine were dissolved in 5 ml of dimethylformamide at room temperature. Subsequently, 82 mg (0.30 mmol) of mercury chloride were added to the system under ice-cooling, followed by stirring of the mixture at room temperature for 2 hours. Ethyl acetate was added to the reaction mixture to dilute it and the mixture was filtered using Celite. The filtered product was washed with ethyl acetate. The filtrate thus obtained and the washing solution were combined, and ethyl acetate and a saturated aqueous NaCI solution were added to the mixture to separate it. The organic layer was dried over magnesium sulfate and filtered, followed by concentration under reduced pressure. The residue was purified over silica gel column chromatography (Kiesel gel 60, 15 g, hexane:ethyl acetate = 2:1) to obtain 120 mg (yield: 88%) of the title compound (E25) as a colorless transparent syrup.
Rf value: 0.30 (hexane:ethyl acetate = 2:1);
¹H-NMR (270MHz, CDCl₃, TMS): δ (ppm) 11.4 (1H, s), 8.46 (1H, d, J=8.7Hz), 6.06 (1H, d, J=8.7Hz), 5.88 (1H, d, J=2.4Hz), 5.37 (1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87 (3H, s), 1.61 (2H, quintet, J=7.5Hz), 1.49 (9H, s), 1.48 (9H, s), 1.38 (3H, s), 1.35 (3H, s), 1.25 (8H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-octanoyl-D-glycero-D-galacto-non-2-enopyranosoic acid (E26)

100 mg (0.15 mmol) of the compound (E25) were dissolved in a mixture of 4 ml (40-times volume) of methanol and 1 ml (10-times volume) of water, and 7.0 mg (0.165 mmol) of lithium hydroxide monohydrate were added to the system at room temperature, followed by stirring of the mixture at room temperature for 8 hours. After confirmation of completion of the reaction, Dowex-50W was added to the system to neutralize it. The reaction mixture was filtered, and the filtered product was washed with methanol. The filtrate and the washing solution were combined and concentrated under reduced pressure. The residue thus obtained was purified over silica gel column chromatography (Kiesel gel 60, ethyl acetate:2-propanol:water= 10:2:1) to obtain 56 mg (yield: 56%) of the desired compound (E26) as a white solid.
Rf value: 0.35 (methylene chloride:methanol = 10:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (18H, s), 1.39 (3H, s), 1.37 (3H, s), 1.25 (8H, bs), 0.88(3H, t, J=6.5Hz).

### v) 5-Acetamido-2,3,4,5-tetradeoxy-4-guanidino-9-O-octanoyl-D-glycero-D-galacto-non-2-enopyranosoic acid trifluoroacetic acid salt (E27)

50 mg (0.075 mmol) of the compound (E26) were dissolved in 3 ml (50-times volume) of methylene chloride at room temperature, and subsequently 1 ml (10-times volume) of trifluoroacetic acid was added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, 2-propanol:water = 5:1) to obtain 38 mg (yield: 88%) of the title compound (E27) as a pale yellow solid.
Rf value: 0.3 (2-propanol:water = 5:1);
¹H-NMR (270MHz, CD₃OD): δ (ppm) 5.55 (1H, bs), 4.40-4.10 (7H, m), 3.65 (1H, d, J=9.0Hz), 2.36 (2H, t, J=7.0Hz), 2.00 (3H, s), 1.70-1.50 (2H, m), 1.30 (8H, bs), 0.90 (3H, t, J=7.0Hz);
FAB-MS (positive): 459 (M+H)⁺;
[α]_{D}²⁴ = +19.2° (c=0.26, MeOH).

### (Example 6)

### 5-Acetamido-2,3,4,5-tetradeoxy-4-guanidino-9-O-decanoyl-D-glycero-D-galacto-non-2-enopyranosoic acid trifluoroacetic acid salt (E32) (Exemplary compound No. 39)

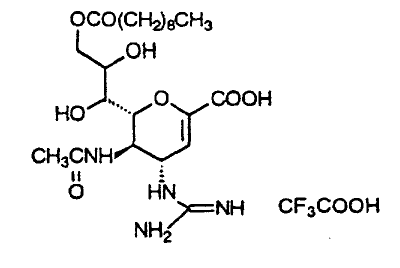

### i) Methyl 5-acetamido-4-azido-2,3,4,5-tetradeoxy-8,9-O-isopropylidene-7-O-decanoyl-D-glycero-D-galacto-non-2-enopyranosoate (E28)

214 mg (0.58 mmol) of the compound (E3) were dissolved in 6.0 ml of methylene chloride at room temperature, and subsequently 152 µl (0.87 mmol) of decanoyl chloride and 106 mg (0.87 mmol) of 4-dimethylaminopyridine were added to the system under ice-cooling, followed by stirring of the mixture at room temperature for 30 minutes. Next, 106 µl (0.87 mmol) of trimethylamine were poured into the reaction mixture at room temperature, followed by further stirring of the mixture for 6 hours. After confirmation of completion of the reaction, methanol was poured into the system and the mixture was stirred for 30 minutes. Next, ethyl acetate and a saturated aqueous NaCI solution were added to the reaction mixture to separate it. The organic layer thus obtained was dried over magnesium sulfate and filtered, followed by concentration under reduced pressure. The residue was purified over silica gel column chromatography (Kiesel gel 60, methylene chloride: methanol = 50:1) to obtain 180 mg (yield: 63%) of the desired compound (E28) as a colorless transparent syrup.
Rf value: 0.56 (methylene chloride:methanol = 20:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9.1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (12H, s), 0.88 (3H, t, J=7.5Hz).

### ii) Methyl 5-acetamido-4-amino-2,3,4,5-tetradeoxy-8,9-O-isopropylidene-7-O-decanoyl-D-glycero-D-galacto-non-2-enopyranosoate (E29)

170 mg (0.32 mmol) of the compound (E28) were dissolved in 10 ml of ethanol at room temperature, and subsequently 60 mg (0.35-times volume) of Lindlar catalyst were added to the system at room temperature, followed by stirring of the mixture at room temperature under a hydrogen atmosphere of 1 atm. for 1.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 15 g, ethyl acetate:2-propanol:water = 5:2:1) to obtain 120 mg (yield: 80%) of the title compound (E29) as a white solid.
Rf value: 0.40 (n-hexane:ethyl acetate = 2:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (12H, s), 0.88 (3H, t, J=7.3Hz).

### iii) Methyl 5-acetamido-4-(N,N'-bis-tert-butoxycarbonyl)guanidino-2,3,4,5-tetradeoxy-8,9-O-isopropylidene-7-O-decanoyl-D-glycero-D-galacto-non-2-enopyranosoate (E30)

100 mg (0.21 mmol) of the compound (E29), 87 mg (0.31 mmol) of N,N'-bis-tert-butoxycarbonylthiourea and 87 µl (0.63 mmol) of triethylamine were dissolved in 5 ml of dimethylformamide at room temperature. Subsequently, 84 mg (0.31 mmol) of mercury chloride were added to the system under ice-cooling, and the mixture was stirred at room temperature for 2 hours. Ethyl acetate was added to the reaction mixture to dilute it and the mixture was filtered using Celite. The filtered product was washed with ethyl acetate. The filtrate thus obtained and the washing solution were combined, and ethyl acetate and a saturated aqueous NaCl solution were added thereto to separate the mixture. The organic layer was dried over magnesium sulfate and filtered, followed by concentration under reduced pressure. The residue was purified over silica gel column chromatography (Kiesel gel 60, hexane:ethyl acetate = 2:1) to obtain 120 mg (yield: 87%) of the title compound (E30) as a colorless transparent syrup.
Rf value: 0.30 (hexane:ethyl acetate = 2:1);
¹H-NMR (270MHz, CDCl₃, TMS): δ (ppm) 11.4 (1H, s), 8.46 (1H, d, J=8.7Hz), 6.06 (1H, d, J=8.7Hz), 5.88 (1H, d, J=2.4Hz), 5.37 (1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87(3H, s), 1.61 (2H, quintet, J=7.5Hz), 1.49 (9H, s), 1.48 (9H, s), 1.38 (3H, s), 1.35 (3H, s), 1.25 (12H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-decanoyl-D-glycero-D-galacto-non-2-enopyranosoic acid (E31)

100 mg (0.14 mmol) of the compound (E30) were dissolved in a mixture of 4 ml (40-times volume) of methanol and 1 ml (10-times volume) of water, and 6.5 mg (0.154 mmol) of lithium hydroxide monohydrate were added to the system at room temperature, followed by stirring of the mixture at room temperature for 8 hours. After confirmation of completion of the reaction, Dowex-50W was added to the system to neutralize it. The reaction mixture was filtered, and the filtered product was washed with methanol. The filtrate and the washing solution were combined and concentrated under reduced pressure. The residue was purified over silica gel column chromatography (Kiesel gel 60, ethyl acetate:2-propanol:water = 10:2:1) to obtain 53 mg (yield: 55%) of the desired compound (E31) as a white solid.
Rf value: 0.38 (methylene chloride:methanol = 10:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (18H, s), 1.39 (3H, s), 1.37 (3H, s), 1.25 (12H, bs), 0.88 (3H, t, J=6.5Hz).

### v) 5-Acetamido-2,3,4,5-tetradeoxy-4-guanidino-9-O-decanoyl-D-glycero-D-galacto-non-2-enopyranosoic acid trifluoroacetic acid salt (E32)

40 mg (0.057 mmol) of the compound (E31) were dissolved in 3 ml (50-times volume) of methylene chloride at room temperature, and subsequently 1 ml (10-times volume) of trifluoroacetic acid was added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, 2-propanol:water = 5:1) to obtain 30 mg (yield: 87%) of the title compound (E32) as a pale yellow solid.
Rf value: 0.3 (2-propanol:water = 5:1);
¹H-NMR (270MHz, CD₃OD): δ (ppm) 5.55 (1H, bs), 4.40-4.10 (7H, m), 3.65 (1H, d, J=9.0Hz), 2.36 (2H, t, J=7.0Hz), 2.00 (3H, s), 1.70-1.50 (2H, m), 1.30 (12H, bs), 0.90 (3H, t, J=7.0Hz);
FAB-MS (positive): 487(M+H)⁺;
[α]_{D}²⁴ = +17.2° (c=0.15, MeOH).

### (Example 7)

### 5-Acetamido-2,3,4,5-tetradeoxy-4-guanidino-9-O-palmitoyl-D-glycero-D-galacto-non -2-enopyranosoic acid trifluoroacetic acid salt (E37) (Exemplary compound No. 42)

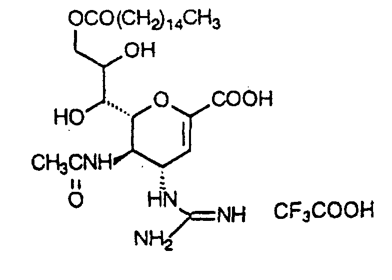

### i) Methyl 5-acetamido-4-azido-2,3,4,5-tetradeoxy-8,9-O-isopropylidene-7-O-palmitoyl-D-glycero-D-galacto-non-2-enopyranosoate (E33)

1.35 g (3.66 mmol) of the compound (E3) were dissolved in 10 ml of methylene chloride at room temperature, and subsequently 1.88 ml (6.22 mmol) of palmitoyl chloride and 759 mg (4.33 mmol) of 4-dimethylaminopyridine were added to the system under ice-cooling, followed by stirring of the mixture at room temperature for 30 minutes. Next, 104 µl (6.21 mmol) of triethylamine were poured into the reaction mixture at room temperature, and the resulting mixture was further stirred for 15 hours. After confirmation of completion of the reaction, methanol was poured to the system, and the mixture was stirred for 30 minutes. Next, ethyl acetate and a saturated aqueous NaCl solution were added to the reaction mixture to separate it. The organic layer thus obtained was dried over magnesium sulfate and filtered, followed by concentration under reduced pressure. The residue was purified over silica gel column chromatography (Kiesel gel 60, n-hexane:ethyl acetate = 2:1) to obtain 1.42 g (yield: 64%) of the desired compound (E33) as a colorless foam.
Rf value: 0.53 (n-hexane:ethyl acetate = 1:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9.1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (24H, s), 0.88 (3H, t, J=7.5Hz).

### ii) Methyl 5-acetamido-4-amino-2,3,4,5-tetradeoxy-8,9-O-isopropylidene-7-O-palmitoyl-D-glycero-D-galacto-non-2-enopyranosoate (E34)

1.42 g (2.34 mmol) of the compound (E33) were dissolved in 15 ml of ethanol at room temperature, and subsequently 477 mg (0.34-times volume) of Lindlar catalyst were added to the system at room temperature, followed by stirring of the mixture. at room temperature under a hydrogen atmosphere of 1 atm. for 2 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 15 g, ethyl acetate:methanol = 10:1) to obtain 1.16 g (yield: 85%) of the title compound (E34) as a colorless foam.
Rf value: 0.18 (ethyl acetate:methanol = 5:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (24H, s), 0.88 (3H, t, J=7.3Hz).

### iii) Methyl 5-acetamido-4-(N,N'-bis-tert-butoxycarbonyl)guanidino-2,3,4,5-tetradeoxy-8,9-O-isopropylidene-7-O-palmitoyl-D-glycero-D-galacto-non-2-enopyranosoate (E35)

1.16 g (2.0 mmol) of the compound (E34), 828 mg (2.99 mmol) of N,N'-bis-tert-butoxycarbonylthiourea and 0.42 ml (3.03 mmol) of triethylamine were dissolved in 12 ml of dimethylformamide at room temperature. Subsequently, 813 mg (2.99 mmol) of mercury chloride were added to the system under ice-cooling, and the mixture was stirred at room temperature for 2 hours. Ethyl acetate was added to the reaction mixture to dilute it and the mixture was filtered using Celite. The filtered product was washed with ethyl acetate. The filtrate thus obtained and the washing solution were combined, and ethyl acetate and a saturated aqueous NaCl solution were added thereto to separate the mixture. The organic layer was dried over magnesium sulfate and filtered, followed by concentration under reduced pressure. The residue was purified over silica gel column chromatography (Kiesel gel 60, 15 g, hexane:ethyl acetate = 2:1) to obtain 1.0 g (yield: 61%) of the title compound (E35) as a colorless foam.
Rf value: 0.52 (hexane:ethyl acetate = 2:1);
¹H-NMR (270MHz, CDCl₃, TMS): δ (ppm) 11.4 (1H, s), 8.46 (1H, d, J=8.7Hz), 6.06(1H, d, J=8.7Hz), 5.88 (1H, d, J=2.4Hz), 5.37(1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87 (3H, s), 1.61 (2H, quintet, J=7.5Hz), 1.49 (9H, s), 1.48 (9H, s), 1.38 (3H, s), 1.35 (3H, s), 1.25 (24H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-palmitoyl-D-glycero-D-galacto-non-2-enopyranosoic acid (E36)

61 mg (0.07 mmol) of the compound (E35) were dissolved in a mixture of 1.2 ml (40-times volume) of methanol and 0.15 ml (10-times volume) of water, and 3.4 mg (0.08 mmol) of lithium hydroxide monohydrate were added to the system at room temperature, followed by stirring of the mixture at room temperature for 3 hours. After confirmation of completion of the reaction, Dowex-50W was added to the system to neutralize it, and the reaction mixture was filtered. The filtered product was washed with methanol. The filtrate and the washing solution were combined, and the mixture was concentrated under reduced pressure. The residue was purified over silica gel column chromatography (Kiesel gel 60, ethyl acetate:methanol = 10:1) to obtain 33 mg (yield: 55%) of the desired compound (E36) as a colorless foam.
Rf value: 0.61 (ethyl acetate:methanol = 5:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (24H, s), 1.39 (3H, s), 1.37 (3H, s), 1.25 (8H, bs), 0.88 (3H, t, J=6.5Hz).

### v) 5-Acetamido-2,3,4,5-tetradeoxy-4-guanidino-9-O-palmitoyl-D-glycero-D-galacto-non-2-enopyranosoic acid trifluoroacetic acid salt (E37)

289 mg (0.36 mmol) of the compound (E36) were dissolved in 3 ml (50-times volume) of methylene chloride at room temperature, and subsequently 1 ml (10-times volume) of trifluoroacetic acid was added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, ethyl acetate:2-propanol:water = 5.2:1) to obtain 206 mg (yield: 84%) of the title compound (E37) as a colorless foam.
Rf value: 0.44 (2-propanol:water = 5:1);
¹H-NMR (270MHz, CD₃OD): δ (ppm) 5.55 (1H, bs), 4.40-4.10 (7H, m), 3.65 (1H, d, J=9.0Hz), 2.36 (2H, t, J=7.0Hz), 2.00 (3H, s), 1.70-1.50 (2H, m), 1.30 (24H, bs), 0.90 (3H, t, J=7.0Hz);
FAB-MS (positive): 571 (M+H)⁺;
[α]_{D}²⁴ = +18.5° (c=0.12, MeOH).

### (Example 8)

### 5-Acetamido-2,3,4,5-tetradeoxy-4-guanidino-9-O-dodecanoyl-D-glycero-D-galacto-non-2-enopyranosoic acid trifluoroacetic acid salt (E42) (Exemplary compound No. 40)

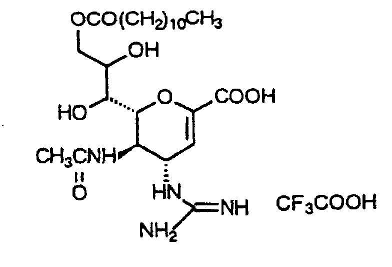

### i) Methyl 5-acetamido-4-azido-2,3,4,5-tetradeoxy-8,9-O-isopropylidene-7-O-dodecanoyl-D-glycero-D-galacto-non-2-enopyranosoate (E38)

1.07 g (2.89 mmol) of the compound (E3) were dissolved in 11 ml of methylene chloride at room temperature, and subsequently 1.03 ml (4.33 mmol) of dodecanoyl chloride and 529 mg (4.33 mmol) of 4-dimethylaminopyridine were added to the system under ice-cooling, followed by stirring of the mixture at room temperature for 30 minutes. Next, 0.6 ml (4.33 mmol) of triethylamine were poured into the reaction mixture at room temperature, and the mixture was further stirred for 2.5 hours. After confirmation of completion of the reaction, methanol was poured into the system, and the mixture was stirred for 30 minutes. Next, ethyl acetate and a saturated aqueous NaCI solution were added to the reaction mixture to separate it. The organic layer thus obtained was dried over magnesium sulfate and filtered, followed by concentration under reduced pressure. The residue was purified over silica gel column chromatography (Kiesel gel 60, n-hexane:ethyl acetate = 2:1) to obtain 1.07 mg (yield: 67%) of the desired compound (E38) as a colorless foam.
Rf value: 0.44 (n-hexane:ethyl acetate = 1:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9,1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (16H, s), 0.88 (3H, t, J=7.5Hz).

### ii) Methyl 5-acetamido-4-amino-2,3,4,5-tetradeoxy-8,9-O-isopropylidene-7-O-dodecanoyl-D-glycero-D-galacto-non-2-enopyranosoate (E39)

1.06 g (1.92 mmol) of the compound (E38) were dissolved in 10 ml of ethanol at room temperature, and subsequently 353 mg (0.33-times volume) of Lindlar catalyst were added to the system at room temperature, followed by stirring of the mixture at room temperature under a hydrogen atmosphere of 1 atm. for 1.5 hours. After confirmation of completion of the reaction, the reaction mixture was filtered. The filtered product was washed with ethanol, and the filtrate and the washing solution were combined, followed by concentration under reduced pressure. The residue was purified over silica gel column chromatography (Kiesel gel 60, 15 g, ethyl acetate:methanol = 5:1) to obtain 871 mg (yield: 86%) of the title compound (E39) as a colorless foam.
Rf value: 0.36 (ethyl acetate:methanol = 5:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (16H, s), 0.88 (3H, t, J=7.3Hz).

### iii) Methyl 5-acetamido-4-(N,N'-bis-tert-butoxycarbonyl)guanidino-2,3,4;5-tetradeoxy-8,9-O-isopropylidene-7-O-dodecanoyl-D-glycero-D-galacto-non-2-enopyranosoate (E40)

868 mg (1.65 mmol) of the compound (E39), 940 mg (3.4 mmol) of N,N'-bis-tert-butoxycarbonylthiourea and 0.95 ml (6.90 mmol) of triethylamine were dissolved in 10 ml of dimethylformamide at room temperature. Subsequently, 926 mg (3.4 mmol) of mercury chloride were added to the system under ice-cooling, and the mixture was stirred at room temperature for 2 hours. Ethyl acetate was added to the reaction mixture to dilute it and the mixture was filtered using Celite. The filtered product was washed with ethyl acetate. The filtrate and the washing solution were combined, and ethyl acetate and a saturated aqueous NaCl solution were added thereto to separate the mixture. The organic layer was dried over magnesium sulfate and filtered, followed by concentration under reduced pressure. The residue was purified over silica gel column chromatography (Kiesel gel 60, 15 g, hexane:ethyl acetate = 2:1) to obtain 1.3 g (yield: 100%) of the title compound (E40) as a colorless foam.
Rf value: 0.47 (hexane:ethyl acetate = 2:1);
¹H-NMR (270MHz, CDCl₃, TMS): δ (ppm) 11.4 (1H, s), 8.46 (1H, d, J=8.7Hz), 6.06(1H, d, J=8.7Hz), 5.88 (1H, d, J=2.4Hz), 5.37 (1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87 (3H, s), 1.61 (2H, quintet, J=7.5Hz), 1.49 (9H, s), 1.48 (9H, s), 1.38 (3H, s), 1.35 (3H, s), 1.25 (16H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-dodecanoyl-D-glycero-D-galacto-non-2-enopyranosoic acid (E41)

100 mg (0.15 mmol) of the compound (E40) were dissolved in a mixture of 4 ml (40-times volume) of methanol and 1 ml (10-times volume) of water, and 7.0 mg (0.165 mmol) of lithium hydroxide monohydrate were added to the system at room temperature, followed by stirring of the mixture at room temperature for 8 hours. After confirmation of completion of the reaction, Dowex-50W was added to the system to neutralize it, and the reaction mixture was filtered. The filtered product was washed with methanol. The filtrate and the washing solution were combined, and the mixture was concentrated under reduced pressure. The residue was purified over silica gel column chromatography (Kiesel gel 60, ethyl acetate:2-propanol:water = 10:2:1) to obtain 56 mg (yield: 56%) of the desired compound (E41) as a white solid.
Rf value: 0.35 (methylene chloride:methanol = 10:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (18H, s), 1.39 (3H, s), 1.37 (3H, s), 1.25 (8H, bs), 0.88 (3H, t, J=6.5Hz).

### v) 5-Acetamido-2,3,4,5-tetradeoxy-4-guanidino-9-O-dodecanoyl-D-glycero-D-galacto-non-2-enopyranosoic acid trifluoroacetic acid salt (E42)

50 mg (0.075 mmol) of the compound (E41) were dissolved in 3 ml (50-times volume) of methylene chloride at room temperature, and subsequently 1 ml (10-times volume) of trifluoroacetic acid was added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, 2-propanol:water = 5:1) to obtain 38 mg (yield: 88%) of the title compound (E42) as a pale yellow solid.
Rf value: 0.3 (2-propanol:water = 5:1);
¹H-NMR (270MHz, CD₃OD): δ (ppm) 5.55 (1H, bs), 4.40-4.10 (7H, m), 3.65 (1H, d, J=9.0Hz), 2.36 (2H, t, J=7.0Hz), 2.00 (3H, s), 1.70-1.50 (2H, m), 1.30 (8H, bs), 0.90 (3H, t, J=7.0Hz);
FAB-MS (positive): 515 (M+H)⁺;
[α]_{D}²⁴ = +20.5° (c=0.08, MeOH).

### (Example 9)

### 5-Acetamido-2,3,4,5-tetradeoxy-4-guanidino-9-O-octadecanoyl-D-glycero-D-galacto-non-2-enopyranosoic acid trifluoroacetic acid salt (E47) (Exemplary compound No. 43)

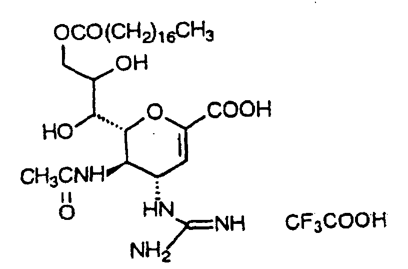

### i) Methyl 5-acetamido-4-azido-2,3,4,5-tetradeoxy-8,9-O-isopropylidene-7-O-octadecanoyl-D-glycero-D-galacto-non-2-enopyranosoate (E43)

1.10 g (2.98 mmol) of the compound (E3) were dissolved in 11 ml of methylene chloride at room temperature, and subsequently 1.70 ml (5.03 mmol) of stearoyl chloride and 620 mg (5.07 mmol) of 4-dimethylaminopyridine were added to the system under ice-cooling, followed by stirring of the mixture at room temperature for 30 minutes. Next, 0.70 ml (5.05 mmol) of triethylamine were poured into the reaction mixture at room temperature, and the resulting mixture was further stirred for 15 hours. After confirmation of completion of the reaction, methanol was poured into the system, and the mixture was stirred for 30 minutes. Next, ethyl acetate and a saturated aqueous NaCl solution were added to the reaction mixture to separate it. The organic layer was dried over magnesium sulfate and filtered, followed by concentration under reduced pressure. The residue was purified over silica gel column chromatography (Kiesel gel 60, n-hexane:ethyl acetate = 2:1) to obtain 1.21 g (yield: 64%) of the desired compound (E43) as a colorless foam.
Rf value: 0.51 (n-hexane:ethyl acetate = 1:1);
¹H-NMR (270MHz, CDCl₃, TMS): δ (ppm) 5.95 (1H, d, J=2.7Hz), 5.88 (1H, d, J=7.9Hz), 5.35 (1H, dd, J=6.0, 1.8Hz), 4.80 (1H, dd, J=9.1, 2.7Hz), 4.71 (1H, dd, J=10.5, 1.8Hz), 4.39 (1H, q, J=6.0Hz), 4.14 (1H, dd, J=8.8, 6.0Hz), 3.945 (1H, dd, J=8.8, 6.0Hz), 3.81 (3H, s), 3.45 (1H, ddd, J=10.5, 9.1, 7.9Hz), 2.41 (1H, t, J=7.5Hz), 2.39 (1H, t, J=7.5Hz), 2.02 (3H, s), 1.63 (2H, quintet, J=7.5Hz), 1.37 (3H, s), 1.35 (3H, s), 1.26 (28H, s), 0.88 (3H, t, J=7.5Hz).

### ii) Methyl 5-acetamido-4-amino-2,3,4,5-tetradeoxy-8,9-O-isopropylidene-7-O-octadecanoyl-D-glycero-D-galacto-non-2-enopyranosoate (E44)

1.20 g (1.88 mmol) of the compound (E43) were dissolved in 12 ml of ethanol at room temperature, and subsequently 399 mg (0.33-times volume) of Lindlar catalyst were added to the system at room temperature, followed by stirring of the mixture at room temperature under a hydrogen atmosphere of 1 atm. for 3 hours. After confirmation of completion of the reaction, the reaction mixture was filtered. The filtered product was washed with ethanol. The filtrate and the washing solution were combined, and the mixture was concentrated under reduced pressure. The residue was purified over silica gel column chromatography (Kiesel gel 60, 15 g, ethyl acetate:methanol = 10:1) to obtain 924 mg (yield: 80%) of the title compound (E44) as a colorless foam.
Rf value: 0.37 (ethyl acetate:methanol = 4:1);
¹H-NMR (270MHz, CD₃OD, TMS): δ (ppm) 5.94 (1H, d, J=2.4Hz), 5.42 (1H, dd, J=4.7, 1.8Hz), 4.39 (1H, dt, J=7.1, 6.0Hz), 4.18 (1H, dd, J=9.5, 1.6Hz), 4.14 (1H, dd, J=8.7, 6.4Hz), 3.93 (1H, dd, J=8.7, 6.4Hz), 3.87 (1H, t, J=9.5Hz), 3.78 (3H, s), 3.44 (1H, dd, J=9.5, 2.4Hz), 2.35 (2H, q, J=7.3Hz), 1.94 (3H, s), 1.60 (2H, quintet, J=7.3Hz), 1.32 (3H, s), 1.31 (3H, s), 1.27 (28H, s), 0.88 (3H, t, J=7.3Hz).

### iii) Methyl 5-acetamido-4-(N,N'-bis-tert-butoxycarbonyl)guanidino-2,3,4,5-tetradeoxy-8,9-O-isopropylidene-7-O-octadecanoyl-D-glycero-D-galacto-non-2-enopyranosoate (E45)

916 mg (1.50 mmol) of the compound (E44), 539 mg (1.95 mmol) of N,N'-bis-tert-butoxycarbonylthiourea and 0.27 ml (1.95 mmol) of triethylamine were dissolved in 12 ml of dimethylformamide at room temperature. Subsequently, 529 mg (1.95 mmol) of mercury chloride were added to the system under ice-cooling, and the mixture was stirred at room temperature for 4 hours. Ethyl acetate was added to the reaction mixture to-dilute it, and the mixture was filtered using Celite. The filtered product was washed with ethyl acetate. The filtrate and the washing solution were combined, and ethyl acetate and a saturated aqueous NaCI solution were added to the mixture separate it. The organic layer was dried over magnesium sulfate and filtered, followed by concentration under reduced pressure. The residue was purified over silica gel column chromatography (Kiesel gel 60, 15 g, hexane:ethyl acetate = 2:1) to obtain 750 mg (yield: 59%) of the title compound (E45) as a colorless foam.
Rf value: 0.29 (hexane:ethyl acetate = 2:1);
¹H-NMR (270MHz, CDCl₃, TMS): δ (ppm) 11.4 (1H, s), 8.46 (1H, d, J=8.7Hz), 6.06 (1H, d, J=8.7Hz), 5.88 (1H, d, J=2.4Hz), 5.37 (1H, dd, J=6.4, 1.5Hz), 5.15 (1H, dt, J=8.7, 2.4Hz), 4.38 (1H, q, J=6.4Hz), 4.28 (1H, dd, J=8.7, 1.5Hz), 4.23 (1H, t, J=8.7Hz), 4.10 (1H, dd, J=9.6, 6.4Hz), 3.95 (1H, dd, J=9.6, 6.4Hz), 3.80 (3H, s), 2.453 (1H, dt, J=16.0, 7.5Hz), 2.33 (1H, dt, J=16.0, 7.5Hz), 1.87 (3H, s), 1.61 (2H, quintet, J=7.5Hz), 1.49 (9H, s), 1.48 (9H, s), 1.38 (3H, s), 1.35 (3H, s), 1.25 (28H, bs), 0.88 (3H, t, J=7.5Hz).

### iv) 5-Acetamido-4-(N,N'-bis-tert-butoxycarbonyl)guanidino-2,3,4,5-tetradeoxy-8,9-O-isopropylidene-7-O-octadecanoyl-D-glycero-D-galacto-non-2-enopyranosoic acid (E46)

741 mg (0.87 mmol) of the compound (E45) were dissolved in a mixture of 15 ml of methanol and 1.5 ml of water, and 38 mg (0.91 mmol) of lithium hydroxide monohydrate were added to the system at room temperature, followed by stirring of the mixture at room temperature for 6 hours. After confirmation of completion of the reaction, a solution of 4N HCI in dioxane was added to the system to neutralize it, followed by concentration under reduced pressure. The residue was purified over silica gel column chromatography (Kiesel gel 60, ethyl acetate:methanol = 10:1) to obtain 430 mg (yield: 59%) of the desired compound (E16) as a colorless foam.
Rf value: 0.40 (ethyl acetate:methanol = 5:1);
¹H-NMR (270MHz, CDCl₃, TMS): δ (ppm) 11.4 (1H, s), 8.48 (1H, d, J=8.0Hz), 6.31 (1H, dull s), 5.90 (1H, bs), 5.30 (1H, bs), 5.10 (1H, bs), 4.60-3.30 (7H, m), 2.48 (1H, dt, J=13.5, 6.5Hz), 2.32 (1H, dt, J=13.5, 6.5Hz), 1.88 (3H, s), 1.60 (2H, quintet, J=6.5Hz), 1.48 (24H, s), 1.39 (3H, s), 1.37 (3H, s), 1.25 (28H, bs), 0.88 (3H, t, J=6.5Hz).

### v) 5-Acetamido-2,3,4,5-tetradeoxy-4-guanidino-9-O-octadecanoyl-D-glycero-D-galacto-non-2-enopyranosoic acid trifluoroacetic acid salt (E47)

422 mg (0.50 mmol) of the compound (E46) were dissolved in 3 ml of methylene chloride at room temperature, and subsequently 1 ml of trifluoroacetic acid was added to the system at room temperature, followed by stirring of the mixture at room temperature for 22 hours. After confirmation of completion of the reaction, the reaction mixture was concentrated under reduced pressure. The residue was purified over silica gel column chromatography (Kiesel gel 60, 5 g, ethyl acetate:2-propanol:water = 5:2:1) to obtain 300 mg (yield: 84%) of the title compound (E47) as a colorless foam.
Rf value: 0.44 (2-propanol:water = 5:1);
¹H-NMR(270MHz, CD₃OD): δ (ppm) 5.55 (1H, bs), 4.40-4.10 (7H, m), 3.65 (1H, d, J=9.0Hz), 2.36 (2H, t, J=7.0Hz), 2.00 (3H, s), 1.70-1.50 (2H, m), 1.30 (28H, bs), 0.90 (3H, t, J=7.0Hz);
FAB-MS (positive): 599 (M+H)⁺;
[α]_{D}²⁴ = +19.8° (c=0.15, MeOH).

### (Example 10)

### Cetyl 5-acetamido-2,3,4,5-tetradeoxy-4-guanidino-D-glycero-D-galacto-non-2-enopyranosoate trifluoroacetic acid salt (E48) (Exemplary compound No. 89)

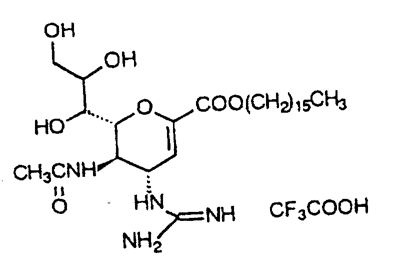

The procedures were carried out in a similar manner to those in Example 1 using cetyl alcohol instead of myristyl alcohol to obtain the title compound.
¹H-NMR (270MHz, CD₃OD): δ (ppm) 5.83 (1H, d, J=2.7Hz), 4.44 (1H, dd, J=9.0, 2.7Hz), 4.38 (1H, dd, J=9.0, <1Hz), 4.18 (2H, t, J=6.2Hz), 4.17 (1H, t, J=9.0Hz), 3.90-3.74 (2H, m), 3.68 (1H, dd, J=12.0, 4.5Hz), 3.65 (1H, d, J=9.0Hz), 1.99 (3H, s), 1.67 (2H, quintet, J=6.2Hz), 1.26 (28H, bs), 0.87 (3H, t, J=6.2Hz).

### (Example 11)

### Stearyl 5-acetamido-2,3,4,5-tetradeoxy-4-guanidino-D-glycero-D-galacto-non-2-enopyranosoate trifluoroacetic acid salt (E49) (Exemplary compound No. 91)

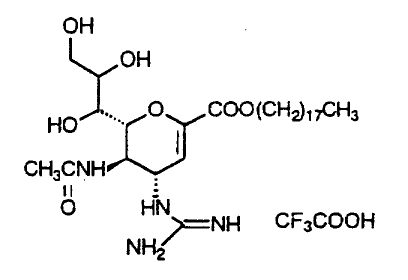

The procedures were carried out in a similar manner to those in Example 1 using stearyl alcohol instead of myristyl alcohol to obtain the title compound.
¹H-NMR (270MHz, CD₃OD): δ (ppm) 5.83 (1H, d, J=2.7Hz), 4.44 (1H, dd, J=9.0, 2.7Hz), 4.38 (1H, dd, J=9.0, <1Hz), 4.18 (2H, t, J=6.2Hz), 4.17 (1H, t, J=9.0Hz), 3.90-3.74 (2H, m), 3.68 (1H, dd, J=12.0, 4.5Hz), 3.65 (1H, d, J=9.0Hz), 1.99 (3H, s), 1.67 (2H, quintet, J=6.2Hz), 1.26 (32H, bs), 0.87 (3H, t, J=6.2Hz).

### (Preparation example 1)

An aqueous solution was prepared so that the compound of Example 4 was 10% (W/W), benzalkonium chloride 0.04% (W/W), phenylethyl alcohol 0.40% (W/W), and purified water 89.56% (W/W).

### (Preparation example 2)

An aqueous co-solvent solution was prepared so that the compound of Example 4 was 10% (W/W), benzalkonium chloride 0.04% (W/W), polyethylene glycol 400 10.0% (W/W), propylene glycol 30% (W/W), and purified water 39.96% (W/W).

### (Preparation example 3)

A dry powder was prepared so that the compound of Example 4 was 40% (W/W) and lactose was 60% (W/W).

### (Preparation example 4)

An aerosol agent was prepared so that the compound of Example 4 was 10% (W/W), lecithin 0.5% (W/W), Freon 11 34.5% and Freon 12 55%.

### (Test Example 1)

### Influenza Virus Sialidase Inhibitory Activity

A cannula was inserted into the trachea of mice (BALB/C, females, 5 to 6 weeks old, body weight: 20 g) under anesthesia followed by injection of 0.5 ml of phosphate-buffered physiological saline. Lung washings were then obtained by collecting the liquid by aspiration, repeating the procedure three times. 1 µM of the test compound (sample), 10 µg of lung washings in terms of the amount of protein, and phosphate-buffered physiological saline were mixed and incubated at 37°C for one to three days at a final volume of 100 µl. After sampling 10 µl of this reaction mixture, a sialidase reaction was carried out in 32.5 mM 2-(N-morpholino)ethanesulfonate buffer (pH 6.5) containing 40 mM calcium chloride using influenza virus A/PR/8/34 (equivalent to 5 × 10⁵ plaque-forming units) as the sialidase enzyme and 0.1 mM ammonium 4-methylumbelliferyl-N-acetyl-α-D-neuraminate as the substrate. The fluorescence intensity of the 4-methylumbelliferone produced in the reaction mixture was measured at an excitation wavelength of 360 nm and measurement wavelength of 460 nm. On the other hand, a concentration vs. inhibition curve was prepared by carrying out similar reactions using as sample various concentrations of Compound A (GG-167). The amount of substance having sialidase inhibitory activity formed in the lung washings from the test compound was quantitatively determined as the amount of Compound A using the above inhibition curve.

Although the compound of the present invention did not exhibit influenza virus sialidase inhibitory activity directly, by treating with biological fractions containing hydrolase (e.g., mouse lung washings), the compound of the present invention exhibited influenza virus sialidase inhibitory activity similar to that of Compound A.

### (Test Example 2)

### Mouse Infection Treatment Experiment 1

A solution was prepared containing 500 pfu (plaque-forming units) of mouse-acclimated influenza virus A/PR/8/34 strain in 50 µl of phosphate buffer containing 0.42% bovine serum albumin which was then used to infect mice (BALB/C, females, 5-6 weeks old, 20 g) by dropping into the nose for infection. The compounds of the present invention were prepared to a dose level of 0.6 µmol/kg/50 µl by suspending in physiological saline, and administered to the animals by dropping into the nose on a total of 3 occasions at 4 hours before, 4 hours after and 17 hours after viral infection. The test was performed on groups of 7 or 8 animals, and results were indicated as the number of surviving mice divided by the number of test mice at 6, 8 and 10 days after infection. Incidentally, Compound A (GG-167) was used as the comparative compound.

**[Table 2]**

| | Day 6 | Day 8 | Day 10 |
|---|---|---|---|
| Physiological saline only | 1/7 | 0/7 | 0/7 |
| Compound A | 7/7 | 3/7 | 0/7 |
| Compound of Example 1 | 8/8 | 5/8 | 3/8 |
| Compound of Example 2 | 8/8 | 2/8 | 1/8 |

Although all of the animals in the group dosed with Compound A were dead on day 10 after infection, 3 or 1 of the animals in the groups dosed with the compounds of Example 1 or Example 2 were still alive. These results indicate that the compound of Example 1 or Example 2 of the present invention possesses influenza infection therapeutic effects superior to those of Compound A.

### (Test Example 3)

### Mouse Infection Treatment Experiment-2

A solution was prepared containing 500 pfu (plaque-forming units) of mouse-acclimated influenza virus A/PR/8/34 strain in 50 µl of phosphate buffer containing 0.42% bovine serum albumin which was then used to infect mice (BALB/C, females, 5-6 weeks old, 20 g) by dropping into the nose for infection. The compounds of the present invention were prepared to a dose level of 0.9 µmol/kg/50 µl by suspending in physiological saline, and administered to the animals by dropping into the nose on a total of 3 occasions at 4 hours before, 4 hours after and 17 hours after viral infection. The test was performed on groups of 4 to 12 animals, and results were indicated as the number of surviving mice divided by the number of test mice at 8 and 10 days after infection. Incidentally, Compound A (GG-167) was used as the comparative compound.

**[Table 3]**

| | Day 8 | Day 10 |
|---|---|---|
| Physiological saline only | 0/4 | 0/4 |
| Compound A | 0/8 | 0/8 |
| Compound of Example 1 | 4/12 | 2/12 |
| Compound of Example 10 | 10/12 | 5/12 |
| Compound of Example 11 | 11/11 | 5/11 |

Although all of the animals in the group dosed with Compound A were dead on day 10 after infection, 2 to 5 of the animals in the groups dosed with the compounds of Example 1, 10 or 11 were still alive. These results indicate that the compound of Example 1, Example 10 or Example 11 of the present invention possesses influenza infection therapeutic effects superior to those of Compound A.

### (Test Example 4)

### Mouse Infection Treatment Experiment-3

A solution was prepared containing 500 pfu (plaque-forming units) of mouse-acclimated influenza virus A/PR/8/34 strain in 50 µl of phosphate buffer containing 0.42% bovine serum albumin which was then used to infect mice (BALB/C, females, 5-6 weeks old, 20 g) by dropping into the nose for infection. The compounds of the present invention were prepared to a dose level of 0.3 µmol/kg/50 µl by suspending in physiological saline, and administered to the animals by dropping into the nose on a total of 3 occasions at 4 hours before, 4 hours after and 17 hours after viral infection. The test was performed on groups of 10 or 11 animals, and results were indicated as the number of surviving mice divided by the number of test mice at 6 and 8 days after infection. Incidentally, Compound A (GG-167) was used as the comparative compound.

**[Table 4]**

| | Day 6 | Day 8 |
|---|---|---|
| Physiological saline only | 0/10 | 0/10 |
| Compound A | 10/10 | 1/10 |
| Compound of Example 3 | 10/10 | 10/10 |
| Compound of Example 4 | 10/10 | 6/10 |
| Compound of Example 5 | 11/11 | 10/11 |
| Compound of Example 6 | 11/11 | 10/11 |
| Compound of Example 7 | 10/10 | 3/10 |
| Compound of Example 8 | 11/11 | 2/11 |
| Compound of Example 9 | 10/10 | 4/10 |

Although only one of the animals in the group dosed with Compound A was alive on day 8 after infection, 2 to 10 animals in the groups dosed with the compound of any one of Examples 3 to 9 were still alive. These results indicate that the compounds of Examples 3 to 9 of the present invention possess influenza infection therapeutic effects superior to those of Compound A.

### [Industrial applicability]

The neuraminic acid compound (1) of the present invention undergoes hydrolysis by hydrolase present in a living body and exhibits excellent viral replication inhibitory activity and sialidase inhibitory activity. In addition, if the neuraminic acid compound (1) is administered to mice infected with influenza virus, the compound exhibits infection therapeutic effects superior to Compound A (GG-167) described in WO91/16320 (Japanese PCT application (Kokai) No. Hei 5-507068). Thus, the neuraminic acid compound (1) of the present invention is useful as a therapeutic agent or preventive agent (preferably therapeutic agent) for viral infections (preferably influenza viral infections).

## Claims

1. A compound represented by the formula (1): 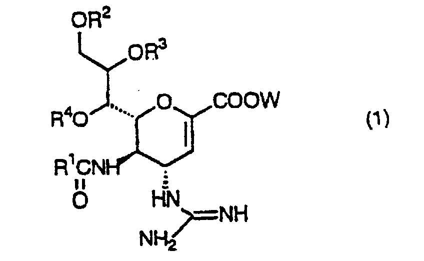 or a pharmacologically acceptable salt thereof, wherein:
R¹ represents a methyl group which may be substituted with one or more halogen atoms;
R², R³ and R⁴ may be the same or different and each represents a hydrogen atom or an aliphatic acyl group having from 3 to 25 carbon atoms; and
W represents a hydrogen atom or an ester residue;
PROVIDED THAT compounds of formula (1) wherein each of R², R³, R⁴ and W is a hydrogen atom are excluded.

2. The compound or pharmacologically acceptable salt thereof according to Claim 1, wherein R¹ is a methyl group which may be substituted with one or more fluorine atoms.

3. The compound or pharmacologically acceptable salt thereof according to Claim 1, wherein R¹ is a methyl, monofluoromethyl or difluoromethyl group.

4. The compound or pharmacologically acceptable salt thereof according to Claim 1, wherein R¹ is a methyl group.

5. The compound or pharmacologically acceptable salt thereof according to any one of Claims 1 to 4, wherein R² is a hydrogen atom or an aliphatic acyl group having from 6 to 25 carbon atoms.

6. The compound or pharmacologically acceptable salt thereof according to any one of Claims 1 to 4, wherein R² is a hydrogen atom or an aliphatic acyl group having from 6 to 20 carbon atoms.

7. The compound or pharmacologically acceptable salt thereof according to any one of Claims 1 to 4, wherein R² is a hydrogen atom, or a hexanoyl, octanoyl, decanoyl, dodecanoyl, myristoyl, palmitoyl or stearoyl group.

8. The compound or pharmacologically acceptable salt thereof according to any one of Claims 1 to 7, wherein R³ is a hydrogen atom or an aliphatic acyl group having from 6 to 25 carbon atoms.

9. The compound or pharmacologically acceptable salt thereof according to any one of Claims 1 to 7, wherein R³ is a hydrogen atom or an aliphatic acyl group having from 6 to 20 carbon atoms.

10. The compound or pharmacologically acceptable salt thereof according to any one of Claims 1 to 7, wherein R³ is a hydrogen atom, or a hexanoyl, octanoyl, decanoyl, dodecanoyl, myristoyl, palmitoyl or stearoyl group.

11. The compound or pharmacologically acceptable salt thereof according to any one of Claims 1 to 10, wherein R⁴ is a hydrogen atom or an aliphatic acyl group having from 6 to 25 carbon atoms.

12. The compound or pharmacologically acceptable salt thereof according to any one of Claims 1 to 10, wherein R⁴ is a hydrogen atom or an aliphatic acyl group having from 6 to 20 carbon atoms.

13. The compound or pharmacologically acceptable salt thereof according to any one of Claims 1 to 10, wherein R⁴ is a hydrogen atom, or a hexanoyl, octanoyl, decanoyl, dodecanoyl, myristoyl, palmitoyl or stearoyl group.

14. The compound or pharmacologically acceptable salt thereof according to any one of Claims 1 to 4, wherein R² is an aliphatic acyl group having from 3 to 25 carbon atoms, and each of R³ and R⁴ is a hydrogen atom.

15. The compound or pharmacologically acceptable salt thereof according to any one of Claims 1 to 4, wherein R² is an aliphatic acyl group having from 6 to 25 carbon atoms, and each of R³ and R⁴ is a hydrogen atom.

16. The compound or pharmacologically acceptable salt thereof according to any one of Claims 1 to 4, wherein R² is an aliphatic acyl group having from 6 to 20 carbon atoms, and each of R³ and R⁴ is a hydrogen atom.

17. The compound or pharmacologically acceptable salt thereof according to any one of Claims 1 to 4, wherein R² is a hexanoyl, octanoyl, decanoyl, dodecanoyl, myristoyl, palmitoyl or stearoyl group, and each of R³ and R⁴ is a hydrogen atom.

18. The compound or pharmacologically acceptable salt thereof according to any one of Claims 1 to 17, wherein W is a hydrogen atom or an alkyl group having from 1 to 18 carbon atoms.

19. The compound or pharmacologically acceptable salt thereof according to any one of Claims 1 to 17, wherein W is a hydrogen atom.

20. The compound or pharmacologically acceptable salt thereof according to any one of Claims 1 to 17, wherein W is an ester residue.

21. The compound or pharmacologically acceptable salt thereof according to any one of Claims 1 to 17, wherein W is an alkyl group having from 6 to 18 carbon atoms.

22. The compound or pharmacologically acceptable salt thereof according to any one of Claims 1 to 4, wherein each of R², R³ and R⁴ is a hydrogen atom, and W is an ester residue.

23. The compound or pharmacologically acceptable salt thereof according to any one of Claims 1 to 4, wherein each of R², R³ and R⁴ is a hydrogen atom, and W is an alkyl group having from 6 to 18 carbon atoms.

24. The compound or pharmacologically acceptable salt thereof according to Claim 1, wherein R¹ is a methyl group which may be substituted with one or more fluorine atoms, R² is an aliphatic acyl group having from 3 to 25 carbon atoms, each of R³ and R⁴ is a hydrogen atom, and W is a hydrogen atom or an ester residue.

25. The compound or pharmacologically acceptable salt thereof according to Claim 1, wherein R¹ is a methyl group, R² is an aliphatic acyl group having from 6 to 25 carbon atoms, each of R³ and R⁴ is a hydrogen atom, and W is a hydrogen atom or an alkyl group having from 1 to 18 carbon atoms.

26. The compound or pharmacologically acceptable salt thereof according to Claim 1, wherein R¹ is a methyl group, R² is an aliphatic acyl group having from 6 to 20 carbon atoms, and each of R³, R⁴ and W is a hydrogen atom.

27. The compound or pharmacologically acceptable salt thereof according to Claim 1, wherein R¹ is a methyl group which may be substituted with one or more fluorine atoms, each of R², R³ and R⁴ is a hydrogen atom, and W is an ester residue.

28. The compound or pharmacologically acceptable salt thereof according to Claim 1, wherein R¹ is a methyl group, each of R², R³ and R⁴ is a hydrogen atom, and W is an alkyl group having from 6 to 18 carbon atoms.

29. The compound or pharmacologically acceptable salt thereof according to Claim 1 selected from the following group of compounds:
5-acetamido-2,3,4,5-tetradeoxy-4-guanidino-9-O-hexanoyl-D-glycero-D-galacto-non-2-enopyranosoic acid,
5-acetamido-2,3,4,5-tetradeoxy-4-guanidino-9-O-octanoyl-D-glycero-D-galacto-non-2-enopyranosoic acid,
5-acetamido-2,3,4,5-tetradeoxy-4-guanidino-9-O-decanoyl-D-glycero-D-galacto-non-2-enopyranosoic acid,
5-acetamido-2,3,4,5-tetradeoxy-4-guanidino-9-0-dodecanoyl-D-glycero-D-galacto-non-2-enopyranosoic acid,
5-acetamido-2,3,4,5-tetradeoxy-4-guanidino-9-O-myristoyl-D-glycero-D-galacto-non-2-enopyranosoic acid,
5-acetamido-2,3,4,5-tetradeoxy-4-guanidino-9-O-palmitoyl-D-glycero-D-galacto-non-2-enopyranosoic acid,
5-acetamido-2,3,4,5 -tetradeoxy-4-guanidino-9-O-stearoyl-D-glycero-D-galacto-non-2-enopyranosoic acid,
hexyl 5-acetamido-2,3,4,5-tetradeoxy-4-guanidino-D-glycero-D-galacto-non-2-enopyranosoate,
myristyl 5-acetamido-2,3,4,5-tetradeoxy-4-guanidino-D-glycero-D-galacto-non-2-enopyranosoate,
cetyl 5-acetamido-2,3,4,5-tetradeoxy-4-guanidino-D-glycero-D-galacto-non-2-enopyranosoate, and
stearyl 5-acetamido-2,3,4,5-tetradeoxy-4-guanidino-D-glycero-D-galacto-non-2-enopyranosoate.

30. A pharmaceutical composition containing as the active ingredient the compound of formula (1) or a pharmacologically acceptable salt thereof as defined in any one of Claims 1 to 29.

31. Use of the compound of formula (1) or a pharmacologically acceptable salt thereof as defined in any one of Claims 1 to 29 in the manufacture of a medicament for the treatment or prevention of viral infections.

## Patentansprüche

1. Durch die Formel (1) dargestellte Verbindung: 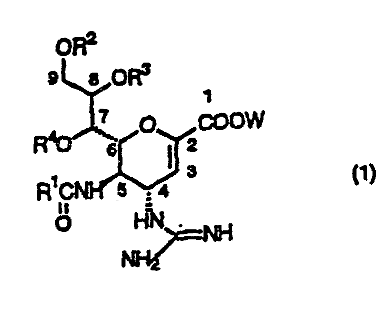 oder pharmakologisch geeignetes Salz davon, worin
R¹ eine Methylgruppe, die mit einem oder mehreren Halogenatomen substituiert sein kann, darstellt;
R², R³ und R⁴ gleich oder verschieden sein können und jeweils ein Wasserstoffatom oder eine aliphatische Acylgruppe mit 3 bis 25 Kohlenstoffatomen darstellen und
W ein Wasserstoffatom oder einen Esterrest darstellt,
mit der Maßgabe, dass Verbindungen der Formel (1), worin jedes von R², R³, R⁴ und W ein Wasserstoffatom ist, ausgeschlossen sind.

2. Verbindung oder pharmakologisch geeignetes Salz davon nach Anspruch 1, worin R¹ eine Methylgruppe ist, die mit einem oder mehreren Fluoratomen substituiert sein kann.

3. Verbindung oder pharmakologisch geeignetes Salz davon nach Anspruch 1, worin R¹ eine Methyl-, Monofluormethyl- oder Difluormethylgruppe ist.

4. Verbindung oder pharmakologisch geeignetes Salz davon nach Anspruch 1, worin R¹ eine Methylgruppe ist.

5. Verbindung oder pharmakologisch geeignetes Salz davon nach einem der Ansprüche 1 bis 4, worin R² ein Wasserstoffatom oder eine aliphatische Acylgruppe mit 6 bis 25 Kohlenstoffatomen ist.

6. Verbindung oder pharmakologisch geeignetes Salz davon nach einem der Ansprüche 1 bis 4, worin R² ein Wasserstoffatom oder eine aliphatische Acylgruppe mit 6 bis 20 Kohlenstoffatomen ist.

7. Verbindung oder pharmakologisch geeignetes Salz davon nach einem der Ansprüche 1 bis 4, worin R² ein Wasserstoffatom oder eine Hexanoyl-, Octanoyl-, Decanoyl-, Dodecanoyl-, Myristoyl-, Palmitoyl- oder Stearoylgruppe ist.

8. Verbindung oder pharmakologisch geeignetes Salz davon nach einem der Ansprüche 1 bis 7, worin R³ ein Wasserstoffatom oder eine aliphatische Acylgruppe mit 6 bis 25 Kohlenstoffatomen ist.

9. Verbindung oder pharmakologisch geeignetes Salz davon nach einem der Ansprüche 1 bis 7, worin R³ ein Wasserstoffatom oder eine aliphatische Acylgruppe mit 6 bis 20 Kohlenstoffatomen ist.

10. Verbindung oder pharmakologisch geeignetes Salz davon nach einem der Ansprüche 1 bis 7, worin R³ ein Wasserstoffatom oder eine Hexanoyl-, Octanoyl-, Decanoyl-, Dodecanoyl-, Myristoyl-, Palmitoyl- oder Stearoylgruppe ist.

11. Verbindung oder pharmakologisch geeignetes Salz dieser nach einem der Ansprüche 1 bis 10, worin R⁴ ein Wasserstoffatom oder eine aliphatische Acylgruppe mit 6 bis 25 Kohlenstoffatomen ist.

12. Verbindung oder pharmakologisch geeignetes Salz davon nach einem der Ansprüche 1 bis 10, worin R⁴ ein Wasserstoffatom oder eine aliphatische Acylgruppe mit 6 bis 20 Kohlenstoffatomen ist.

13. Verbindung oder pharmakologisch geeignetes Salz davon nach einem der Ansprüche 1 bis 10, worin R⁴ ein Wasserstoffatom oder eine Hexanoyl-, Octanoyl-, Decanoyl-, Dodecanoyl-, Myristoyl-, Palmitoyl- oder Stearoylgruppe ist.

14. Verbindung oder pharmakologisch geeignetes Salz davon nach einem der Ansprüche 1 bis 4, worin R² eine aliphatische Acylgruppe mit 3 bis 25 Kohlenstoffatomen ist und jedes von R³ und R⁴ ein Wasserstoffatom ist.

15. Verbindung oder pharmakologisch geeignetes Salz davon nach einem der Ansprüche 1 bis 4, worin R² eine aliphatische Acylgruppe mit 6 bis 25 Kohlenstoffatomen ist und jedes von R³ und R⁴ ein Wasserstoffatom ist.

16. Verbindung oder pharmakologisch geeignetes Salz davon nach einem der Ansprüche 1 bis 4, worin R² eine aliphatische Acylgruppe mit 6 bis 20 Kohlenstoffatomen ist und jedes von R³ und R⁴ ein Wasserstoffatom ist.

17. Verbindung oder pharmakologisch geeignetes Salz davon nach einem der Ansprüche 1 bis 4, worin R² eine Hexanoyl-, Octanoyl-, Decanoyl-, Dodecanoyl-, Myristoyl-, Palmitoyloder Stearoylgruppe ist und jedes von R³ und R⁴ ein Wasserstoffatom ist.

18. Verbindung oder pharmakologisch geeignetes Salz davon nach einem der Ansprüche 1 bis 17, worin W ein Wasserstoffatom oder eine Alkylgruppe mit 1 bis 18 Kohlenstoffatomen ist.

19. Verbindung oder pharmakologisch geeignetes Salz davon nach einem der Ansprüche 1 bis 17, worin W ein Wasserstoffatom ist.

20. Verbindung oder pharmakologisch geeignetes Salz davon nach einem der Ansprüche 1 bis 17, worin W ein Esterrest ist.

21. Verbindung oder pharmakologisch geeignetes Salz davon nach einem der Ansprüche 1 bis 17, worin W ein Alkylgruppe mit 6 bis 18 Kohlenstoffatomen ist.

22. Verbindung oder pharmakologisch geeignetes Salz davon nach einem der Ansprüche 1 bis 4, worin jedes von R², R³ und R⁴ ein Wasserstoffatom ist und W ein Esterrest ist.

23. Verbindung oder pharmakologisch geeignetes Salz davon nach einem der Ansprüche 1 bis 4, worin jedes von R², R³ und R⁴ ein Wasserstoffatom ist und W eine Alkylgruppe mit 6 bis 18 Kohlenstoffatomen ist.

24. Verbindung oder pharmakologisch geeignetes Salz davon nach Anspruch 1, worin R¹ eine Methylgruppe ist, die mit einem oder mehreren Fluoratomen substituiert sein kann, R² eine aliphatische Acylgruppe mit 3 bis 25 Kohlenstoffatomen ist, jedes von R³ und R⁴ ein Wasserstoffatom ist und W ein Wasserstoffatom oder ein Esterrest ist.

25. Verbindung oder pharmakologisch geeignetes Salz davon nach Anspruch 1, worin R¹ eine Methylgruppe ist, R² eine aliphatische Acylgruppe mit 6 bis 25 Kohlenstoffatomen ist, jedes von R³ und R⁴ ein Wasserstoffatom ist und W ein Wasserstoffatom oder eine Alkylgruppe mit 1 bis 18 Kohlenstoffatomen ist.

26. Verbindung oder pharmakologisch geeignetes Salz davon nach Anspruch 1, worin R¹ eine Methylgruppe ist, R² eine aliphatische Acylgruppe mit 6 bis 20 Kohlenstoffatomen ist und jedes von R³ und R⁴ und W ein Wasserstoffatom ist.

27. Verbindung oder pharmakologisch geeignetes Salz davon nach Anspruch 1, worin R¹ eine Methylgruppe, die mit einem oder mehreren Fluoratomen substituiert sein kann, jedes von R², R³ und R⁴ ein Wasserstoffatom ist und W ein Esterrest ist.

28. Verbindung oder pharmakologisch geeignetes Salz davon nach Anspruch 1, worin R¹ eine Methylgruppe ist, jedes von R², R³ und R⁴ ein Wasserstoffatom ist und W eine Alkylgruppe mit 6 bis 18 Kohlenstoffatomen ist.

29. Verbindung oder pharmakologisch geeignetes Salz davon nach Anspruch 1, ausgewählt aus der folgenden Gruppe von Verbindungen:
5-Acetamido-2,3,4,5-tetradeoxy-4-guanidino-9-O-hexanoyl-D-glycero-D-galacto-non-2-enopyrancarbonsäure,
5-Acetamido-2,3,4,5-tetradeoxy-4-guanidino-9-O-octanoyl-D-glycero-D-galacto-non-2-enopyrancarbonsäure,
5-Acetamido-2,3,4,5-tetradeoxy-4-guanidino-9-O-decanoyl-D-glycero-D-galacto-non-2-enopyrancarbonsäure,
5-Acetamido-2,3,4,5-tetradeoxy-4-guanidino-9-Ododecanoyl-D-glycero-D-galacto-non-2-enopyrancarbonsäure,
5-Acetamido-2,3,4,5-tetradeoxy-4-guanidino-9-Omyristoyl-D-glycero-D-galacto-non-2-enopyrancarbonsäure,
5-Acetamido-2,3,4,5-tetradeoxy-4-guanidino-9-Opalmitoyl-D-glycero-D-galacto-non-2-enopyrancarbonsäure,
5-Acetamido-2,3,4,5-tetradeoxy-4-guanidino-9-O-stearoyl-D-glycero-D-glacto-non-2-enopyrancarbonsäure,
Hexyl-5-acetamido-2,3,4,5-tetradeoxy-4-guanidino-Dglycero-D-galacto-non-2-enopyranosat,
Myristyl-5-acetamido-2,3,4,5-tetradeoxy-4-guanidino-Dglycero-D-galacto-non-2-enopyranosat,
Cetyl-5-acetamido-2,3,4,5-tetradeoxy-4-guanidino-Dglycero-D-galacto-non-2-enopyranosat und
Stearyl-5-acetamido-2,3,4,5-tetradeoxy-4-guanidino-Dglycero-D-galacto-non-2-enopyranosat.

30. Pharmazeutische Zusammensetzung, die als aktiven Bestandteil die Verbindung der Formel (1) oder ein pharmakologisch geeignetes Salz davon gemäß der Definition in einem der Ansprüche 1 bis 29 enthält.

31. Verwendung der Verbindung der Formel (1) oder eines pharmakologisch geeigneten Salzes davon gemäß der Definition in einem der Ansprüche 1 bis 29 bei der Herstellung eines Arzneimittels zur Behandlung oder Prävention von Virusinfektionen.

## Revendications

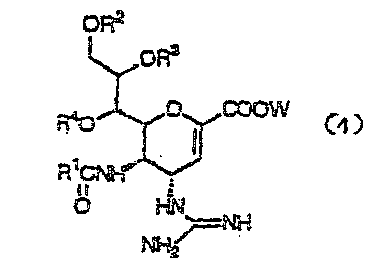

1. Composé représenté par la (formule 1) : ou un sel pharmacologiquement acceptable de celui-ci, dans lequel :
R¹ représente un groupe méthyle qui peut être substitué avec un ou plusieurs atomes d'halogène ;
R², R³ et R⁴ peuvent être identiques ou différents, et représentent chacun un atome d'hydrogène ou un groupe acyle aliphatique comportant de 3 à 25 atomes de carbone ; et
W représente un atome d'hydrogène ou un groupe ester ;
étant étendu que les composés de formule (1) dans laquelle chacun des groupes R², R³, R⁴ et W représente un atome d'hydrogène, sont exclus.

2. Composé ou sel pharmacologiquement acceptable de celui-ci selon la revendication 1, dans lequel R¹ représente un groupe méthyle qui peut être substitué avec un ou plusieurs atomes de fluor.

3. Composé ou sel pharmacologiquement acceptable de celui-ci selon la revendication 1, dans lequel R¹ représente un groupe méthyle, monofluorométhyle ou difluorométhyle.

4. Composé ou sel pharmacologiquement acceptable de celui-ci selon la revendication 1, dans lequel R¹ représente un groupe méthyle.

5. Composé ou sel pharmacologiquement acceptable de celui-ci selon l'une quelconque des revendications 1 à 4, dans lequel R² représente un atome d'hydrogène ou un groupe acyle aliphatique comportant de 6 à 25 atomes de carbone.

6. Composé ou sel pharmacologiquement acceptable de celui-ci selon l'une quelconque des revendication 1 à 4, dans lequel R² représente un atome d'hydrogène ou un groupe acyle aliphatique comportant de 6 à 20 atomes de carbone.

7. Composé ou sel pharmacologiquement acceptable de celui-ci selon l'une quelconque des revendications 1 à 4, dans lequel R² représente un atome d'hydrogène, ou un groupe hexanoyle, octanoyle, décanoyle, dodécanoyle, myristoyle, palmitoyle ou stéaroyle.

8. Composé ou sel pharmacologiquement acceptable de celui-ci selon l'une quelconque des revendications 1 à 7, dans lequel R³ représente un atome d'hydrogène ou un groupe acyle aliphatique comportant de 6 à 25 atomes de carbone.

9. Composé ou sel pharmacologiquement acceptable de celui-ci selon l'une quelconque des revendications 1 à 7, dans lequel R³ représente un atome d'hydrogène ou un groupe acyle aliphatique comportant de 6 à 20 atomes de carbone.

10. Composé ou sel pharmacologiquement acceptable de celui-ci selon l'une quelconque des revendications 1 à 7, dans lequel R³ représente un atome d'hydrogène, ou un groupe hexanoyle, octanoyle, décanoyle, dodécanoyle, myristoyle, palmitoyle ou stéaroyle.

11. Composé ou sel pharmacologiquement acceptable de celui-ci selon l'une quelconque des revendications 1 à 10, dans lequel R⁴ représente un atome d'hydrogène ou un groupe acyle aliphatique comportant de 6 à 25 atomes de carbone.

12. Composé ou sel pharmacologiquement acceptable de celui-ci selon l'une quelconque des revendications 1 à 10, dans lequel R⁴ représente un atome d'hydrogène ou un groupe acyle aliphatique comportant de 6 à 20 atomes de carbone.

13. Composé ou sel pharmacologiquement acceptable de celui-ci selon l'une quelconque des revendications 1 à 10, dans lequel R⁴ représente un atome d'hydrogène, ou un groupe hexanoyle, octanoyle, décanoyle, dodécanoyle, myristoyle, palmitoyle ou stéaroyle.

14. Composé ou sel pharmacologiquement acceptable de celui-ci selon l'une quelconque des revendications 1 à 4, dans lequel R² représente un groupe acyle aliphatique comportant de 3 à 25 atomes de carbone, et chacun des groupes R³ et R⁴ représente un atome d'hydrogène.

15. Composé ou sel pharmacologiquement acceptable de celui-ci selon l'une quelconque des revendications 1 à 4, dans lequel R² représente un groupe acyle aliphatique comportant de 6 à 25 atomes de carbone, et chacun des groupes R³ et R⁴ représente un atome d'hydrogène.

16. Composé ou sel pharmacologiquement acceptable de celui-ci selon l'une quelconque des revendications 1 à 4, dans lequel R² représente un groupe acyle aliphatique comportant de 6 à 20 atomes de carbone, et chacun des groupes R³ et R⁴ représente un atome d'hydrogène.

17. Composé ou sel pharmacologiquement acceptable de celui-ci selon l'une quelconque des revendications 1 à 4, dans lequel R² représente un groupe hexanoyle, octanoyle, décanoyle, dodécanoyle, myristoyle, palmitoyle ou stéaroyle, et chacun des groupes R³ et R⁴ représente un atome d'hydrogène.

18. Composé ou sel pharmacologiquement acceptable de celui-ci selon l'une quelconque des revendications 1 à 17, dans lequel W représente un atome d'hydrogène ou un groupe alkyle comportant de 1 à 18 atomes de carbone.

19. Composé ou sel pharmacologiquement acceptable de celui-ci selon l'une quelconque des revendications 1 à 17, dans lequel W représente un atome d'hydrogène.

20. Composé ou sel pharmacologiquement acceptable de celui-ci selon l'une quelconque des revendications 1 à 17, dans lequel W représente un groupe ester.

21. Composé ou sel pharmacologiquement acceptable de celui-ci selon l'une quelconque des revendications 1 à 17, dans lequel W représente un groupe alkyle comportant de 6 à 18 atomes de carbone.

22. Composé ou sel pharmacologiquement acceptable de celui-ci selon l'une quelconque des revendications 1 à 4, dans lequel chacun des groupes R², R³ et R⁴ représente un atome d'hydrogène, et W représente un groupe ester.

23. Composé ou sel pharmacologiquement acceptable de celui-ci selon l'une quelconque des revendications 1 à 4, dans lequel chacun des groupes R², R³ et R⁴ représente un atome d'hydrogène, et W représente un groupe alkyle comportant de 6 à 18 atomes de carbone.

24. Composé ou sel pharmacologiquement acceptable de celui-ci selon la revendication 1, dans lequel R¹ représente un groupe méthyle qui peut être substitué avec un ou plusieurs atomes de fluor, chacun des groupes R² représente un groupe acyle aliphatique comportant de 3 à 25 atomes de carbone, et chacun des groupes R³ et R⁴ représente un atome d'hydrogène, et W représente un atome d'hydrogène ou un groupe ester.

25. Composé ou sel pharmacologiquement acceptable de celui-ci selon la revendication 1, dans lequel R¹ représente un groupe méthyle, R² représente un groupe acyle aliphatique comportant de 6 à 25 atomes de carbone, et chacun des groupes R³ et R⁴ représente un atome d'hydrogène, et W représente un atome d'hydrogène ou un groupe alkyle comportant de 1 à 18 atomes de carbone.

26. Composé ou sel pharmacologiquement acceptable de celui-ci selon la revendication 1, dans lequel R¹ représente un groupe méthyle, R² représente un groupe acyle aliphatique comportant de 6 à 20 atomes de carbone, et chacun des groupes R³, R⁴ et W représente respectivement un atome d'hydrogène.

27. Composé ou sel pharmacologiquement acceptable de celui-ci selon la revendication 1, dans lequel R¹ représente un groupe méthyle qui peut être substitué avec un ou plusieurs atomes de fluor, R², chacun des groupes R³ et R⁴ représente un atome d'hydrogène, et W représente un groupe ester.

28. Composé ou sel pharmacologiquement acceptable de celui-ci selon la revendication 1, dans lequel R¹ représente un groupe méthyle, chacun des groupes R², R³ et R⁴ représente un atome d'hydrogène, et W représente un groupe alkyle comportant de 6 à 18 atomes de carbone.

29. Composé ou sel pharmacologiquement acceptable de celui-ci selon la revendication 1 choisi parmi le groupe de composés suivants :
l'acide 5-acétamido-2,3,4,5-tétradésoxy-4-guanidino-9-O-hexanoyl-D-glycéro-D-galacto-non-2-énopyrannosoïque,
l'acide 5-acétamido-2,3,4,5-tétradésoxy-4-guanidino-9-O-octanoyl-D-glycéro-D-galacto-non-2-énopyrannosoïque,
l'acide 5-acétamido-2,3,4,5-tétradésoxy-4-guanidino-9-O-décanoyl-D-glycéro-D-galacto-non-2-énopyrannosoïque,
l'acide 5-acétamido-2,3,4,5-tétradésoxy-4-guanidino-9-O-dodécanoyl-D-glycéro-D-galacto-non-2-énopyrannosoïque,
l'acide 5-acétamido-2,3,4,5-tétradésoxy-4-guanidino-9-O-myristoyl-D-glycéro-D-galacto-non-2-énopyrannosoïque,
l'acide 5-acétamido-2,3,4,5-tétradésoxy-4-guanidino-9-O-palmitoyl-D-glycéro-D-galacto-non-2-énopyrannosoïque,
l'acide 5-acétamido-2,3,4,5-tétradésoxy-4-guanidino-9-O-stéaroyl-D-glycéro-D-galacto-non-2-énopyrannosoïque,
le 5-acétamido-2,3,4,5-tétradésoxy-4-guanidino-D-glycéro-D-galacto-non-2-énopyrannosoate d'hexyle,
le 5-acétamido-2,3,4,5-tétradésoxy-4-guanidino-D-glycéro-D-galacto-non-2-énopyrannosoate de myristyle,
le 5-acétamido-2,3,4,5-tétradésoxy-4-guanidino-D-glycéro-D-galacto-non-2-énopyrannosoate de cétyle, et
le 5-acétamido-2,3,4,5-tétradésoxy-4-guanidino-D-glycéro-D-galacto-non-2-énopyrannosoate de stéaryle.

30. Composition pharmaceutique contenant à titre d' ingrédient actif, le composé de formule (1) ou un sel pharmacologiquement acceptable de celui-ci tel que défini dans l'une quelconque des revendications 1 à 29.

31. Utilisation du composé de formule (1) ou d'un sel pharmacologiquement acceptable de celui-ci tel que défini dans l'une quelconque des revendications 1 à 29, pour la préparation d'un médicament destiné au traitement ou la prévention d'infections virales.
